# EUROPEAN PATENT SPECIFICATION

(11) **EP 0 892 795 B1**
(45) Date of publication and mention of the grant of the patent: **08.01.2003**
(21) Application number: 97916641.0
(22) Date of filing: 10.04.1997
(51) Int. Cl.: C07D 401/04, C07D 405/14, A61K 31/505

(54) **PIPERIDINYLPYRAMIDINE DERIVATIVES**
PIPERIDINYLPYRIMIDINE DERIVATE
DERIVES DE PIPERIDINYLPYRIMIDINE

(30) Priority: 12.04.1996 JP 11555696; 27.09.1996 US 722548
(43) Date of publication of application: 27.01.1999
(73) Proprietor: SUMITOMO PHARMACEUTICALS COMPANY, LIMITED, Osaka-shi, Osaka 541 (JP)
(72) Inventor: FUJIWARA, Norio, Yao-shi, Osaka 581 (JP); UEDA, Yutaka, Ikoma-shi, Nara (JP); MURATA, Shinobu, Toyonaka-shi, Osaka 561 (JP); HIROTA, Fumiyo, Nishinomiya-shi, Hyogo 662 (JP); KAWAKAMI, Hajime, Nishinomiya-shi, Hyogo 662 (JP); FUJITA, Hitoshi, Nishinomiya-shi, Hyogo 662 (JP)
(74) Representative: Cresswell, Thomas Anthony
(86) International application number: JP9701240
(87) International publication number: WO97038992

(56) References cited:
- CHEMICAL ABSTRACTS, vol. 101, no. 28, 1984 Columbus, Ohio, US; abstract no. 151875u, page 719; XP002034996 & JP 05 976 082 A (KYOWA HAKKO KOGYO CO.) 28 April 1984

## Description

### Technical Field

The present invention relates to the use of the compounds of the invention in the manufacture of a medicament for inhibiting the production and/or secretion of tumor necrosis factor, the use of a pharmaceutical composition in the manufacture of a medicament for inhibiting the production and/or secretion of tumor necrosis factor and novel piperidinylpyrimidine derivatives.

### Background Art

Tumor necrosis factor alpha (hereinafter abbreviated as TNFα) is a peptide of 157 amino acids, having a molecular weight of about 17,000. TNFα is a cytokine produced from various cells including activated macrophages.

Although TNFα is a cytokine showing cytotoxicity against several kinds of tumor cells, subsequent studies revealed that TNFα has various other activities, and the activities are not only limited to tumor cells but extend to many other normal cells. The diverse effects of TNFα are markedly enormous. Examples of the TNFα activities are suppression of lipoprotein lipase activity in adipocytes; expression of human leukocyte antigens (HLA) on blood endothelial cells and fibroblasts; interleukin-1 production by fibroblasts or macrophages; activation of macrophages; induction of colony stimulating factor by fibroblasts, endothelial cells or some tumor cells; inhibition of synthesis of proteoglycans and stimulation of their resorption in cartilage; activation of neutrophils and generation of superoxide; production of procoagulant factor by blood endothelial cells; proliferation of fibroblasts; IL-6 production by fibroblasts; injury of blood endothelial cells; and the like. According to recent studies, TNFα is recognized as a cytokine involved broadly in vital protection through inflammation and immune response, as described in Vassalli, P., Ann. Rev. Immunol., 10, 411-452 (1992).

On the other hand, it has been found that continuous or excessive production of TNFα can result in extreme action on normal cells which can cause various diseases. It is also reported in Beutler B., Greenwald D., Hulmes J.D. et al., Nature, 316, 552-554 (1985), Kawakami, M., SEIKAGAKU (Biochemistry), 59, 1244-1247 (1987) that TNFa is also known as cachectin, which induces cachexia in cancer or infectious diseases (involving catabolic acceleration of total metabolism leading to extreme wasting).

TNFα is considered to be one of the causes of septic shock and there are many reports on the effectiveness of an anti-TNFα antibody in septic shock (Starnes, H.F. Jr., Pearce, M.K., Tewari, A., Yim, J.H., Zou, J.C., Abrams, J.S., J. Immunol., 145, 4185-4191 (1990), Beutler, B., Milsark, I.W., Cerami, A.C., Science, 229, 869-871 (1985), Hinshaw, L.B., Tekamp-Olson, P., Chang, A.C.K. et al., Circ. Shock, 30, 279-292 (1990)).

An increased level of TNFα is also found in the synovial fluid or blood from rheumatoid arthritis patients, as reported in Tetta, C., Camussi, G., Modena, V., Vittorio, C.D., Baglioni, C., Ann. Rheum. Dis., 49, 665-667 (1990).

In recent years, acquired immunodeficiency syndrome (AIDS) has been explosively prevalent all over the world. AIDS is a disease mainly caused by infection with human immunodeficiency virus-1 (HIV). HIV-infected patients are often complicated by opportunistic infections, Kaposi's sarcoma, neurological disorders (CNS disorders), and/or cachexia, etc., and those deseases often cause fatal.

HIV is a retrovirus classified in the subfamily of Lentiviruses. CD4-positive T lymphocytes and monocytes are the major cellular targets for HIV infection, but HIV also infects other various cells such as macrophages, glial cells and the like. HIV is integrated into the host cell chromosomes as a provirus DNA by means of viral enzymes such as reverse transcriptase and integrase. A new viral RNA is transcripted by the provirus. The viral RNA comprises regions encoding proteins constituting HIV and is translated into the proteins to produce new virions together with the viral RNA. The transcription of retroroviral RNA is mainly controlled by the virus-specific nucleotide sequence named a long terminal repeat (LTR) which is a sequence repeated upstream of the 5' end of integrated retroviral proviruses. The LTR region contains control sites for initiation and elongation of transcription, i.e., binding sites for a plurality of transcription factors in a host cell and transcription activators derived from viruses. Accordingly, it is considered that the transcription of HIV genes would be controlled depending upon the amount of these factors/activators present in the cells (Green, W.C., Annu. Rev. Immunol., 8, 453-475 (1990)).

It is known that in T cells or macrophages stimulated by, e.g., antigens, a lipopolysaccharide, which is one of the main constituents of bacteria, proinflammatory cytokines (TNFα, IL-1 or IL-6) or PMA, signal transduction pathways in cells are activated and unique sets of activated transcription factors increase in the nucleus to accelerate the transcription of HIV (Osborn, L. et al., Proc. Natl. Acad. Sci., USA, 86, 2336-2340 (1989), Poli, G. et al., J. Exp. Med., 172, 151-158 (1990), Poli, G. et al., Proc. Natl. Acad. Sci., USA, 87, 782-785 (1990)).

It is interesting to note that some of the binding sites of those transcription factors contained in the LTR sequence are also found in the enhancer/promoter region of viral genes, such as Rous sarcoma virus, human cytomegalovirus and simian virus, and in the enhancer/promoter region of various vital genes including TNFα (Shakhov, A.N., J. Exp. Med., 171, 35-47 (1990)). It is known that the corresponding transcription factors take some role in activation of the transcription of these genes. It is then suggested that some inhibitors of TNFα production may reduce not only the transcription of TNFα but HIV by interfering directly/indirectly with the activity of these transcriptional factors (Li, C.J. et al., Trends in Microbiology, 2(5), 164-169 (1994)).

On the other hand, an abnormally high concentration of TNFα in blood of AIDS patients is reported (Dezube, B.J. et al., J. Acquir, Immune Defic. Syndr. 5, 1099-1104 (1992), Wright, S.C. et al., J. Immunol, 141(1), 99-104 (1988)). The excessive production of TNFα in the AIDS patients is another evidence suggesting that TNFα would be associated with pathological conditions including cachexia, apoptosis of T cells, abnormal B cells proliferation, etc., in addition to the activity of accelerating HIV proliferation. By reducing the level (concentration) of TNFα in a host, an inhibitor of TNFα or an inhibitor of production and/or secretion of TNFα is therefore expected to show not only the activity of inhibiting HIV proliferation but the activity of improving the pathological conditions of AIDS described above.

In addition, there are many other diseases wherein a high concentration of TNFα is found in blood and/or tissue, e.g., osteoarthritis (Venn, G., Nietfeld, J.J., Duits, A.J., Brennan, F.M.. Arner, E., Covington, M., Billingham, M.E.J., Hardingham, T.E., Arthritis Rheum., 36(6), 819-826 (1993)); multiple sclerosis (Sharief, M.K., Hentges, R., N. Engl. J. Med., 325 (7), 467-472 (1991)); Kawasaki disease (Matsubara, T., Furukawa, S., Yabuta, K., Clin. Immunol. Immunopathol., 56, 29-36 (1990)); inflammatory bowel diseases such as ulcerative colitis or Crohn's disease (Murch, S., Walker-Smith, J.A., Arch. Dis. Child, 66, 561 (1991); Maeda, Y., SHOKAKI-TO-MEN-EKI (Digestive Organ and Immunity), 22, 111-114 (1989)); Beh et disease (Akoglu, T., Direskeneli, H., Yazici, H., Lawrence, R., J. Rheumatol., 17, 1107-1108 (1990)); systemic lupus erythematosus (SLE) (Maury, C.P.J., Teppo, A-M., Arthritis Rheum., 32, 146-150 (1989)); graft versus host disease (GvHD) (Nestel, F.P., Price, K.S., Seemayer, T.A., Lapp, W.S., J. Exp. Med., 175, 405-413 (1992)); multiple organ failure (Fujiwara, T., Kawakami, M., RINSHO-I (Clinician), 17 (10), 2006-2008 (1991)); malaria (Grau, G.E., Fajardo, L.F., Piguet, P.F., et al., Science, 237, 1210-1212 (1987)); meningitis (Waage, A., Halstensen, A., Espevik, T., Lancet, I, 355-357 (1987)); hepatitis (Sugano, K., KANZO (Liver), 33, 213-218 (1992)); non-insulin-dependent diabetes mellitus (NIDDM) (Hotamisligil, G.S., Shargill, N.S., Spiegelman, B.M., Science, 259, 87-91 (1993), asthma, adult respiratory distress syndrome (ARDS), tuberculosis, atherosclerosis, Alzheimer's disease, etc.

As seen from the above publications, it is understood that excessive production of TNFα sometimes adversely affects the living body. Therefore, further investigation is desired to develop an inhibitor of production and/or secretion of TNFα or TNFα inhibitors available for the treatment of these diseases.

As compounds and factors showing an activity of inhibiting TNFα, pentoxifylline, glucocorticoid, protease inhibitors, phospholipase A2 inhibitors, lipoxygenase inhibitors, platelet activating factor (PAF) antagonists, radical scavengers, prostaglandin F₂ or I₂, an anti-TNFα antibody, thalidomide, etc. are known. However, these compounds or factors are accompanied by side effects because of a diversity of their pharmacological activities. It is therefore required to develop highly safe compounds based on a novel mechanism.

TNFβ (lymphotoxin, LT) is also known as a factor belonging to the same family of TNFα. TNFβ is a glycoprotein composed of 171 amino acids, and has homology with TNFα both in gene structure and amino acid sequence. Further, it is known that both TNFβ and TNFα commonly share the same receptors and have identical biological activities. The enhancer/promoter regions at the upstream of TNFα and TNFβ genes have a common partial sequence to which the same transcriptional factors possibly bind. TNFβ may be produced by stimulation with antigen, phorbol ester and mitogen which are known to induce the production of TNFα. Thus, factors or compounds having an inhibitory activity on the production of TNFα may also exhibit an inhibitory activity on TNFβ (Jongeneel, C.V., Tumor Necrosis Factors; Beutler, B, Ed., Raven Press: New York, 1992, pp. 539-559, Poter, A.G., FEMS Microbiol. Immunol., 64, 193-200 (1990), Pauli, U., Critical Rev. Eukaryotic Gene Expression, 4 323-344 (1994)).

On the other hand, piperidinylpyrimidine derivatives are described in WO 9426733 and Japanese Patent KOKOKU No. 49-11710. However, these publications do not suggest any TNFα inhibitory activities. In Chem. Pharm. Bull., 34 (5), 1907-1916 (1986) the description, which is limited to use as intermediates for synthesis, discloses compounds represented by the following formula: 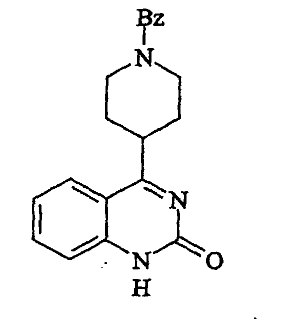 (wherein Bz is benzoyl.).
No utility of these compounds is disclosed anywhere in the report.

JP 59076082 discusses a group of quinazolinylpiperidines for use as antihypertensive agents.

### Disclosure of Invention

This invention provides a method for inhibiting the production and/or secretion of tumor necrosis factor, a pharmaceutical composition inhibiting the production. and/or secretion of tumor necrosis factor and novel piperidinylpyrimidine derivatives having an excellent activity of inhibiting the formation and/or secretion of TNFα.

The present invention relates to the use of a compound which is a piperidinylpyrimidine derivative of formula (1) 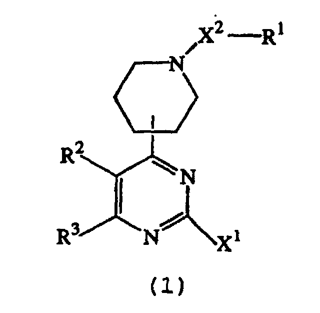 wherein X¹ is amino or hydroxy;
X² is carbonyl (-(CO)-), carbonyloxy (-(CO)O-), carbonylamino (-(CO)NH-), or sulfonyl (-(SO₂)-);
R¹ is an alkyl, a cycloalkyl, an aryl, a substituted aryl, a heteroaryl, or -(CH₂)ₘ- A, wherein m is 1, 2, 3, 4, 5, 6, 7, 8, 9 or 10, and A is a halogen atom, a cycloalkyl, hydroxy, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic, an aryl, a substituted aryl or a heteroaryl;
R² is hydrogen, an alkyl, or an aryl;
R³ is an alkyl, an aryl, a substituted aryl, a heteroaryl, or -(CH₂)_{m'}-A', wherein m' is 1, 2, 3 or 4, and A' is a halogen atom, a cycloalkyl, hydroxy, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic group, an aryl, a substituted aryl or a heteroaryl;
or R² and R³ are combined together with the pyrimidine ring to form quinazoline or pyridopyrimidine, wherein said quinazoline may be substituted with a substituent or substituents selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl and said pyridopyrimidine may be substituted in the 5, 6, 7 and/or 8 position with a substituent selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl;
wherein said aryl group of R¹, A, R², R³ or A' has at least one hydrocarbon ring having up to 10 carbon atoms; said substituted aryl group of R¹, A, R³ or A' is an aryl group substituted with one or more substituents selected from halogen; alkyl having 1 to 10 carbon atoms; alkoxy having 1 to 6 carbon atoms; nitro; cyano; carboxyl; hydroxy; amino; trifluoromethyl; alkylamino having 1 to 4 carbon atoms; dialkylamino having 2 to 8 carbon atoms; alkanoyl having 1 to 6 carbon atoms; alkanoylamino having 1 to 6 carbon atoms; sulfamoyl; alkylaminosulfonyl wherein the alkylamino thereof has 1 to 4 carbon atoms; dialkylaminosulfonyl wherein the dialkylamino thereof has 2 to 8 carbon atoms; alkoxycarbonyl wherein the alkoxy therefore has 1 to 6 carbon atoms; and substituents which are combined together to form methylenedioxy, ethylenedioxy or dihydroxyfuran; and
said heteroaryl group of R¹, A, R³ or A' comprises not more than 9 carbon atoms and 1 to 3 heteroatom(s) selected from nitrogen, oxygen and sulfur, which may be the same or different,
or a pharmaceutically acceptable salt thereof, in the manufacture of a medicament for inhibiting the production or secretion of tumor necrosis factor.

This invention also relates to a pharmaceutical composition comprising a compound represented by the formula (1): 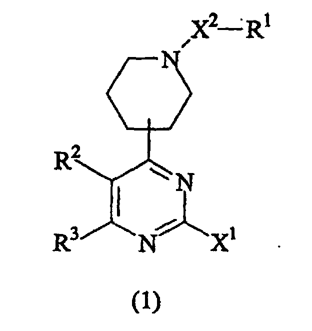 wherein X¹ is amino or hydroxy;
X² is carbonyl (-(CO)-), carbonyloxy (-(CO)O-), carbonylamino (-(CO)NH-), or sulfonyl (-(SO₂)-);
R¹ is an alkyl, a cycloalkyl, an aryl, a substituted aryl, a heteroaryl, or -(CH₂)ₘ- A, wherein m is 1, 2, 3, 4, 5, 6, 7, 8, 9 or 10, and A is a halogen atom, a cycloalkyl, hydroxy, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic, an aryl, a substituted aryl or a heteroaryl;
R² is hydrogen, an alkyl, or an aryl;
R³ is an alkyl, an aryl, a substituted aryl, a heteroaryl, or -(CH₂)_{m'}- A', wherein m' is 1, 2, 3, or 4, and A' is a halogen atom, a cycloalkyl, hydroxyl, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic, an aryl, a substituted aryl or a heteroaryl;
or R² and R³ are combined with the pyrimidine ring to form quinazoline, a substituted quinazoline, pyridopyrimidine or a substituted pyridopyrimidine; or a pharmaceutically acceptable salt thereof.

This invention also relates to a compound which is a piperidinylpyrimidine derivative of formula (1-a) 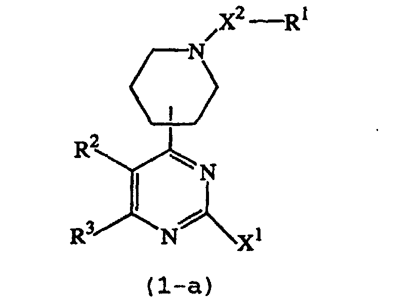 wherein
(1) X¹ is amino or hydroxy;
   X² is carbonyl (-(CO)-), carbonyloxy (-(CO)O-), carbonylamino (-(CO)NH-), or sulfonyl (-(SO₂)-);
   R¹ is an alkyl, a cycloalkyl, an aryl, a substituted aryl, a heteroaryl, or -(CH₂)ₘ- A, wherein m is 1, 2, 3, 4, 5, 6, 7, 8, 9, or 10, and A is a halogen atom, a cycloalkyl, hydroxyl, amino, cyano, nitro, carboxyl, an alkoxy, an alkylthio, an alkokycarbonyl, an aryloxycarbonyl, an alkyloxycarbonylamino, an alkylamino, a dialkylamino, a saturated heterocyclic, an aryl, a substituted aryl or a heteroaryl;
   R² is hydrogen, an alkyl, or an aryl;
   R³ is an alkyl, an aryl, a substituted aryl, a heteroaryl, or -(CH₂)_{m'}- A', wherein m is 1, 2, 3, or 4, and A is a halogen atom, a cycloalkyl, hydroxyl, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic, an aryl, a substituted aryl or a heteroaryl;
   or
(2) X¹ is amino;
   X² is carbonyl (-(CO)-);
   R¹ is a C₆₋₁₀ alkyl, a cycloalkyl, an aryl substituted by two or three halogen atoms, 2,3-methylenedioxyphenyl, 3,4-methylenedioxyphenyl, a C₇₋₁₅ aralkyl or -(CH₂)ₘ-A, wherein m is 6, 7, 8, 9 or 10, and A is a halogen atom, a cycloalkyl, hydroxyl, amino, cyano, carboxyl, an alkoxy, an alkoxycarbonyl, an aryloxycarbonyl, an alkyloxycarbonylamino, an alkylamino, a dialkylamino, a saturated heterocyclic, an aryl or a substituted aryl;
   R² and R³ are combined with the pyrimidine ring to form quinazoline, quinazoline substituted with a substituent or substituents selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl, pyridopyrimidine, or pyridopyrimidine substituted in the 5, 6, 7 and/or 8 position with a substituent selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl;
   wherein said aryl group of R¹, A, R², R³ or A' has at least one hydrocarbon ring having up to 10 carbon atoms; said substituted aryl group of R¹, A, R³ or A' is an aryl group substituted with one or more substituents selected from halogen; alkyl having 1 to 10 carbon atoms; alkoxy having 1 to 6 carbon atoms; nitro; cyano; carboxyl; hydroxy; amino; trifluoromethyl; alkylamino having 1 to 4 carbon atoms; dialkylamino having 2 to 8 carbon atoms; alkanoyl having 1 to 6 carbon atoms; alkanoylamino having 1 to 6 carbon atoms; sulfamoyl; alkylaminosulfonyl;
   wherein the alkylamino thereof has 1 to 4 carbon atoms; dialkylaminosulfonyl wherein the dialkylamino thereof has 2 to 8 carbon atoms; alkoxycarbonyl
   wherein the alkoxy therefore has 1 to 6 carbon atoms; and substituents which are combined together to form methylenedioxy, ethylenedioxy or dihydroxyfuran; and said heteroaryl group of R¹, A, R³ or A' comprises not more than 9 carbon atoms and 1 to 3 heteroatom(s) selected from nitrogen, oxygen and sulfur, which may be the same or different, or a pharmaceutically acceptable salt thereof.

Preferably, X¹ is amino. More preferably X¹ is amino, X² is carbonyl (-(CO)-), carbonyloxy (-(CO)O-), or carbonylamino (-(CO)NH-);
R¹ is cycloalkyl, aryl, substituted aryl, heteroaryl or -(CH₂)ₘ-A, wherein m is 1, 2, 3, 4, 5, 6, 7, 8, 9 or 10, and A is a halogen atom, cycloalkyl, hydroxyl, amino, cyano, nitro, carboxyl, alkoxy, alkylthio, alkoxycarbonyl, aryloxycarbonyl, alkyloxycarbonylamino, alkylamino, dialkylamino, a saturated heterocyclic group, aryl, substituted aryl or heteroaryl;
R² is hydrogen, alkyl, or aryl;
R³ is aryl, substituted aryl, heteroaryl, or -(CH₂)_{m'}-A', wherein m' is 1, 2, 3 or 4 and A' is a halogen atom, cycloalkyl, hydroxyl, amino, cyano, nitro, carboxyl, alkoxy, alkylthio, alkoxycarbonyl, aryloxycarbonyl, alkyloxycarbonylamino, alkylamino, dialkylamino, a saturated heterocyclic group, aryl, substituted aryl or heteroaryl. More preferably, X² is carbonyl (-(CO)-) or carbonylamino (-(CO)NH-).

A preferred compound is represented by the formula (1-b); 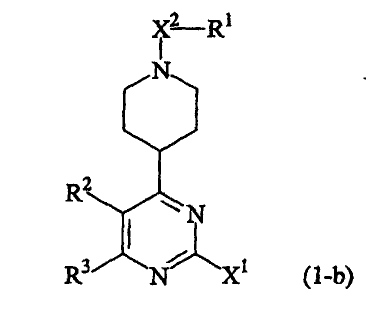 wherein X¹ is amino; X² is carbonyl (-(CO)-), carbonyloxy (-(CO)O-), or carbonylamino (-CONH-);
R¹ is an alkyl, a cycloalkyl, an aryl, a substituted aryl, a heteroaryl, or -(CH₂)ₘ- A, wherein m is 1, 2, 3, 4, 5, 6, 7, 8, 9 or 10, and A is a halogen atom, a cycloalkyl, hydroxy, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic group, an aryl, a substituted aryl or a heteroaryl;
R² is hydrogen or an alkyl;
R³ is an aryl, a substituted aryl, a heteroaryl, or -(CH₂)_{m'}- A', wherein m' is 1 or 2, and A' is an aryl, a substituted aryl or a heteroaryl;
or R² and R³ are combined with the pyrimidine ring to form quinazoline, a substituted quinazoline, pyridopyrimidine or a substituted pyridopyrimidine; or a pharmaceutically acceptable salt thereof.

A preferred compound is represented by formula (1-c), 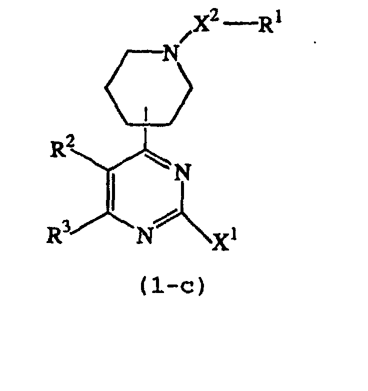 wherein X¹ is amino or hydroxy;
X² is carbonyl (-(CO)-), carbonyloxy (-(CO)O-), carbonylamino (-(CO)NH-), or sulfonyl (-(SO₂)-);
R¹ is an alkyl, a cycloalkyl, an aryl, a substituted aryl, a heteroaryl, or -(CH₂)ₘ- A, wherein m is 1, 2, 3, 4, 5, 6, 7, 8, 9, or 10, and A is a halogen atom, a cycloalkyl, hydroxyl, amino, cyano, nitro, carboxyl, an alkoxy, an alkylthio, an alkokycarbonyl, an aryloxycarbonyl, an alkyloxycarbonylamino, an alkylamino, a dialkylamino, a saturated heterocyclic, an aryl, a substituted aryl or a heteroaryl;
R² is hydrogen, an alkyl, or an aryl;
R³ is an alkyl, an aryl, a substituted aryl, a heteroaryl, or -(CH₂)_{m'}- A', wherein m' is 1, 2, 3, or 4, and A' is a halogen atom, a cycloalkyl, hydroxyl, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic, an aryl, a substituted aryl or a heteroaryl; wherein aryl, substituted aryl and heteroaryl are as defined above;
or a pharmaceutically acceptable salt thereof.

A preferred compound is represented by formula (1-d), 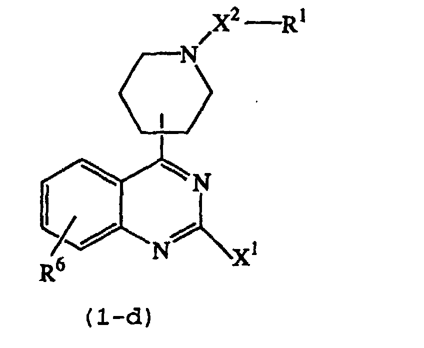 wherein X¹ is amino;
X² is carbonyl (-(CO)-);
R¹ is an C₆₋₁₀ alkyl, a cycloalkyl, an aryl substituted by two or three halogen atoms, 2, 3-methylenedioxyphenyl, 3, 4-methylenedioxyphenyl, a C₇₋₁₅ aralkyl or -(CH₂)ₘ- A, wherein m is 6, 7, 8, 9, or 10, and A is a halogen atom, a cycloalkyl, hydroxyl, amino, cyano, carboxyl, an alkoxy, an alkoxycarbonyl, an aryloxycarbonyl, an alkyloxycarbonylamino, an alkylamino, a dialkylamino, a saturated heterocyclic, an aryl or a substituted aryl;
R⁶ is a halogen atom, an alkyl, an alkoxy, nitro, cyano, carboxyl, hydroxyl, amino, trifluoromethyl, an alkylamino, a dialkylamino, an alkanoyl, an alkanoylamino, sulfamoyl, an alkylaminosulfonyl, a dialkylaminosulfonyl or an alkoxycarbonyl;
or a pharmaceutically acceptable salt thereof.

A more preferred compound is represented by formula (1-e), 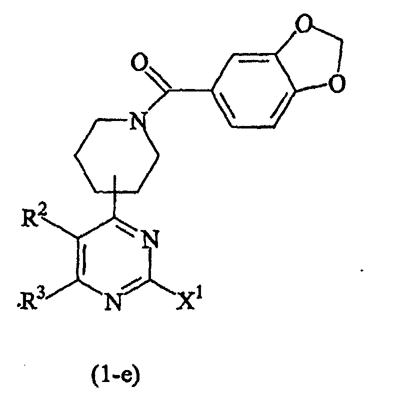 wherein X¹ is amino or hydroxy;
R² is hydrogen, an alkyl, or an aryl;
R³ is an alkyl, an aryl, a substituted aryl, a heteroaryl, or -(CH₂)_{m'}- A', wherein m' is 1, 2, 3 or 4, and A' is a halogen atom, a cycloalkyl, hydroxyl, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic group, an aryl, a substituted aryl or a heteroaryl;
or R² and R³ are combined with the pyrimidine ring to form quinazoline or pyridopyrimidine; wherein said quinazoline may be substituted with a substituent or substituents selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl and said pyridopyrimidine may be substituted in the 5, 6, 7 and/or 8 position with a substituent selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl,
wherein said aryl group of R¹, A, R², R³ or A' has at least one hydrocarbon ring having up to 10 carbon atoms; said substituted aryl group of R¹, A, R³ or A' is an aryl group substituted with one or more substituents selected from halogen; alkyl having 1 to 10 carbon atoms; alkoxy having 1 to 6 carbon atoms; nitro; cyano; carboxyl; hydroxy; amino; trifluoromethyl; alkylamino having 1 to 4 carbon atoms; dialkylamino having 2 to 8 carbon atoms; alkanoyl having 1 to 6 carbon atoms; alkanoylamino having 1 to 6 carbon atoms; sulfamoyl; alkylaminosulfonyl wherein the alkylamino thereof has 1 to 4 carbon atoms; dialkylaminosulfonyl wherein the dialkylamino thereof has 2 to 8 carbon atoms; alkoxycarbonyl wherein the alkoxy therefore has 1 to 6 carbon atoms; and substituents which are combined together to form methylenedioxy, ethylenedioxy or dihydroxyfuran; and
said heteroaryl group of R¹, A, R³ or A' comprises not more than 9 carbon atoms and 1 to 3 heteroatom(s) selected from nitrogen, oxygen and sulfur, which may be the same or different, or a pharmaceutically acceptable salt thereof.

A preferred embodiment included in the formula (1-e) is represented by the formula (1-f). 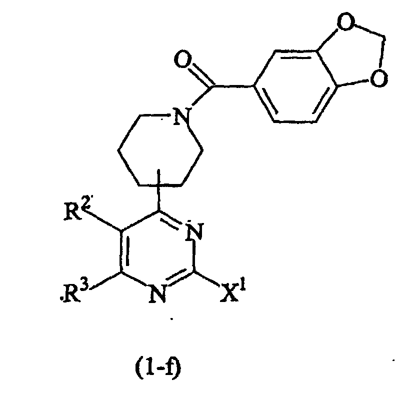 wherein X¹ is amino or hydroxy;
R² is hydrogen, an alkyl, or an aryl;
R³ is an alkyl, an aryl, a substituted aryl, a heteroaryl, or -(CH₂)_{m'}- A', wherein m' is 1, 2, 3 or 4, and A' is a halogen atom, a cycloalkyl, hydroxyl, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic group, an aryl, a substituted aryl or heteroaryl;
or R² and R³ are combined together with the pyrimidine ring to form quinazoline, a substituted quinazoline, a pyridopyrimidine or a substituted pyridopyrimidine; or a pharmaceutically acceptable salt thereof.

This invention also provides the use of the following compounds or a pharmaceutically acceptable salt thereof in the preparation of a medicament for inhibiting retrovirus long terminal repeat transcriptional activation in a patient in need of such inhibition, 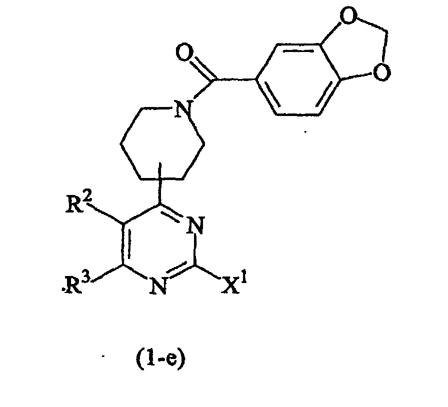 wherein X¹ is amino or hydroxy;
R² is hydrogen, an alkyl, or an aryl;
R³ is an alkyl, an aryl, a substituted aryl, a heteroaryl, or -(CH₂)_{m'},-A', wherein m' is 1, 2, 3 or 4, and A' is a halogen atom, a cycloalkyl, hydroxyl, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic group, an aryl, a substituted aryl or a heteroaryl;
or R² and R³ are combined with the pyrimidine ring to form quinazoline or pyridopyrimidine, wherein said quinazoline may be substituted with a substituent or substituents selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl and said pyridopyrimidine may be substituted in the 5, 6, 7 and/or 8 position with a substituent selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl,
wherein said aryl group of R¹, A, R², R³ or A¹ has at least one hydrocarbon ring having up to 10 carbon atoms; said substituted aryl group of R¹, A, R³ or A' is an aryl group substituted with one or more substituents selected from halogen; alkyl having 1 to 10 carbon atoms; alkoxy having 1 to 6 carbon atoms; nitro; cyano; carboxyl; hydroxy; amino; trifluoromethyl; alkylamino having 1 to 4 carbon atoms; dialkylamino having 2 to 8 carbon atoms; alkanoyl having 1 to 6 carbon atoms; alkanoylamino having 1 to 6 carbon atoms; sulfamoyl; alkylaminosulfonyl wherein the alkylamino thereof has 1 to 4 carbon atoms; dialkylaminosulfonyl wherein the dialkylamino thereof has 2 to 8 carbon atoms; alkoxycarbonyl wherein the alkoxy therefore has 1 to 6 carbon atoms; and substituents which are combined together to form methylenedioxy, ethylenedioxy or dihydroxyfuran; and said heteroaryl group of R¹, A, R³ or A' comprises not more than 9 carbon atoms and 1 to 3 heteroatom(s) selected from nitrogen, oxygen and sulfur, which may be the same or different.

This invention also provides the use of the following compounds or a pharmaceutically acceptable salt thereof in the preparation of a medicament for inhibiting human immunodeficiency virus-1 long terminal repeat transcriptional activation in a patient in need of such inhibition, 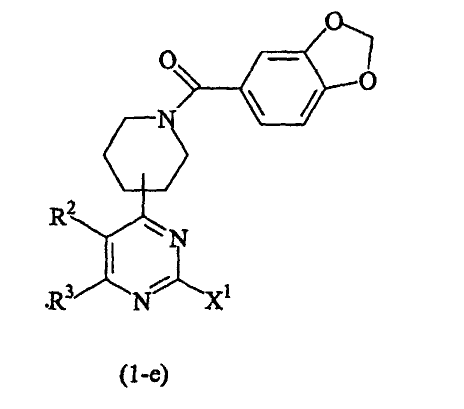 wherein X¹ is amino or hydroxy;
R² is hydrogen, an alkyl, or an aryl;
R³ is an alkyl, an aryl, a substituted aryl, a heteroaryl, or -(CH₂)_{m'}- A', wherein m' is 1, 2, 3 or 4, and A' is a halogen atom, a cycloalkyl, hydroxyl, amino, cyano, nitro, carboxyl, an alkoxy, an alkylthio, an alkoxycarbonyl, an aryloxycarbonyl, an alkyloxycarbonylamino, an alkylamino, a dialkylamino, a saturated heterocyclic, an aryl, a substituted aryl or a heteroaryl;
or R² and R³ are combined with the pyrimidine ring to form quinazoline, or pyridopyrimidine; wherein said quinazoline may be substituted with a substituent or substituents selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl and said pyridopyrimidine may be substituted in the 5, 6, 7 and/or 8 position with a substituent selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl,
wherein said aryl group of R¹, A, R², R³ or A' has at least one hydrocarbon ring having up to 10 carbon atoms; said substituted aryl group of R¹, A, R³ or A' is an aryl group substituted with one or more substituents selected from halogen; alkyl having 1 to 10 carbon atoms; alkoxy having 1 to 6 carbon atoms; nitro; cyano; carboxyl; hydroxy; amino; trifluoromethyl; alkylamino having 1 to 4 carbon atoms; dialkylamino having 2 to 8 carbon atoms; alkanoyl having 1 to 6 carbon atoms; alkanoylamino having 1 to 6 carbon atoms; sulfamoyl; alkylaminosulfonyl wherein the alkylamino thereof has 1 to 4 carbon atoms; dialkylaminosulfonyl wherein the dialkylamino thereof has 2 to 8 carbon atoms; alkoxycarbonyl wherein the alkoxy therefore has 1 to 6 carbon atoms; and substituents which are combined together to form methylenedioxy, ethylenedioxy or dihydroxyfuran; and
said heteroaryl group of R¹, A, R³ or A' comprises not more than 9 carbon atoms and 1 to 3 heteroatom(s) selected from nitrogen, oxygen and sulfur, which may be the same or different.

### Best Mode for Carrying Out the Invention

The functional groups in the present invention represented by the formulae (1), (1-a), (1-b), (1-c), (1-d), (1-e) and (1-f) are described below.

The term "alkyl" as used herein refers to a straight or branched chain hydrocarbon group having 1 to 10 carbon atoms. Examples of the alkyl are methyl, ethyl, propyl, butyl, pentyl, hexyl, heptyl, octyl, nonanyl, decanyl, 1-methylethyl, 1-methylpropyl, 2-methylpropyl, 1,1-dimethylethyl, 1-methylbutyl, 2-methylbutyl and 3-methylbutyl.

The term "C₆₋₁₀alkyl" as used herein refers to a straight chain hydrocarbon group having 6 to 10 carbon atoms. Examples of the C₆₋₁₀alkyl are hexyl, heptyl, octyl, nonanyl and decanyl.

The term "cycloalkyl" as used in herein refers to a saturated hydrocarbon group possessing at least one carbocyclic ring, the ring containing 3 to 7 carbon atoms. Examples of the cycloalkyl are cyclopropyl, cyclobutyl, cyclopentyl and cyclohexyl.

The term "alkenyl" as used herein refers to a hydrocarbon group having 2 to 10 carbon atoms possessing at least one double bond. Examples of the alkenyl are ethenyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl and 3-methyl-2-butenyl.

The term "alkoxy" as used herein refers to a monovalent substituent comprising an alkyl group having 1 to 6 carbon atoms linked through an ether oxygen having its free valence bond from the ether oxygen. Examples of the alkoxy are methoxy, ethoxy, propoxy, 1-methylethoxy, butoxy, 1-methylpropoxy, 2-methylpropoxy, pentyloxy, 1-methylbutoxy, 2-methylbutoxy, 3-methylbutoxy, 1,1-dimethylpropoxy, 1,2-dimethylpropoxy, 2,2-dimethylpropoxy, hexyloxy, 1-methylpentyloxy, 2-methylpentyloxy, 3-methylpentyloxy, 4-methylpentyloxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethyl-1-methylpropoxy and 1-ethyl-2-methylpropoxy.

The term "alkylthio" refers to a monovalent substituent comprising an alkyl group having 1 to 6 carbon atoms linked through an ether sulfur having its free valence bond from the ether sulfur. Examples of the alkylthio are methylthio, ethylthio, propylthio, 1-methylethylthio, butylthio, 1-methylpropylthio, 2-methylpropylthio, pentylthio, 1-methylbutylthio, 2-methylbutylthio, 3-methylbutylthio, 1,1-dimethylpropylthio, 1,2-dimethylpropylthio, 2,2-dimethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethyl-1-methylpropylthio and 1-ethyl-2-methylpropylthio.

The term "alkoxycarbonyl" refers to a monovalent substituent having the formula alkyl-O-(C=O)-. Examples of the alkoxycarbonyl are methoxycarbonyl, ethoxycarbonyl, propoxycarbonyl, 1-methylethoxycarbonyl, butoxycarbonyl and 1,1-dimethylethoxycarbonyl.

The term "alkylamino" refers to a monosubstituted derivative of ammonia, wherein a hydrogen of ammonia is replaced by an alkyl group having 1 to 4 carbon atoms and examples of the alkylamino are methylamino, ethylamino, propylamino, 1-methylethylamino, butylamino and 1,1-dimethylethylamino.

The term "dialkylamino" refers to a disubstituted derivative of ammonia, wherein two hydrogens of ammonia are replaced by alkyl groups having 1 to 4 carbon atoms and examples of the alkylamino are N,N-dimethylamino, N,N-diethylamino, N,N-dipropylamino, N-methyl-N-ethylamino, N,N-dibutylamino and N-methyl-N-(1,1-dimethylethyl)amino.

The term "aryl" as used herein refers to an aromatic hydrocarbon possessing at least one hydrocarbon ring having up to 10 carbon atoms and examples of the aryl are phenyl, 1-naphthyl and 2-naphthyl.

The term "aryloxycarbonyl" refers to a monovalent substituent having the formula aryl-O-(C=O)-. Examples of the group include aryloxycarbonyl groups having 7 to 13 carbon atoms, specifically exemplified by phenoxycarbonyl.

The term "alkyloxycarbonylamino" refers to a primary or secondary amine, which is replaced by a substituent having the formula alkyl-O-(C=O)- and an example of the alkyloxycarbonylamino is t-butoxycarbonylamino.

Examples of the halogen atom are fluorine, chlorine, bromine and iodine atoms.

The substituent of the substituted aryl may be at one or more of the carbons of the aromatic ring. If the aryl group has two or more substituents, the substituents may be same or different from each other. The substituent of the substituted aryl is halogen atom; an alkyl, an alkoxy, nitro, cyano, carboxyl, hydroxy, amino, trifluoromethyl, an alkylamino, a dialkylamino, an alkanoyl, an alkanoylamino, sulfamoyl, an alkylaminosulfonyl, a dialkylaminosulfonyl or, an alkoxycarbonyl. Furthermore, the substituents adjacent to each other may be combined together to form an alkylenedioxy group such as 2,3-methylenedioxy, 3,4-methylenedioxy, 2,3-ethylenedioxy, 3,4-ethylenedioxy, or to form a heterocyclic group fused with an aryl ring such as 2,3-dihydrobenz[b]furan.

The term "alkanoyl" refers to the radical formed by removal of the hydroxyl function from a straight or branched alkanoic acid having 1 to 6 carbon atoms and examples are formyl, acetyl, propionyl, butyryl, valeryl, isovaleryl, pivaloyl and hexanoyl.

The term "alkanoylamino" refers to a primary or secondary amine, wherein a hydrogen of the amine is replaced by an alkanoyl group having 1 to 6 carbon atoms. Examples of the alkanoylamino are formylamino, acetylamino, propionylamino, butyrylamino, valerylamino, isovalerylamino, pivaloylamino and hexanoylamino.

The term "alkylaminosulfonyl" refers to a monovalent substituent comprising an alkylamino group linked through a sulfonyl group having its free valence bond from the sulfur atom and examples of the alkylaminosulfonyl are methylaminosulfonyl, ethylaminosulfonyl, propylaminosulfonyl, 1-methylethylaminosulfonyl, butylaminosulfonyl and 1,1-dimethylethylaminosulfonyl.

The term "dialkylaminosulfonyl" refers to a monovalent substituent comprising an dialkylamino group linked through the sulfonyl group having its free valence bond-from the sulfur atom and examples of the dialkylaminosulfonyl are N,N-dimethylaminosulfonyl, N,N-diethylaminosulfonyl, N,N-dipropylaminosulfonyl, N-methyl-N-ethylaminosulfonyl, N,N-dibutylaminosulfonyl and N-methyl-N-(1,1-dimethylethyl)aminosulfonyl.

The term "heteroaryl" refers to an unsaturated aromatized heterocyclic group such as a monocyclic or bicyclic heteroaryl group. The heteroaryl group comprises not more than 9 carbon atoms, and 1 to 3 hetero atom(s) selected from nitrogen, oxygen and sulfur, which may be the same or different. Examples of heteroaryl are 2-pyridyl, 3-pyridyl, 4-pyridyl, 2-pyrimidinyl, 4-pyrimidinyl, 5-pyrimidinyl, 2-thiazolyl, 4-thiazolyl, 5-thiazolyl, 2-oxazolyl, 4-oxazolyl, 5-oxazolyl, 3-isoxazolyl, 4-isoxazolyl, 5-isoxazolyl, 3-isothiazolyl, 4-isothiazolyl, 5-isothiazolyl, 2-furyl, 3-furyl, 2-imidazolyl, 4-imidazolyl, 2-thienyl, 3-thienyl, 2-pyrrolyl, 3-pyrrolyl, 1-pyrazolyl, 3-pyrazolyl, 4-pyrazolyl, 2-pyrazinyl, 3-pyridazinyl, 4-pyridazinyl, 1H-1,2,4-triazol-1-yl, 1H-1,2,4-triazol-3-yl, 1H-1,2,4-triazol-5-yl, 3-oxadiazolyl, 5-oxadiazolyl, 2-thiazolyl, 4-thiazolyl, 5-thiazolyl, 4-benzo[b]furan, 5-benzo[b]furan, 6-benzo[b]furan, 7-benzo[b]furan, 4-benzimidazole, 5-benzimidazole, 4-benzothiazole, 5-benzothiazole, 6-benzothiazole, 7-benzothiazole, 4-benzoxazole, 5-benzoxazole, 6-benzoxazole and 7-benzoxazole.

The term "saturated heterocyclic" refers to a monocyclic saturated heterocyclic group comprising not more than 6 carbon atoms and one or two hetero atom(s) selected from nitrogen, oxygen and sulfur, which may be the same or different, and preferred embodiments include a 5- or 6-membered saturated heterocyclic group. Examples of the 5-membered saturated heterocyclic group are 1-pyrrolidinyl, 2-pyrrolidinyl, 3-pyrrolidinyl, 2-oxoranyl, 3-oxoranyl, 2-thioranyl and 3-thioranyl. Examples of the 6-membered saturated heterocyclic group are piperidino, 2-piperidyl, 3-piperidyl, 4-piperidyl, 1-piperazinyl, 2-piperazinyl, 2-tetrabydropyranyl, 3-tetrahydropyranyl, 4-tetrahydropyranyl, morpholino, 2-morpholinyl and 3-morpholinyl.

The monocyclic saturated heterocyclic group may be substituted with alkyl.

The aromatic group fused together with the pyrimidine ring forms a group having a benzopyrimidine (quinazoline) structure. The fused aromatic group may be substituted with a substituent or substituents selected from a halogen atom, an alkyl, an alkoxy, nitro, cyano, carboxyl, hydroxy, amino, trifluoromethyl, an alkylamino, a dialkylamino, an alkanoyl, an alkanoylamino, sulfamoyl, an alkylaminosulfonyl, a dialkylaminosulfonyl and an alkoxycarbonyl.

The heteroaryl group fused with the pyrimidine ring forms a group having a pyridopyrimidine structure. Specific examples of such a group include pyrido[2,3-d]pyrimidine, pyrido[3,4-d]pyrimidine, pyrido[4,3-d]pyrimidine and pyrido[3,2-d]pyrimidine. The 5, 6, 7 and 8 positions may each be substituted by a substituent selected from a halogen atom, an alkyl, an alkoxy, nitro, cyano, carboxyl, hydroxy, amino, trifluoromethyl, an alkylamino, a dialkylamino, an alkanoyl, an alkanoylamino, sulfamoyl, an alkylaminosulfonyl, a dialkylaminosulfonyl and an alkoxycarbonyl.

Examples of the pharmaceutically acceptable salt of the invention are a salt with a mineral acid such as hydrogen chloride, hydrogen bromide, sulfuric acid or phosphoric acid; a salt with an organic carboxylic acid such as formic acid, acetic acid, fumaric acid, maleic acid, malic acid, tartaric acid, aspartic acid or glutamic acid; a salt with a sulfonic acid such as methanesulfonic acid, benzenesulfonic acid, p-toluenesulfonic acid, hydroxybenzenesulfonic acid or dihydroxybenzenesulfonic acid; a salt with an alkali metal such as sodium or potassium; a salt with an alkaline earth metal such as calcium or magnesium; and a salt with an organic base such as trimethylamine, triethylamine, pyridine or ammonia.

The compound of the present invention may have a tautomeric isomer, a stereoisomer, a geometrical isomer or an optical isomer. The present invention includes these isomers.

The compound of the present invention may have hydrates, solvates or crystalline forms. The present invention also includes these forms.

This invention provides the use of a compound of formula (1), (1-a), (1-b), (1-c), (1-d) (1-e) or (1-f) in the manufacture of a medicament for treating a patient having a high concentration of TNFα in blood and/or tissue, such as septic shock, osteoarthritis, multiple sclerosis, inflammatory bowel diseases such as ulcerative colitis or Crohn's disease, Behçet disease, systemic lupus erythematosus (SLE), graft versus host disease (GvHD), multiple organ failure, malaria, meningitis, hepatitis, non-insulin-dependent diabetes mellitus, asthma, adult respiratory distress syndrome (ARDS), tuberculosis, atherosclerosis or Alzheimer's disease.

The medicament is for the treatment not only of humans but also of mammals or animals.

This invention also provides the use of a compound of formula (1-e) or (1-f) in the manufacture of a medicament for inhibiting retrovirus long terminal repeat transcriptional activation in a mammal or an animal in need of such inhibition.

Examples of the above retrovirus are HIV-I (human immunodeficiency virus-1), HTLV-I (human adult T-cell leukemia-I virus), HTLV-II (human adult T-cell leukemia-II virus), ALV (avian leukemia virus), RSV (Rous sarcoma virus), Mason-Phizer mammary tumor-associated virus, simian T-cell lymphotropic virus type I, simian T-cell lymphotropic virus type III, feline leukemia virus, equine infectious anemia virus, caprine arthritis and encephalitis virus.

The pharmaceutical composition of the present invention can be administered by conventional dosage forms such as oral, parenteral (including intramuscular, subcutaneous and intravenous), rectal, nasal, topical or implant. The formulations may, where appropriate, be prepared by any of the methods well known in the art of pharmaceutics. All methods include the step of bringing into association the active compound with liquid carriers or finely divided solid carriers or both and then, if necessary, shaping the product into a desired formulation.

For oral administration, the pharmaceutical compositions may take the form of, for example, tablets, capsules(each of which includes sustained release or timed release formulations,), pills, powders, cachets and suppositories. Examples of liquid oral dosage forms are solutions and suspensions. Examples of parenteral preparations are sterile solutions, suspensions, and other preparations prepared by conventional means with pharmaceutically acceptable excipients such as binding agents (e.g. pregelatinised maize starch, polyvinylpyrrolidone, hydroxypropylmethylcellulose, etc.); fillers (e.g. lactose, microcrystalline cellulose, calcium phosphate, etc.); lubricants (e.g. magnesium stearate, talc, silica, etc.); disintegrants (e.g. potato starch, sodium starch glycollate, etc.); or wetting agents (e.g. sodium lauryl sulphate etc.).

Liquid preparations for oral administration may take the form of, for example, solutions, syrups, or suspensions, or they may be presented as a dry product for constitution with water or other suitable vehicle before use. Such liquid preparations may be prepared by conventional means with pharmaceutical acceptable additives such as suspending agents (e.g. sorbitol syrup, methyl cellulose or hydrogenated edible fats, etc.); emulsifying agents (e.g. lectin, acacia, etc.); non-aqueous vehicles ( e.g. almond oil, oily esters, ethyl alcohol, etc.); and preservatives (e.g. methyl or propyl-, p-hydroxybenzoates, sorbic acid, etc.).

For topical administration, the pharmaceutical compositions may take the form of buccal or sublingual tablets, drops or lozenges formulated in a conventional manner.

For topical administration to the epidermis, the compounds may be formulated as creams, gels, ointments, lotions or as transdermal patches. Such compositions may, for example, be formulated with an aqueous or oily base with the addition of suitable thickening, gelling, emulsifying, stabilizing, dispersing, suspending, and/or coloring agents.

The active compounds of the invention may also be formulated as depot preparations. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example as a sparingly soluble salt.

The active compounds of the invention may be formulated for parenteral administration by injection, conveniently intravenous, intramuscular or subcutaneous injection, for example by bolus injection or continuous intravenous infusion. Formulations for injection may be presented in unit dosage form e.g. in ampoules or multidose containers, with added preservatives. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g. sterile pyrogen-free water, before use.

The active compounds of the invention may also be formulated in rectal compositions, such as suppositories or retention enemas, e.g. containing conventional suppository bases such as cocoa butter or other glyceride.

For intranasal administration the active compounds may be used, for example, as a liquid spray, as a powder or in the form of drops.

For administration by inhalation the active compounds are conveniently delivered in the form of an aerosol spray presentation from pressurized packs or a nebuliser, with the use of a suitable propellant, e.g. dichlorodifluoromethane, trichlorofluoromethane, dichlorotetrafluoroethane, 1,1,1,2-tetrafluoroethane, carbon dioxide or other suitable gas. In the case of pressurized aerosol, the dosage unit may be determined by providing a valve to deliver a metered amount. Capsules and cartridges of e.g. gelatin for use in an inhaler or insufflator may be formulated containing a powder mix of an active compound of the invention and a suitable powder base such as lactose or starch.

A convenient unit dose formulation contains the active ingredient in an amount of from about 1 to about 1000 mg.

Any of the pharmaceutical compositions described above may be presented in a conventional manner associated with controlled release forms.

It will be appreciated that the amount of a compound of the formula (1), (1-a), (1-b), (1-c), (1-d), (1-e), or (1-f) required for use in treatment will vary not only with the particular compound selected, but also with the route of administration, the nature of the condition being treated and the age, weight and condition of the patient and ultimately be at the discretion of the attendant physician or veterinarian. In general, the daily dose of the active compound of the invention for adult is selected from the range of from about 10 to about 1000 mg, preferably about 10 to about 500 mg for oral administration and, or from the range of from about 1 to about 100 mg, by single administration or by dividing the dose several times.

The piperidinylpyrimidine derivatives of the present invention may be prepared, e.g., by the following processes. 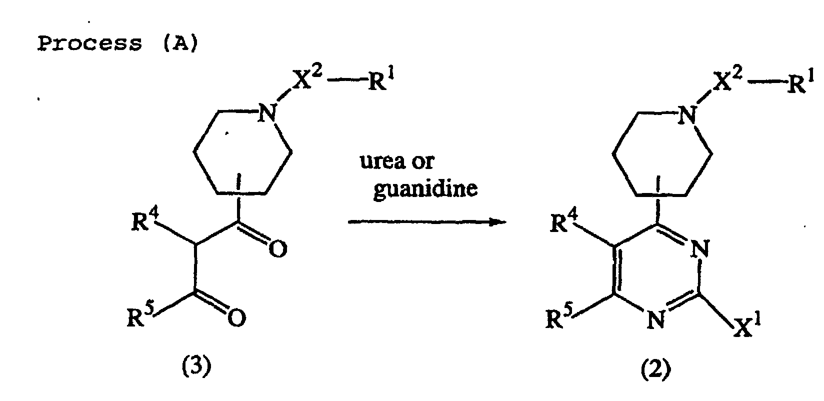 wherein:
X¹, X² and R¹ have the same meaning as defined in the formula 1;
R⁴ is hydrogen atom, an alkyl or an aryl; and,
R⁵ is an alkyl group, an aryl, a heteroaryl or a group represented by the formula: -(CH₂)_{m'}-A', wherein aryl, heteroaryl, m' and A' have the same meaning as defined in the formula (1).

A compound represented by the formula (2) is included by the formula (1). The compound represented by the formula (2) may be prepared by treating a 1,3-diketone compound represented by the formula (3) with urea or guanidine either in the presence or absence of a base. The reaction may be carried out either in the absence or presence of an inert solvent at a temperature ranging from about room temperature to about 200°C. In the reaction above, examples of the base are an alkali metal alkoxide such as potassium t-butoxide, sodium methoxide or sodium ethoxide; an alkali metal hydride such as sodium hydride; an organic base such as triethylamine, pyridine, 4-dimethylaminopyridine or N-methylmorpholine; an inorganic salt such as potassium carbonate, sodium carbonate or sodium hydrogen carbonate.

Examples of the inert solvent are an alcohol such as methanol, ethanol or t-butanol ; an aprotonic solvent such as pyridine, N,N-dimethylformamide dimethylsulfoxide, hexamethylphosphoramide or acetonitrile; a halogenated hydrocarbon such as dichloromethane, chloroform or 1,2-dichloroethane; a hydrocarbon such as benzene, toluene or hexane; and an ether such as tetrahydrofuran, dioxane or diethyl ether.

The starting compound represented by the formula (3) may be prepared by reacting a ketone derivative represented by the formula (4) with an acid chloride or an ester represented formula (5): 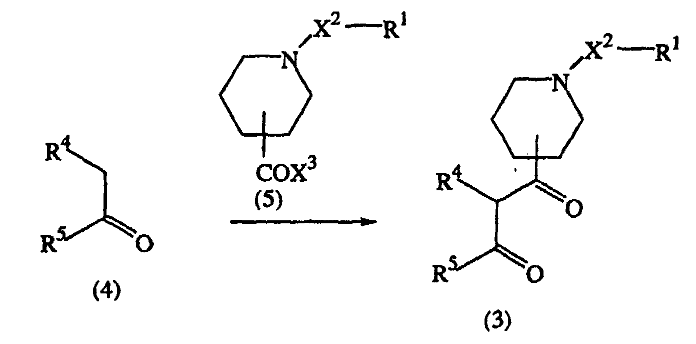 wherein X², R¹, R⁴ and R⁵ have the same meaning as defined in formula (2) and X³ is chlorine atom or alkoxy.

The reaction may be carried out in the presence of a base in an inert solvent at a temperature ranging from about -78°C to about reflux temperature. Examples of the base are an alkali metal alkoxide such as potassium t-butoxide, sodium methoxide or sodium ethoxide; a lithium compound such as n-butyl lithium or lithium N,N-diisopropylamide; a metal hydride such as sodium hydride.

Examples of the solvents are an alcohol such as methanol, ethanol or t-butanol ; an ether such as diethyl ether or tetrahydrofuran.

The starting compounds (5) may be prepared from isonipecotic acid, nipecotic acid, pipecolinic acid or a derivative thereof by a conventional method known to a skilled person in the art of organic chemistry.

A 1,3-diketone compound may also be prepared by the following processes. 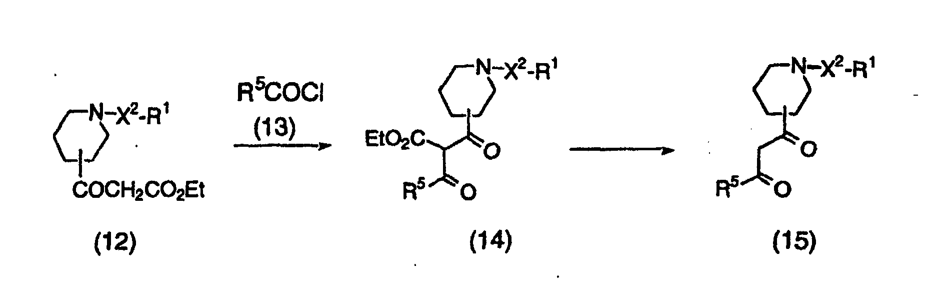 Wherein X² and R⁵ are as defined in the formula (2).

The compound of the formula (14) may be prepared by treating a β-ketoester of the formula (12) with an acid chloride of the formula (13). The reaction is carried out in the presence of a suitable base such as an alkaline metal hydride (e.g. sodium hydride), or an alkaline earth metal alkoxide (e.g. magnesium ethoxide) in an inert solvent such as an aprotonic solvent (e.g. N,N-dimethylformamide or dimethylsulfoxide), an ether (e.g. tetrahydrofuran or diethylether) or a hydrocarbon (e.g. benzene, toluene or hexane), at a temperature ranging from about 0°C to about reflux temperature of the solvent.

The acid chloride (13) may be prepared by treating the corresponding acid with a chlorinating agent using the reactions and techniques well known to a skilled person in the art of organic chemistry. Selected examples of the chlorinating agent include but are not limited to oxalyl chloride, thionyl chloride, phosphorous oxychloride, phosphorous trichloride, and phosphprous pentachloride. The reaction may be carried out either in the absence of solvent or in an inert solvent such as a halogenated hydrocarbon (e.g. dichloromethane or dichloroethane), an ether (e.g. diethylether or tetrahydrofuran), or hydrocarbon (e.g. benzene, toluene or hexane), at a temperature ranging from 0°C to reflux temperature of the solvent either in the absence or presence of base. Selected examples of the base include but are not limited to pyridine, 4-dimethylaminopyridine, triethylamine and imidazole.

The compound of the formula (15) may be prepared by deesterizing the compound of formula (14). The deesterizing reaction may be carried out in a solvent such as an aprotonic solvent (e.g. N,N-dimethylformamide or dimethylsulfoxide), or an acidic solvent (e.g. acetic acid) either in the presence or absence of water at a temperature ranging from room temperature to reflux temperature. 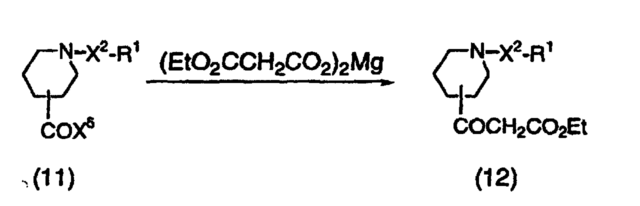 (Wherein X² and R¹ have the same meaning described in the formula (2). X⁵ is a chlorine atom, imidazole or a alkoxycarbonyloxy.)

The β-ketoester of the formula (12) may be prepared by treating an isonipecotic acid derivative of the formula (11) with magnesium monoethylmalonate. The reaction is carried out in an inert solvent such as an aprotonic solvent (e.g. N,N-dimethylformamide or dimethylsulfoxide), an ether (e.g. tetrahydrofuran or diethylether), or a hydrocarbon (e.g. benzene, toluene or hexane), at a temperature ranging from 0°C to reflux temperature of the solvent.

### Process (B)

With respect to a compound within the formula (1) wherein R² and R³ are combined with pyrimidine to form a pyridopyrimidine or a quinazoline, the process may be explained by using an example compound of formula (6) having the quinazoline structure. 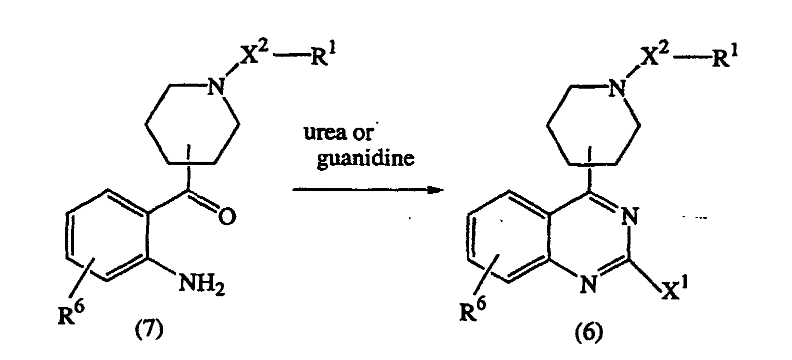 wherein:
X¹, X² and R¹ have the same significance as defined in the formula (1-d);
   R⁶ represents a substituent at the 5, 6, 7 or 8 position of the quinazoline ring. The compound (6) may have one R⁶ or up to 4 R⁶'s. If the compound (6) has more than two R⁶'s, each R⁶ may be the same or different. R⁶ is a halogen, an alkyl, an alkoxy, nitro, cyano, carboxyl, hydroxy, amino, trifluoromethyl, an alkylamino, a dialkylamino, an alkanoyl, an alkanoylamino, sulfamoyl, an alkylaminosulfonyl, a dialkylamino or an alkoxycarbonyl.

A compound of the formula (6) may be prepared by treating an aminoketone compound represented by the formula (7) with a reagent such as urea, sodium cyanate, potassium cyanate, guanidine or cyanamide. The reaction may be carried out either in the absence or presence of an inert solvent, either in the presence or absence of a base, at a temperature ranging from room temperature to 200°C.

In the reaction described above, examples of the base are an alkali metal alkoxide such as potassium t-butoxide, sodium methoxide or sodium ethoxide; a metal hydride such as sodium hydride; an organic amine such as triethylamine, pyridine, 4-dimethylaminopyridine or N-methylmorpholine; an inorganic salt such as potassium carbonate, sodium carbonate or sodium hydrogencarbonate.

Examples of the solvent are an alcohol such as methanol, ethanol or t-butanol; an aprotonic polar solvent such as pyridine, N,N-dimethylformamide, dimethylsulfoxide, hexamethylphosphoramide or acetonitrile; a halogenated hydrocarbon such as dichloromethane, chloroform or 1,2-dichloroethane; a hydrocarbon such as benzene, toluene or hexane; an ether such as tetrahydrofuran, dioxane or diethyl ether.

The starting compounds (7) may be prepared by treating an aniline derivative of the formula (8) with a nitrile or an acid chloride of the formula (9). 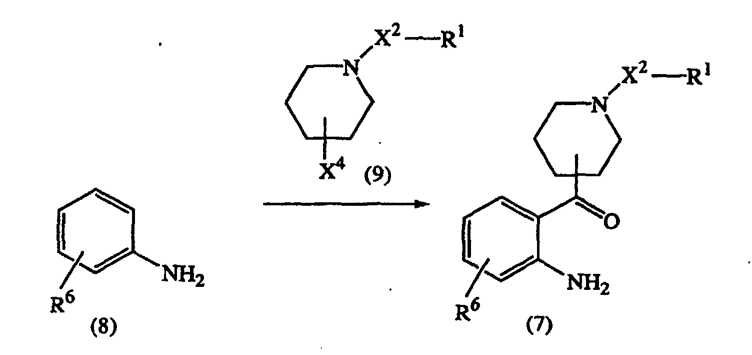 wherein X², R¹ and R⁶ have the same meaning as defined in the formula (1-d) and X⁴ is cyano or -COCl. The reaction may be carried out in an inert solvent in the presence of a Lewis acid at a temperature ranging from room temperature to reflux temperature.

Examples of the Lewis acid are aluminum chloride, tin tetrachloride, titanium tetrachloride, zinc dichloride and boron trichloride. Two or more Lewis acids in combination may be applied to accelerate the reaction.

Examples of the solvent are a halogenated hydrocarbon such as dichloromethane, chloroform or 1,2-dichloroethane; a hydrocarbon such as benzene, toluene or hexane; an ether such as tetrahydrofuran, dioxane or diethyl ether; and carbon disulfide.

The starting compounds (9) may be prepared from isonipecotinic acid, nipecotic acid, pipecolinic acid or a derivative thereof by a method well known to a skilled person in the art of organic chemistry. 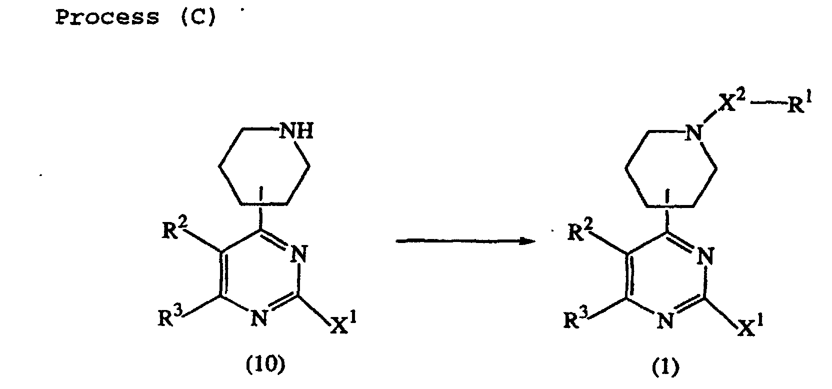 wherein X¹, X², R¹, R² and R³ have the same meaning as defined in the formula (1).

The piperidinylpyrimidine derivative of the formula (1) may be prepared by introducing the corresponding substituent into a piperidine compound of the formula (10).

A compound of formula (1), wherein X² is -(CO)-, may be prepared by treating a compound of the formula (10) with a corresponding acid anhydride or acid chloride. The reaction may be carried out in an inert solvent in the presence of a condensing reagent at a temperature ranging from 0°C to reflux temperature.

A compound of formula (1), wherein X² is -(CO)-, may also be prepared by treating a compound of the formula (10) with a corresponding acid. The reaction may be carried out in an inert solvent in the presence of a condensing reagent at a temperature ranging from 0°C to reflux temperature.

A compound of formula (1), wherein X² is -(CO)O-, may be prepared by treating a compound of the formula (10) with a corresponding chloroformate. The reaction may be carried out in an inert solvent in the presence of a base at a temperature ranging from 0°C to reflux temperature.

A compound of formula (1), wherein X² is -(CO)NH-, may be prepared by treating a compound of the formula (10) with a corresponding isocyanate. The reaction may be carried out in an inert solvent at a temperature ranging from 0°C to reflux temperature.

A compound of formula (1), wherein X² is -(SO₂)-, may be prepared by treating a compound of the formula (10) with a corresponding sulfonic acid chloride. The reaction may be carried out in an inert solvent in the presence of a base at a temperature ranging from 0°C to reflux temperature.

In the reaction described above, examples of the base are an organic amine such as triethylamine, pyridine, 4-dimethylaminopyridine or N-methylmorpholine; and an inorganic salt such as potassium carbonate, sodium carbonate or sodium hydrogen carbonate.

Examples of the solvent are an aprotonic polar solvent such as N,N-dimethylformamide, dimethylsulfoxide, hexamethylphosphoramide or acetonitrile; a hydrocarbon such as benzene, toluene or hexane; a halogenated hydrocarbon such as dichloromethane, chloroform or 1,2-dichloroethane and the like; and an ether such as tetrahydrofuran, dioxane or diethyl ether.

Examples of the condensing reagent are N,N'-dicyclohexylcarbodiimide and 1-ethyl-3-(3-dimethylaminopropyl)carbodiimide.

The compound of formula (10) may be prepared by hydrolyzing a compound of formula (11): 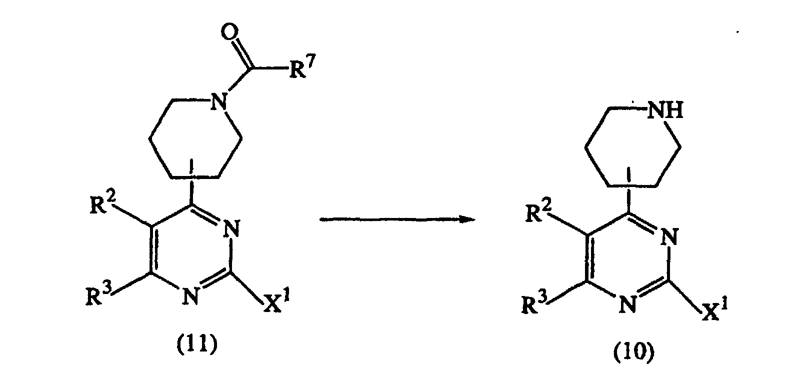 wherein X¹, R² and R³ have the same meaning as defined in the formula (1) and R⁷ is an alkyl or an aryl.

The hydrolysis above may be carried out in a solvent mixture of an aqueous solution of potassium hydroxide or sodium hydroxide and an alcohol such as ethanol, ethylene glycol or methoxyethanol, or an ether such as 1,4-dioxane or tetrahydrofuran, at a temperature ranging from room temperature to the reflux temperature.

In the reactions described above, where the substituent R¹, R², R³, R⁴ or R⁵ contains a residue such as amino, an alkyl amino or hydroxy, the residue may be protected prior to the reaction and the protective group is then deprotected to prepare the desired compound. A man skilled in the art of organic chemistry may choose an appropriate protective group from groups described below depending on the nature or condition of reactions or processes. Examples of the protective group available are an alkanoyl such as acetyl or benzoyl for protecting the amino and alkylamino groups, and an alkanoyl such as acetyl or benzoyl, alloyl, benzyl, methyl, methoxymethyl or trimethylsilyl for protecting the hydroxy group. The details of the protective groups and procedures are described in T.W. Greene, "Protective Groups in Organic Synthesis", John Wiley & Sons Inc. (1981).

Following are typical compounds of the invention, wherein the symbol "Ph" is phenyl and "Boc" is t-butoxycarbonyl.

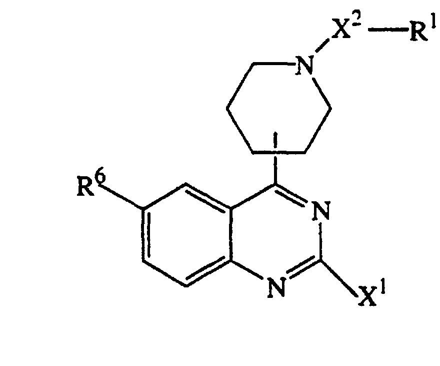

**Table 1**

| X² | R¹ | R⁶ | X² | R¹ | R⁶ |
|---|---|---|---|---|---|
| CO | cyclohexyl | H | CO | 2-fluorophenyl | H |
| | cyclohexylmethyl | | | 3-fluorophenyl | |
| | -CH₃ | | | 4-fluorophenyl | |
| | -CH₂CH₃ | | | 2-methylphenyl | |
| | -(CH₂)₂CH₃ | | | 3-methylphenyl | |
| | -(CH₂)₃CH₃ | | | 4-methylphenyl | |
| | -(CH₂)₄CH₃ | | | 2-furyl | |
| | -(CH₂)₅CH₃ | | | 3-furyl | |
| | - (CH₂)₆CH₃ | | | 2-pyridyl | |
| | -(CH₂)₇CH₃ | | | 3-pyridyl | |
| | - (CH₂)₈CH₃ | | | 4-pyridyl | |
| | - (CH₂)₉CH₃ | | | 2,3-dimethoxyphenyl | |
| | -(CH₂)₅OH | | | 2,4-dimethoxyphenyl | |
| | -(CH₂)₆OH | | | 2,5-dimethoxyphenyl | |
| | -(CH₂)₇OH | | | 2,6-dimethoxyphenyl | |
| | -(CH₂)₈OH | | | 3,4-dimethoxyphenyl | |
| | -(CH₂)₉OH | | | 3, 5-dimethoxyphenyl | |
| | phenyl | | | 2,3-dichlorophenyl | |
| | 2-methoxyphenyl | | | 2,4-dichloropheny | |
| | 3-methoxyphenyl | | | 2,5-dichlorophenyl | |
| | 4-methoxyphenyl | | | 2,6-dichlorophenyl | |
| | 2-chlorophenyl | | | 3,4-dichlorophenyl | |
| | 3-chlorophenyl | | | 2,4-dinitrophenyl | |
| | 4-chlorophenyl | | | 3,4-methylenedioxyphenyl | |
| CO | 2,3-methylenedioxyphenyl | H | CO | 2,5-dimethoxybenzyl | H |
| | 1-naphthyl | | | 3,4-dimethoxybenzyl | |
| | 2-naphthyl | | | 3,5-dimethoxybenzyl | |
| | 2,3,4-trimethoxyphenyl | | | 2,4-dichlorobenzyl | |
| | 2,3,6-trimethoxyphenyl | | | 2,6-dichlorobenzyl | |
| | 2,4,5-trimethoxyphenyl | | | 3,4-dichlorobenzyl | |
| | 2,4,6-trimethoxyphenyl | | | 2,4-dinitrobenzyl | |
| | 3,4,5-trimethoxyphenyl | | | 3, 4-methylenedioxybenzyl | |
| | 2,3,5-trichlorophenyl | | | 2,3-methylenedioxybenzyl | |
| | 2,4,6-trichlorophenyl | | | 3,4,5-trimethoxybenzyl | |
| | benzyl | | | 2-phenylethyl | |
| | 2-methoxybenzyl | | | 2-(2-methoxyphenyl)ethyl | |
| | 3-methoxybenzyl | | | 2-(3-methoxyphenyl)ethyl | |
| | 4-methoxybenzyl | | | 2-(4-methoxyphenyl)ethyl | |
| | 2-chlorobenzyl | | | 2-(2,3-dimethoxyphenyl)ethyl | |
| | 3-chlorobenzyl | | | 2-(3,4-dimethoxyphenyl)ethyl | |
| | 4-chlorobenzyl | | | | |
| | 2-fluorobenzyl | | | | |
| | 3-fluorobenzyl | | | 2-(2,3,4-trimethoxyphenyl)ethyl | |
| | 4-fluorobenzyl | | | | |
| | 2-methylbenzyl | | | 2-(3,4,5-trimethoxyphenyl)ethyl | |
| | 3-methylbenzyl | | | | |
| | 4-methylbenzyl | | | 2-(2-chlorophenyl)ethyl | |
| | 2-pyridylmethyl | | | 2-(4-chlorophenyl)ethyl | |
| | 3-pyridylmethyl | | | | |
| CO | 2-(3,4-dichlorophenyl)ethyl | H | SO₂ | -(CH₂)₇CH₃ | H |
| | | | | -Ph | |
| | 2-(3,4-methylenedioxyphenyl)ethyl | | | -CH₂Ph | |
| | 3-phenylpropyl | | | | |
| | 3-(4-methoxyphenyl)propyl | | | | |
| | 3-(3,4-dimethoxyphenyl)propyl | | | | |
| | 3-(3,4-methylenedioxyphenyl)propyl | | | | |
| (CO)O | Ph | | | | |
| | -CH₂Ph | | | | |
| (CO)NH | -(CH₂)₇CH₃ | | | | |
| | -Ph | | | | |
| | -CH₂Ph | | | | |
| CO | cyclohexyl | OCH₃ | CO | 2-fluorophenyl | OCH₃ |
| | cyclohexylmethyl | | | 3-fluorophenyl | |
| | -CH₃ | | | 4-fluorophenyl | |
| | -CH₂CH₃ | | | 2-methylphenyl | |
| | -(CH₂)₂CH₃ | | | 3-methylphenyl | |
| | -(CH₂)₃CH₃ | | | 4-methylphenyl | |
| | -(CH₂)₄CH₃ | | | 2-furyl | |
| | -(CH₂)₅CH₃ | | | 3-furyl | |
| | -(CH₂)₆CH₃ | | | 2-pyridyl | |
| | -(CH₂)₇CH₃ | | | 3-pyridyl | |
| | - (CH₂)₈CH₃ | | | 4-pyridyl | |
| | -(CH₂)₉CH₃ | | | 2,3-dimethoxyphenyl | |
| | -(CH₂)₅OH | | | 2,4-dimethoxyphenyl | |
| | -(CH₂)₆OH | | | 2,5-dimethoxyphenyl | |
| | -(CH₂)₇OH | | | 2,6-dimethoxyphenyl | |
| | -(CH₂)₈OH | | | 3,4-dimethoxyphenyl | |
| | -(CH₂)₉OH | | | 3,5-dimethoxyphenyl | |
| | phenyl | | | 2,3-dichlorophenyl | |
| | 2-methoxyphenyl | | | 2,4-dichlorophenyl | |
| | 3-methoxyphenyl | | | 2,5-dichlorophenyl | |
| | 4-methoxyphenyl | | | 2,6-dichlorophenyl | |
| | 2-chlorophenyl | | | 3,4-dichlorophenyl | |
| | 3-chlorophenyl | | | 2,4-dinitrophenyl | |
| | 4-chlorophenyl | | | 3,4-methylenedioxyphenyl | |
| CO | 2,3-methylenedioxyphenyl | OCH₃ | CO | 2,5-dimethoxybenzyl | OCH₃ |
| | 1-naphthyl | | | 3,4-dimethoxybenzyl | |
| | 2-naphthyl | | | 3,5-dimethoxybenzyl | |
| | 2,3,4-trimethoxyphenyl | | | 2,4-dichlorobenzyl | |
| | 2,3,6-trimethoxyphenyl | | | 2,6-dichlorobenzyl | |
| | 2,4,5-trimethoxyphenyl | | | 3,4-dichlorobenzyl | |
| | 2,4,6-trimethoxyphenyl | | | 2,4-dinitrobenzyl | |
| | 3,4,5-trimethoxyphenyl | | | 3,4-methylenedioxybenzyl | |
| | 2,3,5-trichlorophenyl | | | 2,3-methylenedioxybenzyl | |
| | 2,4,6-trichlorophenyl | | | 3,4,5-trimethoxybenzyl | |
| | benzyl | | | 2-phenylethyl | |
| | 2-methoxybenzyl | | | 2-(2-methoxyphenyl)ethyl | |
| | 3-methoxybenzyl | | | 2-(3-methoxyphenyl)ethyl | |
| | 4-methoxybenzyl | | | 2-(4-methoxyphenyl)ethyl | |
| | 2-chlorobenzyl | | | 2-(2,3-dimethoxyphenyl)ethyl | |
| | 3-chlorobenzyl | | | 2-(3,4-dimethoxyphenyl)ethyl | |
| | 4-chlorobenzyl | | | | |
| | 2-fluorobenzyl | | | | |
| | 3-fluorobenzyl | | | 2-(2,3,4-trimethoxyphenyl)ethyl | |
| | 4-fluorobenzyl | | | | |
| | 2-methylbenzyl | | | 2-(3,4,5-trimethoxyphenyl)ethyl | |
| | 3-methylbenzyl | | | | |
| | 4-methylbenzyl | | | 2-(2-chlorophenyl)ethyl | |
| | 2-pyridylmethyl | | | 2-(4-chlorophenyl)ethyl | |
| | 3-pyridylmethyl | | | | |
| CO | 2-(3,4-dichlorophenyl)ethyl | OCH₃ | SO₂ | -(CH₂)₇CH₃ | OCH₃ |
| | 2-(3,4-methylenedioxyphenyl)ethyl | | | -Ph | |
| | 3-phenylpropyl | | | -CH₂Ph | |
| | 3-(4-methoxyphenyl)propyl | | | | |
| | 3-(3,4-dimethoxyphenyl)propyl | | | | |
| | 3-(3,4-methylenedioxyphenyl)propyl | | | | |
| (CO)O | Ph | | | | |
| | -CH₂Ph | | | | |
| (CO)NH | -(CH₂)₇CH₃ | | | | |
| | -Ph | | | | |
| | -CH₂Ph | | | | |
| CO | cyclohexyl | Cl | CO | 2-fluorophenyl | Cl |
| | cyclohexylmethyl | | | 3-fluorophenyl | |
| | -CH₃ | | | 4-fluorophenyl | |
| | -CH₂CH₃ | | | 2-methylphenyl | |
| | -(CH₂)₂CH₃ | | | 3-methylphenyl | |
| | -(CH₂)₃CH₃ | | | 4-methylphenyl | |
| | -(CH₂)₄CH₃ | | | 2-furyl | |
| | -(CH₂)₅CH₃ | | | 3-furyl | |
| | -(CH₂)₆CH₃ | | | 2-pyridyl | |
| | -(CH₂)₇CH₃ | | | 3-pyridyl | |
| | -(CH₂)₈CH₃ | | | 4-pyridyl | |
| | -(CH₂)₉CH₃ | | | 2,3-dimethoxyphenyl | |
| | -(CH₂)₅OH | | | 2,4-dimethoxyphenyl | |
| | -(CH₂)₆OH | | | 2,5-dimethoxyphenyl | |
| | -(CH₂)₇OH | | | 2,6-dimethoxyphenyl | |
| | -(CH₂)₈OH | | | 3,4-dimethoxyphenyl | |
| | -(CH₂)₉OH | | | 3,5-dimethoxyphenyl | |
| | phenyl | | | 2,3-dichlorophenyl | |
| | 2-methoxyphenyl | | | 2,4-dichlorophenyl | |
| | 3-methoxyphenyl | | | 2,5-dichlorophenyl | |
| | 4-methoxyphenyl | | | 2,6-dichlorophenyl | |
| | 2-chlorophenyl | | | 3,4-dichlorophenyl | |
| | 3-chlorophenyl | | | 2,4-dinitrophenyl | |
| | 4-chlorophenyl | | | 3,4-methylenedioxyphenyl | |
| CO | 2,3-methylenedioxyphenyl | Cl | CO | 2,5-dimethoxybenzyl | Cl |
| | 1-naphthyl | | | 3,4-dimethoxybenzyl | |
| | 2-naphthyl | | | 3,5-dimethoxybenzyl | |
| | 2,3,4-trimethoxyphenyl | | | 2,4-dichlorobenzyl | |
| | 2,3,6-trimethoxyphenyl | | | 2,6-dichlorobenzyl | |
| | 2,4,5-trimethoxyphenyl | | | 3,4-dichlorobenzyl | |
| | 2,4,6-trimethoxyphenyl | | | 2,4-dinitrobenzyl | |
| | 3,4,5-trimethoxyphenyl | | | 3,4-methylenedioxybenzyl | |
| | 2,3,5-trichlorophenyl | | | 2,3-methylenedioxybenzyl | |
| | 2,4,6-trichlorophenyl | | | 3,4,5-trimethoxybenzyl | |
| | benzyl | | | 2-phenylethyl | |
| | 2-methoxybenzyl | | | 2-(2-methoxyphenyl)ethyl | |
| | 3-methoxybenzyl | | | 2-(3-methoxyphenyl)ethyl | |
| | 4-methoxybenzyl | | | 2-(4-methoxyphenyl)ethyl | |
| | 2-chlorobenzyl | | | 2-(2,3-dimethoxyphenyl)ethyl | |
| | 3-chlorobenzyl | | | 2-(3,4-dimethoxyphenyl)ethyl | |
| | 4-chlorobenzyl | | | | |
| | 2-fluorobenzyl | | | | |
| | 3-fluorobenzyl | | | 2-(2,3,4-trimethoxyphenyl)ethyl | |
| | 4-fluorobenzyl | | | | |
| | 2-methylbenzyl | | | 2-(3,4,5-trimethoxyphenyl)ethyl | |
| | 3-methylbenzyl | | | | |
| | 4-methylbenzyl | | | 2-(2-chlorophenyl)ethyl | |
| | 2-pyridylmethyl | | | 2-(4-chloro phenyl)ethyl | |
| | 3-pyridylmethyl | | | | |
| CO | 2-(3,4-dichlorophenyl)ethyl | Cl | SO₂ | -(CH₂)₇CH₃ | Cl |
| | 2-(3,4-methylenedioxyphenyl)ethyl | | | -Ph | |
| | 3-phenylpropyl | | | -CH₂Ph | |
| | 3-(4-methoxyphenyl)propyl | | | | |
| | 3-(3,4-dimethoxyphenyl)propyl | | | | |
| | 3-(3,4-methylenedioxyphenyl)propyl | | | | |
| (CO)O | Ph | | | | |
| | -CH₂Ph | | | | |
| (CO)NH | -(CH₂)₇CH₃ | | | | |
| | -Ph | | | | |
| | -CH₂Ph | | | | |

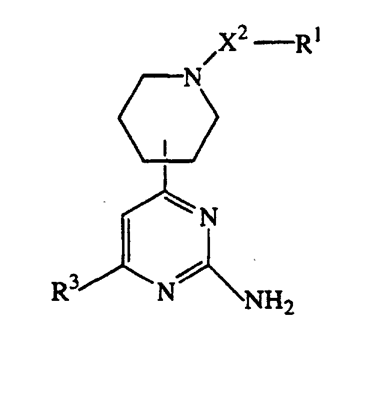

**Table 2**

| X² | R¹ | R³ | X² | R¹ | R³ |
|---|---|---|---|---|---|
| CO | cyclohexyl | Ph | CO | 2-fluorophenyl | Ph |
| | cyclohexylmethyl | | | 3-fluorophenyl | |
| | -CH₃ | | | 4-fluorophenyl | |
| | -CH₂CH₃ | | | 2-methylphenyl | |
| | -(CH₂)₂CH₃ | | | 3-methylphenyl | |
| | -(CH₂)₃CH₃ | | | 4-methylphenyl | |
| | -(CH₂)₄CH₃ | | | 2-furyl | |
| | -(CH₂)₅CH₃ | | | 3-furyl | |
| | -(CH₂)₆CH₃ | | | 2-pyridyl | |
| | -(CH₂)₇CH₃ | | | 3-pyridyl | |
| | -(CH₂)₈CH₃ | | | 4-pyridyl | |
| | -(CH₂)₉CH₃ | | | 2,3-dimethoxyphenyl | |
| | -(CH₂)₅OH | | | 2,4-dimethoxyphenyl | |
| | -(CH₂)₆OH | | | 2,5-dimethoxyphenyl | |
| | -(CH₂)₇OH | | | 2,6-dimethoxyphenyl | |
| | -(CH₂)₈OH | | | 3,4-dimethoxyphenyl | |
| | -(CH₂)₉OH | | | 3,5-dimethoxyphenyl | |
| | phenyl | | | 2,3-dichlorophenyl | |
| | 2-methoxyphenyl | | | 2,4-dichlorophenyl | |
| | 3-methoxyphenyl | | | 2,5-dichlorophenyl | |
| | 4-methoxyphenyl | | | 2,6-dichlorophenyl | |
| | 2-chlorophenyl | | | 3,4-dichlorophenyl | |
| | 3-chlorophenyl | | | 2,4-dinitrophenyl | |
| | 4-chlorophenyl | | | 3,4-methylenedioxyphenyl | |
| CO | 2,3-methylenedioxyphenyl | Ph | CO | 2,5-dimethoxybenzyl | Ph |
| | 1-naphthyl | | | 3,4-dimethoxybenzyl | |
| | 2-naphthyl | | | 3,5-dimethoxybenzyl | |
| | 2,3,4-trimethoxyphenyl | | | 2,4-dichlorobenzyl | |
| | 2,3,6-trimethoxyphenyl | | | 2,6-dichlorobenzyl | |
| | 2,4,5-trimethoxyphenyl | | | 3,4-dichlorobenzyl | |
| | 2,4,6-trimethoxyphenyl | | | 2,4-dinitrobenzyl | |
| | 3,4,5-trimethoxyphenyl | | | 3,4-methylenedioxybenzyl | |
| | 2,3,5-trichlorophenyl | | | 2,3-methylenedioxybenzyl | |
| | 2,4,6-trichlorophenyl | | | 3,4,5-trimethoxybenzyl | |
| | benzyl | | | 2-phenylethyl | |
| | 2-methoxybenzyl | | | 2-(2-methoxyphenyl)ethyl | |
| | 3-methoxybenzyl | | | 2-(3-methoxyphenyl)ethyl | |
| | 4-methoxybenzyl | | | 2-(4-methoxyphenyl)ethyl | |
| | 2-chlorobenzyl | | | 2-(2,3-dimethoxyphenyl) ethyl | |
| | 3-chlorobenzyl | | | 2-(3,4-dimethoxyphenyl)ethyl | |
| | 4-chlorobenzyl | | | | |
| | 2-fluorobenzyl | | | | |
| | 3-fluorobenzyl | | | 2-(2,3,4-trimethoxyphenyl)ethyl | |
| | 4-fluorobenzyl | | | | |
| | 2-methylbenzyl | | | 2-(3,4,5-trimethoxyphenyl)ethyl | |
| | 3-methylbenzyl | | | | |
| | 4-methylbenzyl | | | 2-(2-chlorophenyl)ethyl | |
| | 2-pyridylmethyl | | | 2-(4-chlorophenyl)ethyl | |
| | 3-pyridylmethyl | | | | |
| CO | 2-(3,4-dichlorophenyl)ethyl | Ph | SO₂ | -(CH₂)₇CH₃ | Ph |
| | 2-(3,4-methylenedioxyphenyl)ethyl | | | -CH₂Ph | |
| | 3-phenylpropyl | | | | |
| | 3-(4-methoxyphenyl)propyl | | | | |
| | 3-(3,4-dimethoxyphenyl)propyl | | | | |
| | 3-(3,4-methylenedioxyphenyl)propyl | | | | |
| (CO)O | Ph | | | | |
| | -CH₂Ph | | | | |
| (CO)NH | -(CH₂)₇CH₃ | | | | |
| | -Ph | | | | |
| | -CH₂Ph | | | | |
| CO | cyclohexyl | 4-chlorophlenyl | CO | 2-fluorophenyl | 4-chlorophenyl |
| | cyclohexylmethyl | | | 3-fluorophenyl | |
| | -CH₃ | | | 4-fluorophenyl | |
| | -CH₂CH₃ | | | 2-methylphenyl | |
| | -(CH₂)₂CH₃ | | | 3-methylphenyl | |
| | -(CH₂)₃CH₃ | | | 4-methylphenyl | |
| | - (CH₂)₄CH₃ | | | 2-furyl | |
| | -(CH₂)₅CH₃ | | | 3-furyl | |
| | -(CH₂)₆CH₃ | | | 2-pyridyl | |
| | -(CH₂)₇CH₃ | | | 3-pyridyl | |
| | -(CH₂)₈CH₃ | | | 4-pyridyl | |
| | -(CH₂)₉CH₃ | | | 2,3-dimethoxyphenyl | |
| | -(CH₂)₅OH | | | 2,4-dimethoxyphenyl | |
| | - (CH₂)₆OH | | | 2,5-dimethoxyphenyl | |
| | -(CH₂)₇OH | | | 2,6-dimethoxyphenyl | |
| | -(CH₂)₈OH | | | 3,4-dimethoxyphenyl | |
| | - (CH₂)₉OH | | | 3,5-dimethoxyphenyl | |
| | phenyl | | | 2,3-dichlorophenyl | |
| | 2-methoxyphenyl | | | 2,4-dichlorophenyl | |
| | 3-methoxyphenyl | | | 2,5-dichlorophenyl | |
| | 4-methoxyphenyl | | | 2,6-dichlorophenyl | |
| | 2-chlorophenyl | | | 3,4-dichlorophenyl | |
| | 3-chlorophenyl | | | 2,4-dinitrophenyl | |
| | 4-chlorophenyl | | | 3,4-methylenedioxyphenyl | |
| CO | 2,3-methylenedioxyphenyl | 4-chlorophenyl | CO | 2,5-dimethoxybenzyl | 4-chlorophenyl |
| | 1-naphthyl | | | 3,4-dimethoxybenzyl | |
| | 2-naphthyl | | | 3,5-dimethoxybenzyl | |
| | 2,3,4-trimethoxyphenyl | | | 2,4-dichlorobenzyl | |
| | 2,3,6-trimethoxyphenyl | | | 2,6-dichlorobenzyl | |
| | 2,4,5-trimethoxyphenyl | | | 3,4-dichlorobenzyl | |
| | 2,4,6-trimethoxyphenyl | | | 2,4-dinitrobenzyl | |
| | 3,4,5-trimethoxyphenyl | | | 3,4-methylenedioxybenzyl | |
| | 2,3,5-trichlorophenyl | | | 2,3-methylenedioxybenzyl | |
| | 2,4,6-trichlorophenyl | | | 3,4,5-trimethoxybenzyl | |
| | benzyl | | | 2-phenylethyl | |
| | 2-methoxybenzyl | | | 2-(2-methoxyphenyl)ethyl | |
| | 3-methoxybenzyl | | | 2-(3-methoxyphenyl)ethyl | |
| | 4-methoxybenzyl | | | 2-(4-methoxyphenyl)ethyl | |
| | 2-chlorobenzyl | | | 2-(2,3-dimethoxyphenyl)ethyl | |
| | 3-chlorobenzyl | | | 2-(3,4-dimethoxyphenyl)ethyl | |
| | 4-chlorobenzyl | | | | |
| | 2-fluorobenzyl | | | | |
| | 3-fluorobenzyl | | | 2-(2,3,4-trimethoxyphenyl)ethyl | |
| | 4-fluorobenzyl | | | | |
| | 2-methylbenzyl | | | 2-(3,4,5-trimethoxyphenyl)ethyl | |
| | 3 -methylbenzyl | | | | |
| | 4-methylbenzyl | | | 2-(2-chlorophenyl)ethyl | |
| | 2-pyridylmethyl | | | 2-(4-chlorophenyl)ethyl | |
| | 3-pyridylmethyl | | | | |
| CO | 2-(3,4-dichlorophenyl)ethyl | 4-chlorophenyl | SO₂ | -(CH₂)₇CH₃ | 4-chlorophenyl |
| | 2-(3,4-methylenedioxyphenyl)ethyl | | | -Ph | |
| | 3-phenylpropyl | | | -CH₂Ph | |
| | 3-(4-methoxyphenyl)propyl | | | | |
| | 3-(3,4-dimethoxyphenyl)propyl | | | | |
| | 3-(3,4-methylenedioxyphenyl)propyl | | | | |
| (CO)O | Ph | | | | |
| | -CH₂Ph | | | | |
| (CO)NH | -(CH₂)₇CH₃ | | | | |
| | -Ph | | | | |
| | -CH₂Ph | | | | |
| CO | cyclohexyl | 4-methoxyphenyl | CO | 2-fluorophenyl | 4-methoxyphenyl |
| | cyclohexylmethyl | | | 3-fluorophenyl | |
| | -CH₃ | | | 4-fluorophenyl | |
| | -CH₂CH₃ | | | 2-methylphenyl | |
| | -(CH₂)₂CH₃ | | | 3-methylphenyl | |
| | -(CH₂)₃CH₃ | | | 4-methylphenyl | |
| | -(CH₂)₄CH₃ | | | 2-furyl | |
| | -(CH₂)₅CH₃ | | | 3-furyl | |
| | -(CH₂)₆CH₃ | | | 2 -pyridyl | |
| | -(CH₂)₇CH₃ | | | 3-pyridyl | |
| | -(CH₂)₈CH₃ | | | 4-pyridyl | |
| | -(CH₂)₉CH₃ | | | 2,3-dimethoxyphenyl | |
| | -(CH₂)₅OH | | | 2,4-dimethoxyphenyl | |
| | -(CH₂)₆OH | | | 2,5-dimethoxyphenyl | |
| | -(CH₂)₇OH | | | 2,6-dimethoxyphenyl | |
| | -(CH₂)₈OH | | | 3,4-dimethoxyphenyl | |
| | -(CH₂)₉OH | | | 3,5-dimethoxyphenyl | |
| | phenyl | | | 2,3-dichlorophenyl | |
| | 2-methoxyphenyl | | | 2,4-dichlorophenyl | |
| | 3-methoxyphenyl | | | 2,5-dichlorophenyl | |
| | 4-methoxyphenyl | | | 2,6-dichlorophenyl | |
| | 2-chlorophenyl | | | 3,4-dichlorophenyl | |
| | 3-chlorophenyl | | | 2,4-dinitrophenyl | |
| | 4-chlorophenyl | | | 3,4-methylenedioxyphenyl | |
| CO | 2,3-methylenedioxyphenyl | 4-methoxyphenyl | CO | 2,5-dimethoxybenzyl | 4-methoxyphenyl |
| | 1-naphthyl | | | 3,4-dimethoxybenzyl | |
| | 2-naphthyl | | | 3,5-dimethoxybenzyl | |
| | 2,3,4-trimethoxyphenyl | | | 2,4-dichlorobenzyl | |
| | 2,3,6-trimethoxyphenyl | | | 2,6-dichlorobenzyl | |
| | 2,4,5-trimethoxyphenyl | | | 3,4-dichlorobenzyl | |
| | 2,4,6-trimethoxyphenyl | | | 2,4-dinitrobenzyl | |
| | 3,4,5-trimethoxyphenyl | | | 3,4-methylenedioxybenzyl | |
| | 2,3,5-trichlorophenyl | | | 2,3-methylenedioxybenzyl | |
| | 2,4,6-trichlorophenyl | | | 3,4,5-trimethoxybenzyl | |
| | benzyl | | | 2-phenylethyl | |
| | 2-methoxybenzyl | | | 2-(2-methoxyphenyl)ethyl | |
| | 3-methoxybenzyl | | | 2-(3-methoxyphenyl)ethyl | |
| | 4-methoxybenzyl | | | 2-(4-methoxyphenyl)ethyl | |
| | 2-chlorobenzyl | | | 2-(2,3-dimethoxyphenyl)ethyl | |
| | 3-chlorobenzyl | | | 2-(3,4-dimethoxyphenyl)ethyl | |
| | 4-chlorobenzyl | | | | |
| | 2-fluorobenzyl | | | | |
| | 3-fluorobenzyl | | | 2-(2,3,4-trimethoxyphenyl)ethyl | |
| | 4-fluorobenzyl | | | | |
| | 2-methylbenzyl | | | 2-(3,4,5-trimethoxyphenyl)ethyl | |
| | 3-methylbenzyl | | | | |
| | 4-methylbenzyl | | | 2-(2-chlorophenyl)ethyl | |
| | 2-pyridylmethyl | | | 2-(4-chlorophenyl)ethyl | |
| | 3-pyridylmethyl | | | | |
| CO | 2-(3,4-dichlorophenyl)ethyl | 4-methoxyphenyl | SO₂ | -(CH₂)₇-CH₃ | 4-methoxyphenyl |
| | | | | -Ph | |
| | 2-(3,4-methylenedioxyphenyl)ethyl | | | -CH₂Ph | |
| | 3-phenylpropyl | | | | |
| | 3-(4-methoxyphenyl)propyl | | | | |
| | 3-(3,4-dimethoxyphenyl)propyl | | | | |
| | 3-(3,4-methylenedioxyphenyl)propyl | | | | |
| (CO)O | Ph | | | | |
| | -CH₂Ph | | | | |
| (CO)NH | -(CH₂)₇CH₃ | | | | |
| | -Ph | | | | |
| | -CH₂Ph | | | | |
| CO | cyclohexyl | 4-methoxybenzyl | CO | 2-fluorophenyl | 4-methoxybenzyl |
| | cyclohexylmethyl | | | 3-fluorophenyl | |
| | -CH₃ | | | 4-fluorophenyl | |
| | -CH₂CH₃ | | | 2-methylphenyl | |
| | -(CH₂)₂CH₃ | | | 3-methylphenyl | |
| | -(CH₂)₃CH₃ | | | 4-methylphenyl | |
| | -(CH₂)₄CH₃ | | | 2-furyl | |
| | -(CH₂)₅CH₃ | | | 3-furyl | |
| | -(CH₂)₆CH₃ | | | 2-pyridyl | |
| | -(CH₂)₇CH₃ | | | 3-pyridyl | |
| | -(CH₂)₈CH₃ | | | 4-pyridyl | |
| | -(CH₂)₉CH₃ | | | 2,3-dimethoxyphenyl | |
| | -(CH₂)₅OH | | | 2,4-dimethoxyphenyl | |
| | -(CH₂)₆OH | | | 2,5-dimethoxyphenyl | |
| | -(CH₂)₇OH | | | 2,6-dimethoxyphenyl | |
| | -(CH₂)₈OH | | | 3,4-dimethoxyphenyl | |
| | -(CH₂)₉OH | | | 3,5-dimethoxyphenyl | |
| | phenyl | | | 2,3-dichlorophenyl | |
| | 2-methoxyphenyl | | | 2,4-dichlorophenyl | |
| | 3-methoxyphenyl | | | 2,5-dichlorophenyl | |
| | 4-methoxyphenyl | | | 2,6-dichlorophenyl | |
| | 2-chlorophenyl | | | 3,4-dichlorophenyl | |
| | 3-chlorophenyl | | | 2,4-dinitrophenyl | |
| | 4-chlorophenyl | | | 3,4-methylenedioxyphenyl | |
| CO | 2,3-methylenedioxyphenyl | 4-methoxybenzyl | CO | 2,5-dimethoxybenzyl | 4-methoxybenzyl |
| | 1-naphthyl | | | 3,4-dimethoxybenzyl | |
| | 2-naphthyl | | | 3,5-dimethoxybenzyl | |
| | 2,3,4-trimethoxyphenyl | | | 2,4-dichlorobenzyl | |
| | 2,3,6-trimethoxyphenyl | | | 2,6-dichlorobenzyl | |
| | 2,4,5-trimethoxyphenyl | | | 3,4-dichlorobenzyl | |
| | 2,4,6-trimethoxyphenyl | | | 2,4-dinitrobenzyl | |
| | 3,4,5-trimethoxyphenyl | | | 3,4-methylenedioxybenzyl | |
| | 2,3,5-trichlorophenyl | | | 2,3-methylenedioxybenzyl | |
| | 2,4,6-trichlorophenyl | | | 3,4,5-trimethoxybenzyl | |
| | benzyl | | | 2-phenylethyl | |
| | 2-methoxybenzyl | | | 2-(2-methoxyphenyl)ethyl | |
| | 3-methoxybenzyl | | | 2-(3-methoxyphenyl)ethyl | |
| | 4-methoxybenzyl | | | 2-(4-methoxyphenyl)ethyl | |
| | 2-chlorobenzyl | | | 2-(2,3-dimethoxyphenyl)ethyl | |
| | 3-chlorobenzyl | | | 2-(3,4-dimethoxyphenyl)ethyl | |
| | 4-chlorobenzyl | | | | |
| | 2-fluorobenzyl | | | | |
| | 3-fluorobenzyl | | | 2-(2,3,4-trimethoxyphenyl)ethyl | |
| | 4-fluorobenzyl | | | | |
| | 2-methylbenzyl | | | 2-(3,4,5-trimethoxyphenyl)ethyl | |
| | 3-methylbenzyl | | | | |
| | 4-methylbenzyl | | | 2-(2-chlorophenyl)ethyl | |
| | 2-pyridylmethyl | | | 2-(4-chlorophenyl)ethyl | |
| | 3-pyridylmethyl | | | | |
| CO | 2-(3,4-dichlorophenyl)ethyl | 4-methoxybenzyl | SO₂ | -(CH₂)₇CH₃ | 4-methoxybenzyl |
| | | | | -Ph | |
| | 2-(3,4-methylenedioxyphenyl)ethyl | | | -CH₂Ph | |
| | 3-phenylpropyl | | | | |
| | 3-(4-methoxyphenyl)propyl | | | | |
| | 3-(3,4-dimethoxyphenyl)propyl | | | | |
| | 3-(3,4-methylenedioxyphenyl)propyl | | | | |
| (CO)O | Ph | | | | |
| | -CH₂Ph | | | | |
| (CO)NH | -(CH₂)₇CH₃ | | | | |
| | -Ph | | | | |
| | -CH₂Ph | | | | |
| CO | cyclohexyl | 4-chlorobenzyl | CO | 2-fluorophenyl | 4-chlorobenzyl |
| | cyclohexylmethyl | | | 3-fluorophenyl | |
| | -CH₃ | | | 4-fluorophenyl | |
| | -CH₂CH₃ | | | 2-methylphenyl | |
| | -(CH₂)₂CH₃ | | | 3-methylphenyl | |
| | -(CH₂)₃CH₃ | | | 4-methylphenyl | |
| | -(CH₂)₄CH₃ | | | 2-furyl | |
| | -(CH₂)₅CH₃ | | | 3-furyl | |
| | -(CH₂)₆CH₃ | | | 2-pyridyl | |
| | -(CH₂)₇CH₃ | | | 3-pyridyl | |
| | -(CH₂)₈CH₃ | | | 4-pyridyl | |
| | -(CH₂)₉CH₃ | | | 2,3-dimethoxyphenyl | |
| | -(CH₂)₅OH | | | 2,4-dimethoxyphenyl | |
| | -(CH₂)₆OH | | | 2,5-dimethoxyphenyl | |
| | -(CH₂)₇OH | | | 2, 6-dimethoxyphenyl | |
| | -(CH₂)₈OH | | | 3,4-dimethoxyphenyl | |
| | -(CH₂)₉OH | | | 3,5-dimethoxyphenyl | |
| | phenyl | | | 2,3-dichlorophenyl | |
| | 2-methoxyphenyl | | | 2,4-dichlorophenyl | |
| | 3-methoxyphenyl | | | 2,5-dichlorophenyl | |
| | 4-methoxyphenyl | | | 2,6-dichlorophenyl | |
| | 2-chlorophenyl | | | 3,4-dichlorophenyl | |
| | 3-chlorophenyl | | | 2,4-dinitrophenyl | |
| | 4-chlorophenyl | | | 3,4-methylenedioxyphenyl | |
| CO | 2,3-methylenedioxyphenyl | 4-chlorobenzyl | CO | 2,5-dimethoxybenzyl | 4-chlorobenzyl |
| | 1-naphthyl | | | 3,4-dimethoxybenzyl | |
| | 2-naphthyl | | | 3,5-dimethoxybenzyl | |
| | 2,3,4-trimethoxyphenyl | | | 2,4-dichlorobenzyl | |
| | 2,3,6-trimethoxyphenyl | | | 2,6-dichlorobenzyl | |
| | 2,4,5-trimethoxyphenyl | | | 3,4-dichlorobenzyl | |
| | 2,4,6-trimethoxyphenyl | | | 2,4-dinitrobenzyl | |
| | 3,4,5-trimethoxyphenyl | | | 3,4-methylenedioxybenzyl | |
| | 2,3,5-trichlorophenyl | | | 2,3-methylenedioxybenzyl | |
| | 2,4,6-trichlorophenyl | | | 3,4,5-trimethoxybenzyl | |
| | benzyl | | | 2-phenylethyl | |
| | 2-methoxybenzyl | | | 2-(2-methoxyphenyl)ethyl | |
| | 3-methoxybenzyl | | | 2-(3-methoxyphenyl)ethyl | |
| | 4-methoxybenzyl | | | 2-(4-methoxyphenyl)ethyl | |
| | 2-chlorobenzyl | | | 2-(2,3-dimethoxyphenyl)ethyl | |
| | 3-chlorobenzyl | | | 2-(3,4-dimethoxyphenyl)ethyl | |
| | 4-chlorobenzyl | | | | |
| | 2-fluorobenzyl | | | | |
| | 3-fluorobenzyl | | | 2-(2,3,4-trimethoxyphenyl)ethyl | |
| | 4-fluorobenzyl | | | | |
| | 2-methylbenzyl | | | 2-(3,4,5-trimethoxyphenyl)ethyl | |
| | 3-methylbenzyl | | | | |
| | 4-methylbenzyl | | | 2-(2-chlorophenyl)ethyl | |
| | 2-pyridylmethyl | | | 2-(4-chlorophenyl)ethyl | |
| | 3-pyridylmethyl | | | | |
| CO | 2-(3,4-dichlorophenyl)ethyl | 4-chlorobenzyl | SO₂ | -(CH₂)₇CH₃ | 4-chlorobenzyl |
| | | | | | |
| | 2-(3,4-methylenedioxyphenyl)ethyl | | | -Ph | |
| | 3-phenylpropyl | | | -CH₂Ph | |
| | 3-(4-methoxyphenyl)propyl | | | | |
| | 3-(3,4-dimethoxyphenyl)propyl | | | | |
| | 3-(3,4-methylenedioxyphenyl)propyl | | | | |
| (CO)O | Ph | | | | |
| | -CH₂Ph | | | | |
| (CO)NH | -(CH₂)₇CH₃ | | | | |
| | -Ph | | | | |
| | -CH₂Ph | | | | |
| CO | cyclohexyl | 2-phenylethyl | CO | 2-fluorophenyl | 2-phenylethyl |
| | cyclohexylmethyl | | | 3-fluorophenyl | |
| | -CH₃ | | | 4-fluorophenyl | |
| | -CH₂CH₃ | | | 2-methylphenyl | |
| | -(CH₂)₂CH₃ | | | 3-methylphenyl | |
| | -(CH₂)₃CH₃ | | | 4-methylphenyl | |
| | -(CH₂)₄CH₃ | | | 2-furyl | |
| | -(CH₂)₅CH₃ | | | 3-furyl | |
| | - (CH₂)₆CH₃ | | | 2-pyridyl | |
| | -(CH₂)₇CH₃ | | | 3-pyridyl | |
| | -(CH₂)₈CH₃ | | | 4-pyridyl | |
| | - (CH₂)₉CH₃ | | | 2,3-dimethoxyphenyl | |
| | -(CH₂)₅OH | | | 2,4-dimethoxyphenyl | |
| | - (CH₂)₆OH | | | 2,5-dimethoxyphenyl | |
| | -(CH₂)₇OH | | | 2,6-dimethoxyphenyl | |
| | -(CH₂)₈OH | | | 3,4-dimethoxyphenyl | |
| | -(CH₂)₉OH | | | 3,5-dimethoxyphenyl | |
| | phenyl | | | 2,3-dichlorophenyl | |
| | 2-methoxyphenyl | | | 2,4-dichlorophenyl | |
| | 3-methoxyphenyl | | | 2,5-dichlorophenyl | |
| | 4-methoxyphenyl | | | 2,6-dichlorophenyl | |
| | 2-chlorophenyl | | | 3,4-dichlorophenyl | |
| | 3-chlorophenyl | | | 2,4-dinitrophenyl | |
| | 4-chlorophenyl | | | 3,4-methylenedioxyphenyl | |
| CO | 2,3-methylenedioxyphenyl | 2-phenylethyl | CO | 2,5-dimethoxybenzyl | 2-phenylethyl |
| | 1-naphthyl | | | 3,4-dimethoxybenzyl | |
| | 2-naphthyl | | | 3,5-dimethoxybenzyl | |
| | 2,3,4-trimethoxyphenyl | | | 2,4-dichlorobenzyl | |
| | 2,3,6-trimethoxyphenyl | | | 2,6-dichlorobenzyl | |
| | 2,4,5-trimethoxyphenyl | | | 3,4-dichlorobenzyl | |
| | 2,4,6-trimethoxyphenyl | | | 2,4-dinitrobenzyl | |
| | 3,4,5-trimethoxyphenyl | | | 3,4-methylenedioxybenzyl | |
| | 2,3,5-trichlorophenyl | | | 2,3-methylenedioxybenzyl | |
| | 2,4,6-trichlorophenyl | | | 3,4,5-trimethoxybenzyl | |
| | benzyl | | | 2-phenylethyl | |
| | 2-methoxybenzyl | | | 2-(2-methoxyphenyl)ethyl | |
| | 3-methoxybenzyl | | | 2-(3-methoxyphenyl)ethyl | |
| | 4-methoxybenzyl | | | 2-(4-methoxyphenyl)ethyl | |
| | 2-chlorobenzyl | | | 2-(2,3-dimethoxyphenyl)ethyl | |
| | 3-chlorobenzyl | | | 2-(3,4-dimethoxyphenyl)ethyl | |
| | 4-chlorobenzyl | | | | |
| | 2-fluorobenzyl | | | | |
| | 3-fluorobenzyl | | | 2-(2,3,4-trimethoxyphenyl)ethyl | |
| | 4-fluorobenzyl | | | | |
| | 2-methylbenzyl | | | 2-(3,4,5-trimethoxyphenyl)ethyl | |
| | 3-methylbenzyl | | | | |
| | 4-methylbenzyl | | | 2-(2-chlorophenyl)ethyl | |
| | 2-pyridylmethyl | | | 2-(4-chlorophenyl)ethyl | |
| | 3-pyridylmethyl | | | | |
| CO | 2-(3,4-dichlorophenyl)ethyl | 2-phenylethyl | SO₂ | -(CH₂)₇CH₃ | 2-phenylethyl |
| | | | | -Ph | |
| | 2-(3,4-methylenedioxyphenyl)ethyl | | | -CH₂Ph | |
| | 3-phenylpropyl | | CO | 3,4-methylene dioxyphenyl | 4-iodobenzyl |
| | 3-(4-methoxyphenyl)propyl | | | | 4-fluorobenzyl |
| | 3-(3,4-dimethoxyphenyl)propyl | | | | 4-methylthio-benzyl |
| | | | | | 4-methylsulfinylbenzyl |
| | | | | | 4-methylsulfonylbenzyl |
| | 3-(3,4-methylene-dioxyphenyl)propyl | | | | 4-aminobenzyl |
| | | | | | 4-pyridylmethyl |
| (CO)O | -Ph | | | | |
| | -CH₂Ph | | | | |
| (CO)NH | -(CH₂)₇CH₃ | | | | |
| | -Ph | | | | |
| | -CH₂Ph | | | | |

Hereinafter the present invention will be described in more detail by referring to the following Examples but is not deemed to be limited thereto. In the chemical formulas illustrated hereinbelow, the symbols "Bz" and "Ac" are used to mean benzoyl and acetyl, respectively. Melting points are uncorrected.

### Example 1 Synthesis of 2-amino-4-(1-benzoyl-4-piperidinyl)-6-phenylpyrimidine

(1) 1-Benzoyl-4-[1-(1,3-dioxo-3-phenylpropyl)]piperidine 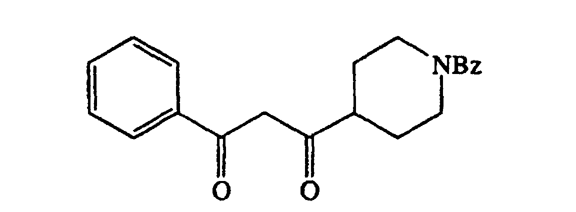
   Ethyl 1-benzoylisonipecotate (30 g, 0.115 mol) and acetophenone (13.82 g, 0.115 mol) were dissolved in 320 ml of tetrahydrofuran (THF). To the solution was added sodium hydride (60% dispersion in mineral oil) (4.6 g, 0.115 mol). The mixture was heated at about 60°C for about 3.5 hours under a nitrogen atmosphere. The reaction mixture was then neutralized with 1 N hydrochloric acid and the solvent was removed in vacuo. The residue was partitioned between ethyl acetate and water. The organic layer was dried over sodium sulfate, and the solvent was removed in vacuo. The residue was purified by silica gel column chromatography (hexane : ethyl acetate = 8 : 2). Crystallization from diethyl ether gave the titled compound (11.86 g).
   Melting point: 104-105°C
   ¹H-NMR (CDCl₃): δ 1.77 (4H, m), 2.62 (1H, m), 2.99 (2H, m), 3.88 (1H, m), 4.78 (1H, m), 6.21 (1H, s), 7.49 (8H, m), 7.88 (2H, dd, J=1.3, J=6.6), 16.21 (1H, s)
(2) 2-Amino-4-(1-benzoyl-4-piperidinyl)-6-phenylpyrimidine 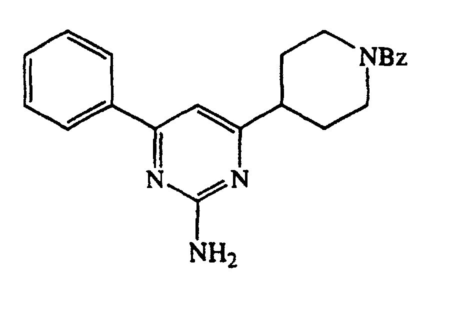
   A mixture of 1-benzoyl-4-[1-(1,3-dioxo-3-phenylpropyl)]piperidine (11 g, 0.033 mol), guanidine hydrochloride (6.3 g, 0.066 mol) and potassium carbonate (9.12 g, 0.066 mol) in pyridine (50 ml) was stirred at about 100°C for about 6 hours. Potassium carbonate (4.56 g, 0.033 mol) and guanidine hydrochloride (3.15 g, 0.033 mol) were further added to the reaction mixture and stirred for about 10 hours at the same temperature. Thereafter dichloromethane and water were added to the reaction mixture followed by extraction. The organic layer was dried over sodium sulfate, and the solvent was removed in vacuo. The residue was purified by silica gel column chromatography (hexane : ethyl acetate = 1 : 1). Crystallization from isopropyl alcohol gave the titled compound (8.60 g).
   Melting point: 147-148°C
   ¹H-NMR (CDCl₃) : δ 1.88 (4H, m), 2.77-3.44 (3H, m), 3.93 (1H, m), 4.89 (1H, m), 5.05 (2H, brs), 6.93 (1H, s), 7.45 (8H, m), 7.97 (2H, m)

The resulting free amine compound (600 mg) was dissolved in methanol and acidified with 1 N hydrogen chloride-diethyl ether solution. The solvent was then removed in vacuo. The residue was triturated with methanol-diethyl ether to give the hydrochloride (582 mg) of the titled compounds.
Melting point: 132-134°C

### Example 2 Synthesis of 2-amino-4-(1-benzoyl-4-piperidinyl)-6-(4-methoxyphenylmethyl)pyrimidine

(1) 1-Benzoyl-4-piperidinecarbonyl chloride 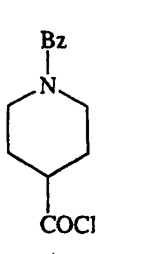
   A mixture of 1-benzoylisonipecotic acid (60 g, 0.257 mol) thionyl chloride (38 ml) and chloroform (360 ml) was refluxed for about 3 hours. Thereafter the reaction mixture was concentrated in vacuo. Toluene was added to the residue and the mixture was concentrated in vacuo to remove an excess of thionyl chloride. The titled compound was obtained as a crude oil.
(2) 1-Benzoyl-4-{1-[4-(4-methoxyphenyl)-1,3-dioxobutyl]}piperidine 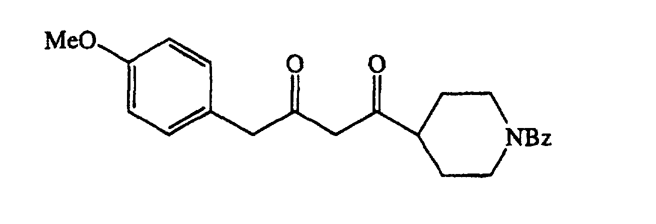
   Diisopropylamine (72 ml, 0.514 mol) was dissolved in THF (510 ml). Under a nitrogen atmosphere n-butyl lithium-hexane solution (310 ml, 1.66 mmol/ml) was added dropwise to the solution over a period of about 1.5 hours at about 0°C. After stirring at the same temperature for about 30 minutes, the mixture was cooled to about -78°C and 4-methoxyphenylacetone (84.4 g, 1.03 mol) was added dropwise over a period of about 1.5 hour and stirred for about 30 minutes. A solution of 1-benzoyl-4-piperidinecarbonyl chloride of Example 2 (1) in THF (510 ml) was added dropwise to the mixture over a period of about 2 hours at the same temperature.
   Thirty minutes later conc. hydrochloric acid was added to the reaction mixture. Ethyl acetate and water were then added to the mixture for extraction. The organic layer was washed with water and dried over magnesium sulfate, and then the solvent was removed in vacuo. The residue was purified by silica gel column chromatography (hexane : ethyl acetate = 6 : 4) to give the titled compound (35.5 g) as an oil.
   ¹H-NMR (CDCl₃) : δ 1.70 (4H, m), 2.38 (1H, m), 2.86 (2H, m), 3.65 (2H, s), 3.75 (1H, m), 3.82 (3H, s), 4.73 (1H, m), 5.43 (1H, s), 6.90 (1H, d, J=7.6), 6.97 (1H, d, J=7.6), 7.18 (1H, d, J=7.6), 7.28 (1H, d, J=7.6),7.40 (5H, m), 15.45 (1H, s)
   In this reaction 35.5 g of 1-benzoyl-4-{1-[2-(4-methoxyphenyl)-1,3-dioxobutyl]} piperidine was also obtained as a crystal. 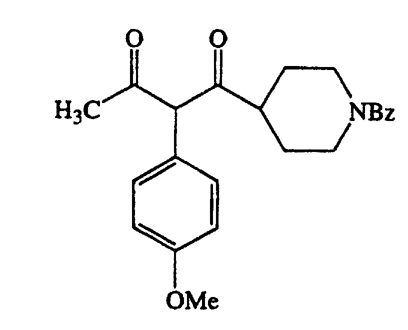 Melting point: 134-137°C
   ¹HNMR (CDCl₃) : δ 1.56-1.72 (4H, m), 1.88 (3H, s), 2.42 (1H, m), 2.65 (2H, m), 3.70 (1H, m), 3.85 (3H, s), 4.67 (1H, m), 6.92 (2H, d, J=7.9), 7.06 (2H, d, J=7.9), 7.37 (5H, m), 16.86 (1H, s)
(3) 2-Amino-4-(1-benzoyl-4-piperidinyl)-6-(4-methoxyphenylmethyl)pyrimidine 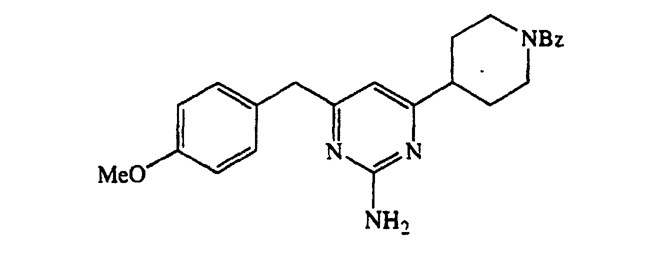
   By a procedure similar to that described in Example 1(2) and crystallization from ethanol, the titled compound (15.3 g) was prepared from 1-benzoyl-4-{1-[4-(4-methoxyphenyl)-1,3-dioxobutyl]}piperidine (33 g, 0.084 mol).
   Melting point: 170-171°C
   ¹H-NMR (CDCl₃): δ 1.75 (4H, m), 2.65 (1H, m), 2.93(2H, m), 3.80 (3H, s), 3.83 (2H, s), 3.85(1H, m), 4.80 (1H, m), 4.94 (2H, brs), 6.26 (1H, s), 6.86 (2H, d, J=8.6), 7.16 (2H, d, J=8.6), 7.40 (5H, m)

The titled compound (250 mg) in methanol was acidified with 1 N hydrogen chloride-diethyl ether solution. The solvent was then removed in vacuo. The residue was triturated with tetrahydrofuran-diethyl ether to give the hydrochloride (270mg) of the titled compound.
Melting point: 169-170°C

### Example 3 Synthesis of 2-amino-4-(1-benzoyl-4-piperidinyl)-5-(4-methoxyphenyl)-6-methylpyrimidine

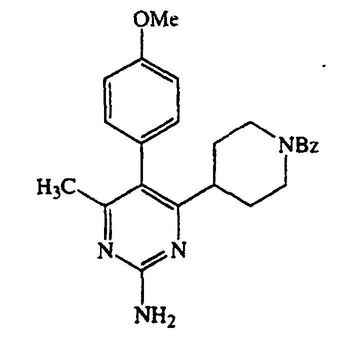

By a procedure similar to that described in Example 1(2) and crystallization from ethanol, the titled compound (2.14 g) was prepared from 1-benzoyl-4-{1-[2-(4-methoxyphenyl)-1,3-dioxobutyl]}piperidine(30 g, 0.076 mol).
Melting point: 226-227°C
¹H-NMR (CDCl₃): δ 1.63-1.98 (4H, m), 2.52-2.81 (3H, m), 2.08 (3H, s), 3.78 (1H, m), 3.87(3H, s), 4.71 (1H, m), 4.90 (2H, brs), 7.00 (4H, m), 7.39 (5H, m).

The titled compound (50 mg) in methanol was acidified with hydrogen chloride-diethyl ether solution. The solvent was then removed in vacuo. The residue was triturated with tetrahydrofuran-diethyl ether to give the hydrochloride (50 mg) of the titled compound.

### Example 4 Synthesis of 2-amino-4-(1-benzoyl-4-piperidinyl)-6-(2-phenylethyl)pyrimidine

(1) 1-Benzoyl-4-[1-(1,3-dioxo-5-phenylpentyl)]piperidine 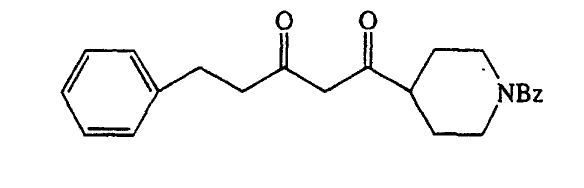
   By a procedure similar to that described in Example 2 (1) and (2), the titled compound (29.9 g) as an oil was prepared from 1-benzoylisonipecotic acid (50 g, 0.214 mol) and benzylacetone (38.06g, 0.257 mol).
   ¹H-NMR (CDCl₃): δ 1.63 (3H, m), 2.43 (1H, m), 2.63(2H, t, J=7.3), 2.91 (5H, m), 3.83 (1H, m), 4.71 (1H, m), 5.46 (1H, s), 7.30 (10H, m), 15.48 (1H, brs)
(2) 2-Amino-4-(1-benzoyl-4-piperidinyl)-6-(2-phenylethyl)pyrimidine 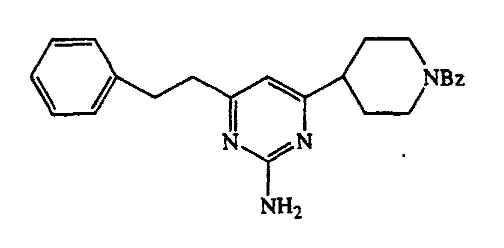
   By a procedure similar to that described in Example 1(2) and crystallization from ethanol, the titled compound (23.8 g) was prepared from 1-benzoyl-4-[1-(1,3-dioxo-5-phenyl-pentyl)]piperidine (29 g, 0.080 mol).
   Melting point: 104-106°C
   ¹H-NMR (CDCl₃): δ 1.78 (4H, m), 2.67 (1H, m), 2.84-3.01 (6H, m), 3.85 (1H, m), 4.80 (1H, m), 4.93 (2H, brs), 6.27 (1H, s), 7.17-7.42 (10H, m)

The titled compound (400 mg) in methanol was acidified with 1 N hydrogen chloride-diethyl ether solution. The solvent was then removed in vacuo. The residue was triturated with tetrahydrofuran-diethyl ether to give of the hydrochloride (437 mg) of the titled compound.
Melting point: 216-217°C

### Example 5 Synthesis of 2-amino-4-(1-benzoyl-4-piperidinyl)-6-(4-methoxyphenyl)pyrimidine

(1) 1-Benzoyl-4-{1-[3-(4-methoxyphenyl)-1,3-dioxopropyl]}piperidine 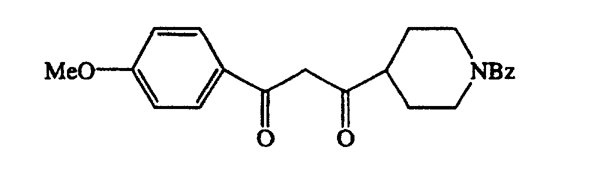
   By a procedure similar to that described in Example 2(1) and (2) and crystallization from diethyl ether, the titled compound (11.67 g) was prepared from 1-benzoylisonipecotic acid (15.16 g, 0.065 mol) and 4'-methoxyacetophenone (19.52 g, 0.13 mol).
   Melting point: 226-227°C
   1H-NMR (CDCl₃): δ 1.76 (4H, m), 2.58 (1H, m), 2.91 (2H, m), 3.88 (4H, m), 4.78 (1H, m), 6.14 (1H, s), 6.95 (2H, d, J=8.6), 7.42 (5H, m), 7.87 (2H, d, J=8.6), 16.38 (1H, s)
(2) 2-Amino-4-(1-benzoyl-4-piperidinyl)-6-(4-methoxyphenyl)pyrimidine 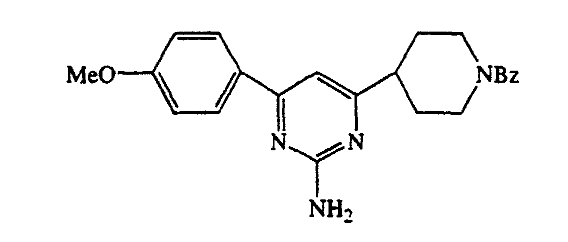
   By a procedure similar to that described in Example 1(2) and crystallization from ethanol, the titled compound (7.81 g) was prepared from 1-benzoyl-4-{1-[3-(4-methoxyphenyl)-1,3-dioxopropyl]}piperidine (10 g, 0.027 mol).
   Melting point: 165-166°C
   ¹H-NMR (CDCl₃): δ 1.88 (4H, m), 2.79 (1H, m), 3.00(2H, m), 3.87 (4H, m), 4.90 (1H, m), 5.02 (2H, s), 6.87 (1H, s), 6.98 (2H, d, J=8.6), 7.43 (5H, m), 7.96(2H, d, J=8.6).

The titled compound (110 mg) in chloroform was acidified with 1 N-hydrogen chloride-diethyl ether solution. The solvent was removed in vacuo. The residue was triturated with chloroform-diethyl ether to give the hydrochloride (100 mg) of the titled compound.
Melting point: 249-253°C

### Example 6 Synthesis of 2-amino-4-(1-benzoyl-4-piperidinyl)-6-(4-chlorophenyl)pyrimidine

(1) 1-Benzoyl-4-{1-[3-(4-chlorophenyl)-1,3-dioxopropyl]}piperidine 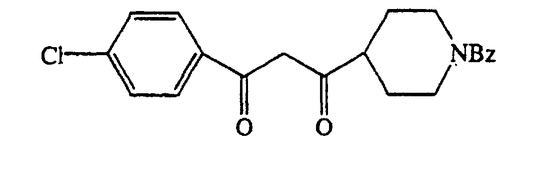
   By a procedure similar to that described in Example 2(1) and (2) and crystallization from diethyl ether, the titled compound (11.92 g) was prepared from 1-benzoylisonipecotic acid (15.16 g, 0.065 mol) and 4'-chloroacetophenone (20.98 g, 0.13 mol).
   Melting point: 129-130°C
   ¹H-NMR (CDCl₃) : δ 1.77 (4H, m), 2.62 (1H, m), 2.95 (2H, m), 3.88 (1H, m), 4.78 (1H, m), 6.16 (1H, s), 7.44 (7H, m), 7.83(2H, d, J=8.6), 16.16 (1H, s).
(2) 2-Amino-4-(1-benzoyl-4-piperidinyl)-6-(4-chlorophenyl)pyrimidine 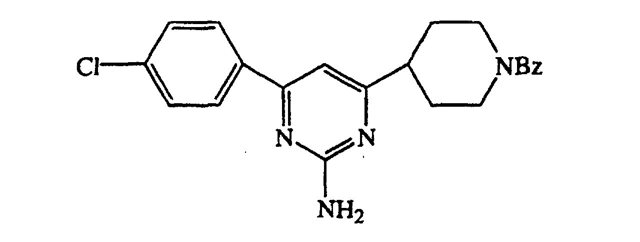
   By a procedure similar to that described in Example 1(2) and crystallization from isopropanol, the titled compound (7.25 g) was prepared from 1-benzoyl-4-{1-[3-(4-chlorophenyl)-1,3-dioxopropyl]}piperidine(10 g, 0.027mol).
   Melting point: 210-211°C
   ¹H-NMR (CDCl₃): δ 1.87 (4H, m), 2.81 (1H, m),3.00 (2H, m), 3.90 (1H, m), 4.87 (1H, m), 5.08 (2H, s), 6.89 (1H, s),7.44 (7H, m), 7.94 (2H, d, J=8.6).

The titled compound (110 mg) in chloroform was acidified with 1 N-hydrogen chloride-diethyl ether solution. The solvent was removed in vacuo. The residue was triturated with chloroform-diethyl ether to give the hydrochloride (85 mg) of the titled compound.
Melting point: 238-239°C

### Example 7 Synthesis of 2-amino-4-(1-benzoyl-4-piperidinyl)-6-(4-chlorophenylmelthyl)pyrimidine

(1) 1-Benzoyl-4-{1-[4-(4-chlorophenyl)-1,3-dioxobutyl]}piperidine 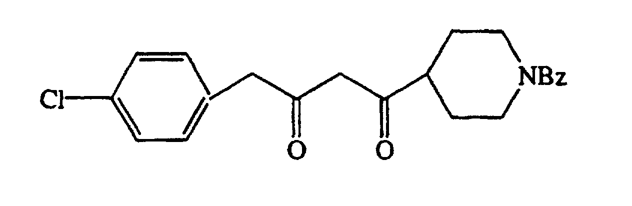
   By a procedure similar to that described in Example 2(1) and (2), the title compound (9.86 g) was prepared from 1-benzoylisonipecotic acid (27.64 g, 0.119 mol) and 4'-chlorophenylacetone (40 g, 0.237 mol).
   ¹H-NMR (CDCl₃): δ 1.55-1.90 (4H, m), 2.41 (1H, m), 2.92 (2H, m), 3.58 (2H, s), 3.78(1H, m), 4.71 (1H, m), 5.44 (1H, s), 7.17(2H, d, J=8.6), 7.27 (2H, d, J=8.6),7.40 (5H, m), 15.37 (1H, s).
(2) 2-Amino-4-(1-benzoyl-4-piperidinyl)-6-(4-chlorophenylmethyl)pyrimidine 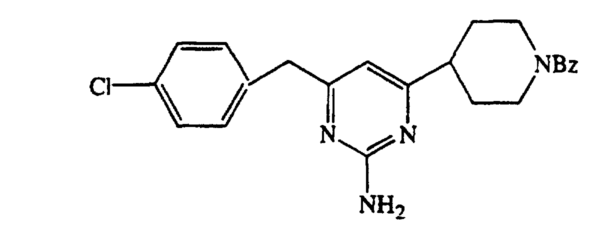
   By a procedure similar to that described in Example 1(2) and crystallization from methanol, the titled compound (5.54 g) was prepared from 1-benzoyl-4-{1-[4-(4-chlorophenyl)-1,3-dioxobutyl]}piperidine (9.0 g, 0.023mol).
   ¹H-NMR (CDCl₃): δ 1.76 (4H, m), 2.66 (1H, m), 2.86(2H, m), 3.85 (2H, s), 3.90 (1H, m), 4.80 (1H, m), 4.99 (2H, brs), 6.26(1H, s), 7.18 (2H, d, J=8.3), 7.28(2H, d, J=8.3), 7.40 (5H, m).

The titled compound (180 mg) in chloroform was acidified with 1 N-hydrogen chloride-diethyl ether solution. The solvent was removed in vacuo. The residue was triturated with chloroform-diethyl ether to give the hydrochloride (194 mg) of the titled compound.
Melting point: 148-150°C

### Example 8 Synthesis of 2-amino-4-(1-benzoyl-4-piperidinyl)quinazoline

(1) 1-Benzoyl-4-cyanopiperidine (2) 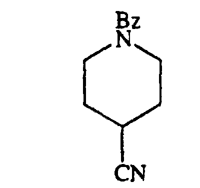
   By a procedure similar to that described later in Reference Example 1 and crystallization from isopropanol, the titled compound (73.5 g) was prepared from 4-cyanopiperidine(40 g, 0.363mol).
   ¹H-NMR (CDCl₃): δ 1.91 (4H, m), 2.93 (1H, m), 3.20-4.10 (4H, m), 7.40 (5H, m)
(2) 1-Benzoyl-4-(2-aminobenzoyl)piperidine 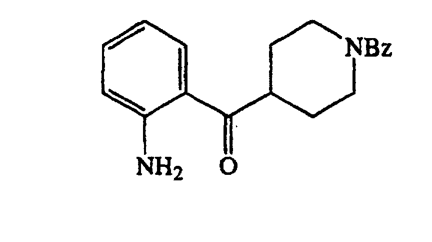
   To an ice cold solution of boron trichloride (26.3 ml, 0.303 mol) in 300 ml of 1,2-dichloroethane was added dropwise aniline (28.21 g, 0.303 mol) over a period of about 15 minutes under a nitrogen atmosphere. Then to the solution was added portionwise 1-benzoyl-4-cyanopiperidine (50 g, 0.233 mol) and aluminum chloride (40.39 g, 0.303 mol) successively. After stirring at room temperature for about 15 minutes, the mixture was refluxed for about 14 hours. After 1 N hydrochloric acid aqueous solution was added to the reaction mixture at about ice cooling temperature, the mixture was refluxed for about an hour. To the reaction mixture 3 N sodium hydroxide was added to make basic. The resulting slurry was filtered over Celite. The filtrate was extracted with dichloromethane. The organic layer was dried over sodium sulfate, and the solvent was removed in vacuo. The residue was crystallized from ethanol to give 51.4 g of the titled compound.
   Melting point: 138-138.5°C
   ¹H-NMR (CDCl₃): δ 1.64-1.90 (4H, m), 3.07 (2H,m),3.56 (1H, m), 3.92 (1H, m), 4.75 (1H, m), 6.31 (2H, brs), 6.64 (2H, t, J=7.6), 7.25-7.41 (6H, m), 7.74 (1H, d, J=7.6)
(3) 2-Amino-4-(1-benzoyl-4-piperidinyl)quinazoline 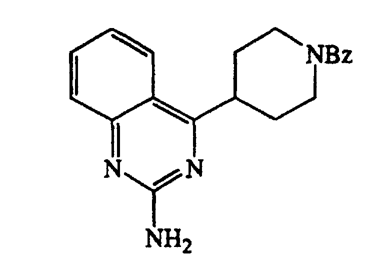
   The titled compound was prepared by warming a mixture of 1-benzoyl-4-(2-aminobenzoyl)piperidine hydrochloride (17 g, 0.049 mol) and cyanamide (4.12 g, 0.098 mol). At about 50°C, vigorous exothermic reaction occurred. After the reaction was finished, the reaction mixture was cooled, water was added to the reaction mixture, and then added sodium hydrogencarbonate to make the mixture basic. The mixture was then extracted with dichloromethane. The organic layer was washed with aqueous solution of saturated sodium chloride, and then dried over sodium sulfate and evaporated in vacuo. The residue was purified by silica gel column chromatography (hexane : ethyl acetate = 1 : 1) to give the titled compound (15.9 g) as a foam.
   ¹H-NMR (CDCl₃): δ 1.80-2.10 (4H, m), 3.20 (2H, m), 3.70 (1H, m), 3.96 (1H, m), 4.89 (1H, m), 5.13 (2H, brs), 7.26-7.71 (9H, m)

### Example 9 Synthesis of 2-amino-4-(1-benzoyl-4-piperidinyl)-6-methoxyquinazoline

(1) 1-Benzoyl-4-(2-amino-5-methoxybenzoyl)piperidine 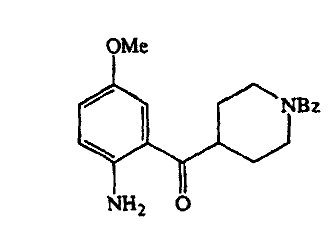
   By a procedure similar to that described in Example 8(2), the title compound (8.12 g) was prepared from 4-methoxyaniline (10.26 g, 0.083 mol) and 1-benzoyl-4-cyanopiperidine (21.53 g, 0.10 mol).
   ¹H-NMR (CDCl₃) : δ 1.60-1.95 (4H, m), 2.95 (2H, m), 3.41 (1H, m), 3.69 (3H, s), 3.75(1H, m), 4.65 (1H, m), 5.95 (2H, brs), 6.57 (1H, d, J=8.9), 6.90 (1H, dd, J=8.9, J=3.0), 7.12 (1H, d, J=3.0), 7.30 (5H, m).
(2) 2-Amino-4-(1-benzoyl-4-piperidinyl)-6-methoxyquinazoline 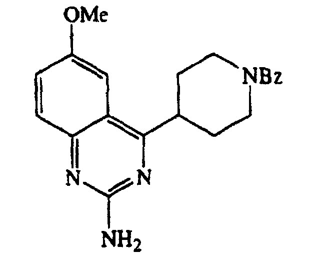
   By a procedure similar to that described in Example 8(3), the titled compound (4.15 g) was prepared from 1-benzoyl-4-(2-amino-5-methoxybenzoyl)piperidine hydrochloride ( 4.51 g, 0.012 mol).
   Melting point: 172-173°C
   ¹H-NMR (CDCl₃): δ 1.88-2.08 (4H, m), 3.07 (2H, m), 3.61 (1H, m), 3.92 (3H, s), 3.95 (1H, m), 5.00 (3H, m), 7.19 (1H, d, J=2.4), 7.27-7.50 (6H, m), 7.56 (1H, d, J=9.2).

### Example 10 Synthesis of 2-amino-4-(1-benzoyl-4-piperidinyl)-6-chloroquinazoline

(1) 1-Benzoyl-4-(2-amino-5-chlorobenzoyl)piperidine 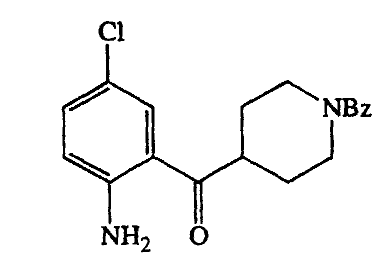
   By a procedure similar to that described in Example 8(2), the titled compound (808 mg) was prepared from 4-chloroaniline (2.18 g, 0.017 mol) and 1-benzoyl-4-cyanopiperidine (4.03 g, 0.018 mol).
   ¹H-NMR (CDCl₃): δ 1.76-1.99 (4H, m), 2.98 (2H, m), 3.46 (1H, m), 3.83 (1H, m), 4.72 (1H, m), 6.40 (2H, brs), 6.61 (1H, d, J=8.9), 7.17 (1H, dd, J=8.9, J=2.2), 7.40 (5H, m), 7.64 (1H, d, J=2.2).
(2) 2-Amino-4-(1-benzoyl-4-piperidinyl)-6-chloroquinazoline 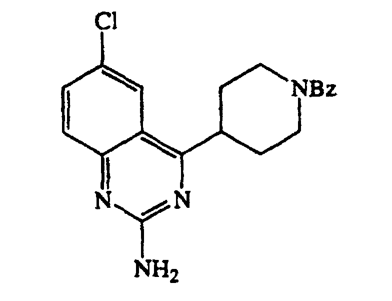
   By a procedure similar to that described in Example 8(3), the titled compound (477 mg) was prepared from 1-benzoyl-4-(2-amino-5-methoxybenzoyl)-piperidine hydrochloride (800 mg, 2.1 mmol).
   Melting point: 155-158°C
   ¹H-NMR (CDCl₃): δ 1.80-2.12 (4H, m), 3.14 (2H, m), 3.61 (1H, m), 3.96 (1H, m), 4.91 (1H, m), 5.15 (2H, s), 7.42-7.65 (7H, m), 7.90 (1H, d, J=2.0).

### Example 11 Synthesis of 4-(1-acetyl-4-piperidinyl)-2-aminoquinazoline

(1) 2-Amino-4-(4-piperidinyl)quinazoline 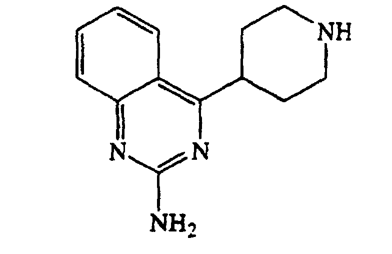
   A mixture of 2-amino-4-(1-benzoyl-4-piperidinyl)quinazoline (24 g, 0.072 mol) obtained in Example 8 (3), ethanol (250 ml) and 6 N sodium hydroxide aqueous solution (250 ml) was refluxed for about 7 hours under a nitrogen atmosphere. Thereafter ethanol was removed from the reaction mixture in vacuo. The residue was extracted with dichloromethane. The organic layer was washed with saturated sodium chloride aqueous solution, and then dried over magnesium sulfate and evaporated in vacuo. The residue was crystallized from isopropyl alcohol to give 13.0 g of the titled compound.
   Melting point: 184-186°C
   ¹H-NMR (CDCl₃): δ 1.78-1.96 (5H, m), 2.85 (2H, ddd, J=3.3, J=11.9, J=11.9), 3.26 (2H, dd, J=3.3, J=11.9), 3.56 (1H, m), 5.14 (2H, brs), 7.27 (1H, dd, J=8.3, J=5.9), 7.57-7.69 (2H, m), 7.96 (1H, d, J=8.3)
(2) 4- (1-Acetyl-4-piperidinyl)-2-aminoquinazoline 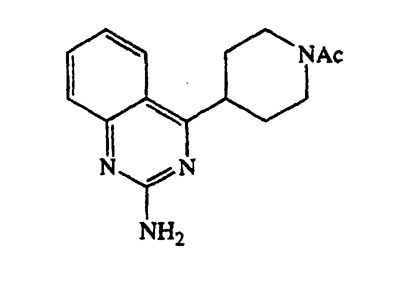
   To an ice cold solution of 2-amino-4-(4-piperidyl)quinazoline (500 mg, 2.19 mmols) and triethylamine (233 mg, 2.3 mmol) in N,N-dimethylformamide (10 ml) was added dropwise acetic anhydride (0.22 ml, 2.30 mmol) under a nitrogen atmosphere. The reaction mixture was stirred for about 3 hours and was evaporated. The residue was partitioned between dichloromethane and saturated sodium hydrogencarbonate aqueous solution. The organic layer was washed with water and then dried over sodium sulfate and evaporated in vacuo. The residue was crystallized from ethanol. Recrystallization from methanol gave the titled compound(452 mg).
   Melting point: 239-240°C
   ¹H-NMR (CDCl₃): δ 1.96 (4H, m), 2.16 (3H, s), 2.77 (1H, m), 3.30 (1H, m), 3.66 (1H, m), 4.00 (1H, m), 4.79 (1H, m), 5.12 (2H, brs), 7.28 (1H, m), 7.65 (2H, m), 7.94 (1H, d, J=7.9)

The titled compound (290 mg) in methanol was acidified with 1 N hydrogen chloride-diethyl ether solution. The solvent was then removed in vacuo. The residue was crystallized from isopropyl alcohol to give the hydrochloride (324 mg) of the titled compound.
Melting point: 275°C (decomposed)

### Example 12 Synthesis of 2-amino-4-[1-(3,4-methylenedioxyphenylacetyl)-4-piperidinyl]quinazoline

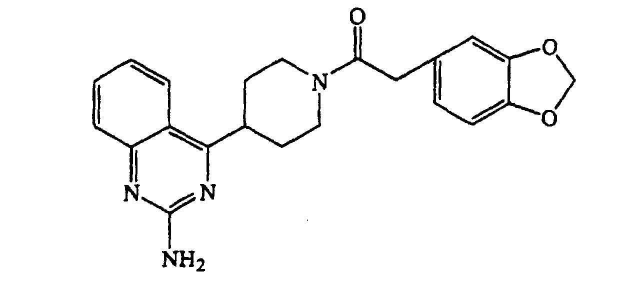

To a suspension of 3,4-methylenedioxyphenylacetic acid (569 mg, 3.16 mmol) in chloroform (6 ml) was added thionyl chloride (1.2 ml). The mixture was refluxed for about 3 hours, and was evaporated. Toluene was added to the residue and the mixture was evaporated to remove the excess of thionyl chloride. To the residue was added dichloromethane (3 ml) to give a dichloromethane solution of 3,4-methylenedioxyphenylacetyl chloride. To an ice cold mixture of 2-amino-4-(4-piperidinyl)quinazoline (600 mg, 2.63 mmol) and triethylamine (319 mg, 3.16 mmol) in dichloromethane (16 ml) was added dropwise the solution of 3,4-methylenedioxyphenylacetyl chloride prepared above under a nitrogen atmosphere. The mixture was stirred for about two hours and to the reaction mixture was added dichloromethane and saturated sodium hydrogencarbonate aqueous solution followed by extraction. The organic layer was washed with water, and then dried over sodium sulfate and evaporated in vacuo. The residue was purified by silica gel column chromatography (dichloromethane : methanol = 30 : 1). Crystallization from methanol gave the titled compound (617 mg).
Melting point: 167-168°C.
¹H-NMR (CDCl₃): δ 1.89 (4H, m), 2.83 (1H, m), 3.21(1H, m), 3.59 (1H, m), 3.70 (2H, s), 4.05 (1H, m), 4.79 (1H, m), 5.10 (2H, brs), 5.95 (2H, s), 6.75 (3H, m), 7.27 (1H, m), 7.59 (1H, d, J=7.6), 7.67 (1H, m), 7.90 (1H, d, J=7.6)

The titled compound (260 mg) in methanol was acidified with 1 N hydrogen chloride-diethyl ether solution. The solvent was then removed in vacuo. Crystallization from ethanol gave the hydrochloride (280 mg) of the titled compound.
Melting point: 157-160°C

### Example 13 Synthesis of 2-amino-4-[1-(3,4-dihydroxyphenylacetyl]-4-piperidinyl]quinazoline

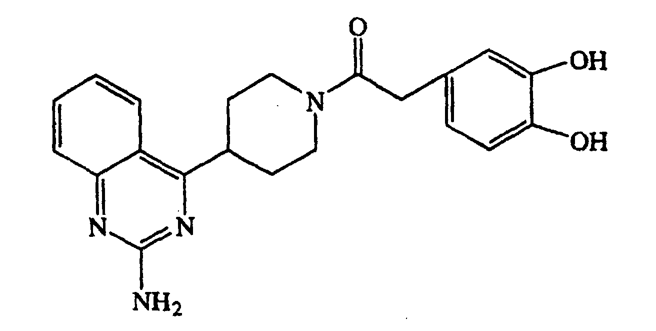

To an ice-cold solution of 2-amino-4-(4-piperidinyl)quinazoline (600 mg, 2.63 mmol) obtained in example 11(1), 3,4-dihydroxyphenylacetic acid (530 mg, 3.16 mmol) and 4-dimethylaminopyridine (386 mg, 3.16 mmol) in dimethylformamide (20 ml) was added 1-ethyl-3-(N,N'-dimethylaminopropyl)carbodiimide hydrochloride (605 mg, 3.16 mmol) under a nitrogen atmosphere. The mixture was stirred overnight at room temperature. The solvent was removed in vacuo and the residue was crystallized from methanol to give the titled compound (599 mg).
Melting point: 250-253°C
¹H-NMR (DMSO-d₆): δ 1.72 (4H, m), 2.79 (1H, m), 3.17-3.58 (3H, m), 3.76 (1H, m), 4.06 (1H, m), 4.54 (1H, m), 6.51 (1H, d, J=7.9), 6.66 (4H, m), 7.20 (1H, dd, J=6.9, J=7.3), 7.41 (1H, d, J=7.3), 7.64 (1H, dd, J=6.9, J=7.6), 8.07 (1H, d, J=7.6), 8.79 (2H, brs).

The titled compound (200 mg) in methanol was acidified with 1 N-hydrogen chloride-diethylether solution. The solvent was removed in vacuo. Crystallization from ethanol gave the hydrochloride (150 mg) of the titled compound.

### Examples 14 to 267

(1) 2-Amino-4-phenyl-6-(4-piperidinyl)pyrimidine 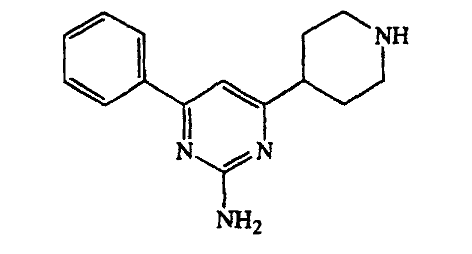
   By a procedure similar to that described in Example 11(1) and crystallization from methanol, the titled compound (4.8 g) was prepared from 2-amino-4-(1-benzyl-4-piperidinyl)-6-phenylpyrimidine (7 g, 20 mmol) obtained in Example 1 (2).
   Melting point: 165-170°C
   ¹H-NMR (CDCl₃) : δ 1.73 (3H, m), 1.92 (2H, m), 2.66 (1H, m), 2.75 (2H, dt, J=2.6, J=12.2), 3.12 (2H, m), 5.04 (2H, brs), 6.94 (1H, s), 7.46 (3H, m), 7.97 (2H, m)
(2) 2-Amino-4-(4-methoxyphenylmethyl)-6-(4-piperidinyl)pyrimidine 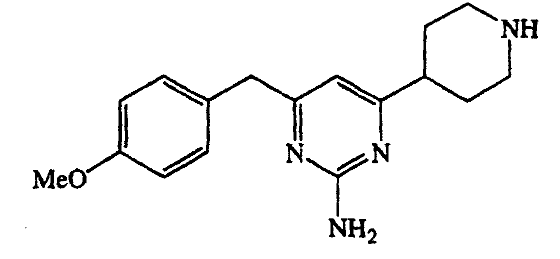
   By a procedure similar to that described in Example 11(1) and crystallization from isopropyl alcohol-diethyl ether, the titled compound (8.81 g) was prepared from 2-amino-4-(1-benzoyl-4-piperidinyl)-6-(4-methoxyphenyl-methyl)pyrimidine (15 g, 37 mmol) obtained in Example 2 (3).
   Melting point: 112-113°C
   ¹H-NMR (CDCl₃) : δ 1.57 (3H, m), 1.79 (2H, m), 2.50 (1H, m), 2.67 (2H, dt, J=12.2, J=2.3), 3.14 (2H, m), 3.80 (3H, s), 3.83 (2H, s),4.96 (2H, brs), 6.28 (1H, s), 6.85 (2H, d,J=8.9), 7.16 (2H, d, J=8.9)
(3) 2-Amino-4-(2-phenylethyl)-6-(4-piperidinyl)-pyrimidine 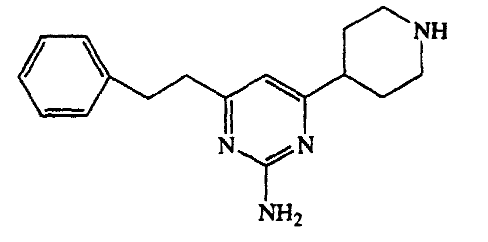
   By a procedure similar to that described in Example 11(1) and crystallization from isopropyl alcohol-diethyl ether, the titled compound (10.8 g) was prepared from 2-amino-4-(1-benzyl-4-piperidinyl)-6-(2-phenylethyl)pyrimidine (22 g, 57 mmol) obtained in Example 4(2). Melting point: 205°C (decomposed)
   ¹H-NMR (DMSO-d₆) : δ 1.84 (4H, m), 2.73 (3H, m), 2.92 (4H, m), 3.28 (2H, d, J=12.5), 6.36 (1H, s), 6.50 (2H, s), 7.23 (5H, m), 8.99 (1H, brs)
(4) 2-Amino-4-methyl-5-(4-methoxyphenyl)-6-(4-piperidinyl)pyrimidine 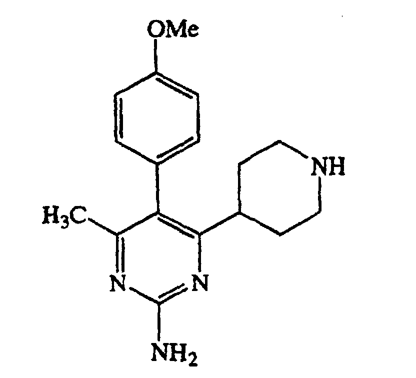
   By a procedure similar to that described in Example 11(1) and crystallization from isopropanoldiethyl ether, the titled compound (1.24 g) was prepared from 2-amino-4-(1-benzoyl-4-piperidinyl)-5-(4-methoxyphenyl)-6-methylpyrimidine (1.9 g, 4.7 mmol) obtained in Example 3.
   Melting point: 191-192°C
   ¹H-NMR (CDCl₃): δ 1.71 (5H, m), 2.06 (3H, s), 2.45 (3H, m), 3.04 (2H, m), 3.87 (3H, s), 4.87 (2H, brs),6.99 (4H, m).
(5) 2-Amino-4-(4-methoxyphenyl)-6-(4-piperidinyl)-pyrimidine 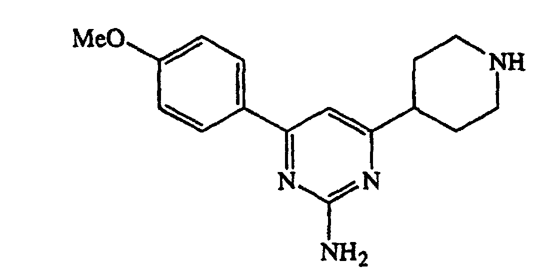
   By a procedure similar to that described in Example 11(1) and crystallization from isopropanol, the title compound (3.73 g) was prepared from 2-amino-4-(1-benzoyl-4-piperidinyl)-6-(4-methoxyphenyl)pyrimidine(7.0 g, 0.018 mol) obtained in Example 5(2).
   Melting point: 185-187°C
   ¹H-NMR (CDCl₃) : δ 1.73 (3H, m), 1.92 (2H, m), 2.64 (1H, m), 2.74 (2H, m), 3.21 (2H, m), 3.87 (3H, s), 5.03 (2H, brs), 6.89 (1H, s), 6.98 (2H, d, J=8.6), 7.96 (2H, d, J=8.6).
(6) 2-Amino-4-(4-chlorophenyl)-6-(4-piperidinyl)-pyrimidine 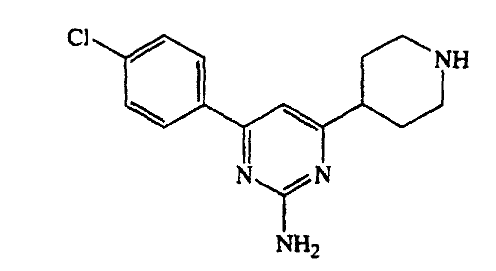
   By a procedure similar to that described in Example 11(1) and crystallization from isopropanol, the titled compound (4.47 g) was prepared from 2-amino-4-(1-benzoyl-4-piperidinyl)-6-(4-chlorophenyl)pyrimidine (7.0 g, 0.018 mol) obtained in Example 6(2).
   Melting point: 205-207°C
   ¹H-NMR (CDCl₃) : δ 1.72 (3H, m), 1.91 (2H, m), 2.74 (3H, m), 3.21 (2H, m), 5.07(2H, brs), 6.90 (1H, s), 7.43 (2H, d, J=8.6), 7.93 (2H, d, J=8.6).
(7) 2-Amino-4-(4-chlorophenylmethyl)-6-(4-piperidinyl)-pyrimidine 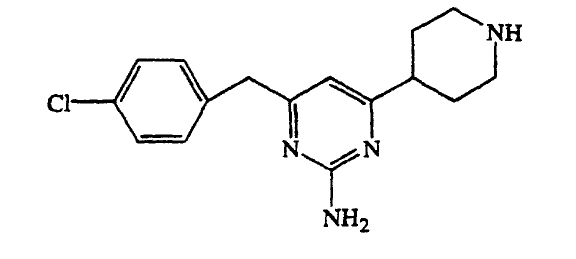
   By a procedure similar to that described in Example 11(1) and crystallization from ethanol-diethyl ether, the title compound (3.01 g) was prepared from 2-amino-4-(1-benzoyl-4-piperidinyl)-6-(4-chlorophenylmethyl) pyrimidine (5.3 g, 0.013 mol) obtained in Example 7(2).
   Melting point: 147-147.5°C
   ¹H-NMR (CDCl₃): δ 1.60 (3H, m), 1.80 (2H, m), 2.51 (1H, m), 2.68 (2H, dt, J=2.6, 12.2), 3.15 (2H, m), 3.84 (2H, s), 5.00 (2H, brs), 6.27 (1H, s), 7.17 (2H, d, J=8.6), 7.27 (2H. d, J=8.6).
(8) 2-Amino-6-methoxy-4-(4-piperidinyl)quinazoline 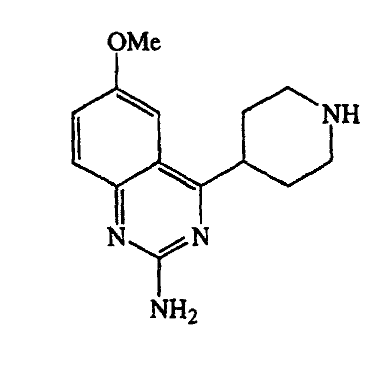
   By a procedure similar to that described in Example 11(1), the titled compound (2.60 g) was prepared from 2-amino-4-(1-benzoyl-4-piperidinyl)-6-methoxyquinazoline (4.15 g, 0.011 mol) obtained in Example 9(2).
   Melting point: 213-216°C
(9) 2-Amino-6-chloro-4-(4-piperidinyl)-quinazoline 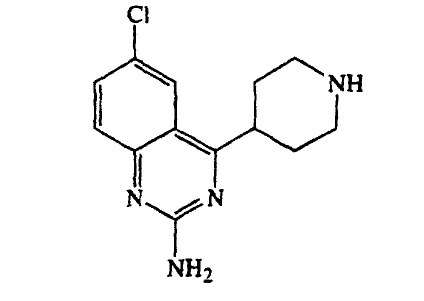
   By a procedure similar to that described in Example 11(1), the title compound (440 mg) was prepared from 2-amino-4-(1-benzoyl-4-piperidinyl) -6-chloroquinazoline (477 mg, 1.2 mmol) obtained in Example 10(2).
   ¹H-NMR (CDCl₃): δ 1.85-2.00 (4H, m), 2.85 (2H, m), 3.25 (2H, m), 3.45 (1H, m), 5.10 (2H, brs), 7.58 (2H, s), 7.90 (1H, d, J=2.3).
(10) The compounds shown in Tables 3-5 were prepared by a procedure similar to that described in Example 12 and Example 13 treating the piperidinyl derivatives obtained hereinabove such as 2-amino-4-(4-piperidinyl)quinazoline, 2-amino-6-methoxy-4-(4-piperidinyl)-quinazoline, 2-amino-6-chloro-4-(4-piperidinyl) -quinazoline, 2-amino-4-phenyl-6-(4-piperidinyl)pyrimidine, 2-amino-4- (4-methoxyphenyl)6-(4-piperidinyl)pyrimidine, 2-amino-4-(4-chlorophenyl)-6-(4-piperidinyl)-pyrimidine, 2-amino-4-methyl-5-(4-methoxyphenyl)-6-(4-piperidinyl)pyrimidine, 2-amino-4-(4-methoxyphenylmethyl)-6-(4-piperidinyl)pyrimidine, 2-amino-4-(4-chlorophenylmethyl)-6-(4-piperidinyl)pyrimidine, 2-amino-4-(2-phenylethyl)-6-(4-piperidinyl)-pyrimidine with various acid chlorides or carboxylic acids.

The acid chlorides may be prepared from the corresponding carboxylic acids in a conventional method well known to a skilled person in the art of organic chemistry, or some of them are commercially available.

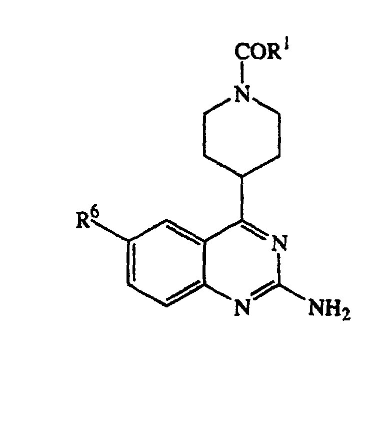

**Table 3**

| Example No | R¹ | R⁶ | Yield (%) | M.P. (°C) |
|---|---|---|---|---|
| 14 | cyclohexyl | H | 65 | 203-204 |
| 15 | cyclohexylmethyl | | 77 | 195-196 |
| 16 | -CH₂CH₃ | | 65 | 229-230 |
| 17 | -(CH₂)₂CH₃ | | 62 | 222-223 |
| 18 | -(CH₂)₃CH₃ | | 65 | 216-217 |
| 19 | -(CH₂)₄CH₃ | | 72 | 152-155 |
| 20 | -(CH₂)₅CH₃ | | 84 | amorphous |
| 21 | -(CH₂)₆CH₃ | | 77 | amorphous |
| 22 | -(CH₂)₇CH₃ | | 68 | amorphous |
| 23 | -(CH₂)₈CH₃ | | 84 | 87-90 |
| 24 | -(CH₂)₉CH₃ | | 90 | 91-92 |
| 25 | i-Propyl | | 85 | 217-218 |
| 26 | t-Butyl | | 98 | 249-253 |
| 27 | -(CH₂)₆NHBoc | | 95 | amorphous |
| 28 | -(CH₂)₇MHBoc | | 72 | amorphous |
| 29 | -(CH₂)₁₀NHBoc | | 96 | amorphous |
| 30 | -(CH₂)₉OH | | 80 | 117-118 |
| 31 | -CH₂OCH₃ | | 48 | 180-181 |
| 32 | 2-methoxyphenyl | | 84 | 254-256 |
| 33 | 3-methoxyphenyl | | 87 | amorphous |
| 34 | 4-methoxyphenyl | | 80 | 172-172.5 |
| 35 | 2-chlorophenyl | | 60 | amorphous |
| 36 | 3-chlorophenyl | | 86 | amorphous |
| 37 | 4-chlorophenyl | | 93 | 200.5-202 |
| 38 | 2-nitrophenyl | | 97 | amorphous |
| 39 | 3-nitrophenyl | H | 99 | amorphous |
| 40 | 4-nitrophenyl | | 87 | 203-204 |
| 41 | 2-fluorophenyl | | 96 | 193-195 |
| 42 | 3-fluorophenyl | | 99 | amorphous |
| 43 | 4-fluorophenyl | | 83 | amorphous |
| 44 | 2-methylphenyl | | 88 | 213-214 |
| 45 | 3-methylphenyl | | 86 | 178-179 |
| 46 | 4-methylphenyl | | 86 | 230-231 |
| 47 | 2-furyl | | 69 | 217-218 |
| 48 | 3-furyl | | 59 | 215-216 |
| 49 | 2-pyridyl | | 60 | 235-236 |
| 50 | 3-pyridyl | | 96 | 177-179 |
| 51 | 4-pyridyl | | 94 | 220-222 |
| 52 | 2,3-dimethoxyphenyl | | 85 | 205-207 |
| 53 | 2,4-dimethoxyphenyl | | 24 | 250-252 |
| 54 | 2,5-dimethoxyphenyl | | 69 | 201-203 |
| 55 | 2,6-dimethoxyphenyl | | 76 | 232-234 |
| 56 | 3,4-dimethoxyphenyl | | 71 | 173-175 |
| 57 | 3,5-dimethoxyphenyl | | 86 | 173-174 |
| 58 | 2,3-dichlorophenyl | | 79 | 256-258 |
| 59 | 2,4-dichlorophenyl | | 92 | 196-197 |
| 60 | 2,5-dichlorophenyl | | 97 | 125-128 |
| 61 | 2,6-dichlorophenyl | | 20 | 232-234 |
| 62 | 3,4-dichlorophenyl | | 100 | 192-193 |
| 63 | 3,5-dichlorophenyl | | 93 | 144-146 |
| 64 | 2,4-dinitrophenyl | H | 62 | 204-206 |
| 65 | 3,4-methylenedioxyphenyl | | 45 | 197-198 |
| 66 | 1-naphthyl | | 84 | 214-215 |
| 67 | 2-naphthyl | | 81 | 230-231 |
| 68 | 2,3,4-trimethoxyphenyl | | 57 | amorphous |
| 69 | 2,3,6-trimethoxyphenyl | | 57 | 230-231.5 |
| 70 | 2,4,5-trimethoxyphenyl | | 31 | 105-108 |
| 71 | 2,4,6-trimethoxyphenyl | | 29 | 125-127 |
| 72 | 3,4,5-trimethoxyphenyl | | 88 | 199-202 |
| 73 | 2,3,5-trichlorophenyl | | 87 | 241-242 |
| 74 | 2,3,6-trichlorophenyl | | 33 | 225-227 |
| 75 | 2,4,6-trichlorophenyl | | 40 | 213-214 |
| 76 | benzyl | | 53 | 225-226 |
| 77 | 2-methoxybenzyl | | 97 | amorphous |
| 78 | 3-methoxybenzyl | | 100 | amorphous |
| 79 | 4-methoxybenzyl | | 90 | 220-221 |
| 80 | 2-chlorobenzyl | | 99 | 187-188 |
| 81 | 3-chlorobenzyl | | 92 | 190-191 |
| 82 | 4-chlorobenzyl | | 89 | 206-207 |
| 83 | 2-fluorobenzyl | | 98 | 204-205 |
| 84 | 3-fluorobenzyl | | 89 | 207.5-209 |
| 85 | 4-fluorobenzyl | | 96 | 196-198 |
| 86 | 2-nitrobenzyl | | 79 | 202-203 |
| 87 | 3-nitrobenzyl | | 99 | amorphous |
| 88 | 4-nitrobenzyl | H | 94 | 205-206 |
| 89 | 2-pyridylmethyl | | 77 | 168-170 |
| 90 | 3-pyridylmethyl | | 99 | 232-235 |
| 91 | 2,5-dimethoxybenzyl | | 94 | 147-148 |
| 92 | 3,4-dimethoxybenzyl | | 98 | 158-159 |
| 93 | 3,5-dimethoxybenzyl | | 95 | 108-110 |
| 94 | 2,4-dichlorobenzyl | | 84 | 224-225 |
| 95 | 2,6-dichlorobenzyl | | 80 | 202-203 |
| 96 | 3,4-dichlorobenzyl | | 98 | 149-150 |
| 97 | 2,4-dinitrobenzyl | | 69 | 229-231 |
| 98 | 2,3,6-trichlorobenzyl | | 80 | 208-209 |
| 99 | 3,4,5-trimethoxybenzyl | | 49 | 188-189 |
| 100 | 2-phenylethyl | | 69 | amorphous |
| 101 | 2-(3,4-methylenedioxyphenyl)ethyl | | 71 | 160-163 |
| 102 | 3-phenylpropyl | | 58 | amorphous |
| 103 | -(CH₂)₅CH₃ | OCH₃ | 82 | 124-125 |
| 104 | -(CH₂)₆CH₃ | | 90 | 138-139 |
| 105 | -(CH₂)₇CH₃ | | 82 | 104-106 |
| 106 | -(CH₂)₈CH₃ | | 74 | 84-86 |
| 107 | -(CH₂)₉CH₃ | | 81 | 94-96 |
| 108 | 2-methoxyphenyl | | 100 | 196-197 |
| 109 | 3-methoxyphenyl | | 98 | amorphous |
| 110 | 4-methoxyphenyl | | 100 | 201-202 |
| 111 | 2-chlorophenyl | | 100 | 201-202 |
| 112 | 3-chlorophenyl | OCH₃ | 100 | 186-187 |
| 113 | 4-chlorophenyl | | 88 | 218-221 |
| 114 | 2-nitrophenyl | | 100 | 218-220 |
| 115 | 3-nitrophenyl | | 95 | 240-242 |
| 116 | 4-nitrophenyl | | 60 | 210-212 |
| 117 | 3,4-methylenedioxyphenyl | | 71 | 260 dec. (hydrochloride) |
| 118 | 2-methoxybenzyl | | 97 | amorphous |
| 119 | 3-methoxybenzyl | | 64 | 150-152 |
| 120 | 4-methoxybenzyl | | 67 | 165-167 |
| 121 | 2-chlorobenzyl | | 100 | 173-174 |
| 122 | 3-chlorobenzyl | | 80 | 172-173 |
| 123 | 4-chlorobenzyl | | 100 | 179-180 |
| 124 | 2-nitrobenzyl | | 93 | 215-218 |
| 125 | 3-nitrobenzyl | | 89 | 195-196.5 |
| 126 | 4-nitrobenzyl | | 100 | 198-199 |
| 127 | -(CH₂)₆CH₃ | Cl | 62 | 135-136 |
| 128 | -(CH₂)₇CH₃ | | 52 | 225-228 |
| 129 | -(CH₂)₈CH₃ | | 56 | 230-233 |
| 130 | 3,4-methylenedioxyphenyl | | 41 | 250 dec. (hydrochloride) |
| 131 | 4-methoxybenzyl | | 60 | 193-195 |
| 132 | 4-chlorobenzyl | | 60 | 188-192 |
| 133 | 4-nitrobenzyl | | 26 | 233 dec. |

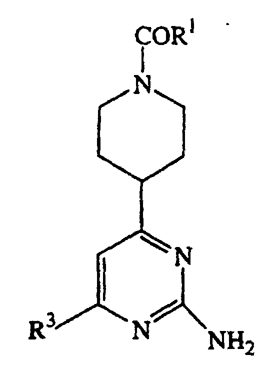

**Table 4**

| Example No | R¹ | R³ | Yield (%) | M.P. (°C) |
|---|---|---|---|---|
| 134 | cyclohexyl | Ph | 88 | amorphous |
| 135 | cyclohexylmethyl | | 76 | amorphous |
| 136 | -(CH₂)₆CH₃ | | 89 | 112-113 |
| 137 | -(CH₂)₇CH₃ | | 100 | 140-141 (hydrochloride) |
| 138 | -(CH₂)₈CH₃ | | 100 | amorphous |
| 139 | 2-furyl | | 77 | 122-123 |
| 140 | 3,4-methylenedioxyphenyl | | 77 | 201-202 |
| 141 | benzyl | | 74 | 141-142 |
| 142 | 4-methoxybenzyl | | 100 | amorphous |
| 143 | 4-chlorobenzyl | | 100 | 157-158 |
| 144 | 4-nitrobenzyl | | 100 | amorphous |
| 145 | 3,4-methylenedioxybenzyl | | 68 | amorphous |
| 146 | 3,4,5-trimethoxybenzyl | | 48 | 98-100 |
| 147 | -(CH₂)₆CH₃ | 4-chlorophenyl | 93 | 118-120 |
| 148 | -(CH₂)₇CH₃ | | 92 | 97-98 |
| 149 | -(CH₂)₈CH₃ | | 94 | 108-109 |
| 150 | 3,4-methylenedioxyphenyl | | 84 | 103-106 |
| 151 | 4-methoxybenzyl | 4-chlorophenyl | 99 | 178.5-180 |
| 152 | 4-chlorobenzyl | | 89 | 183-184 |
| 153 | 4-nitrobenzyl | | 94 | 200-202 |
| 154 | -(CH₂)₆CH₃ | 4-methoxyphenyl | 93 | 118-120 |
| 155 | -(CH₂)₇CH₃ | | 92 | 97-98 |
| 156 | -(CH₂)₈CH₃ | | 94 | 108-109 |
| 157 | 3,4-methylenedioxyphenyl | | 84 | 103-106 |
| 158 | 4-methoxybenzyl | | 99 | 178.5-180 |
| 159 | 4-chlorobenzyl | | 89 | 183-184 |
| 160 | 4-nitrobenzyl | | 94 | 200-202 |
| 161 | cyclohexyl | 4-methoxybenzyl | 76 | 159-160 |
| 162 | -CH₃ | | 89 | 203-205 |
| 163 | -CH₂CH₃ | | 93 | 133-135 |
| 164 | -(CH₂)₂CH₃ | | 98 | 140-141 |
| 165 | -(CH₂)₃CH₃ | | 94 | 100-103 |
| 166 | -(CH₂)₄CH₃ | | 100 | 120-121 |
| 167 | -(CH₂)₅CH₃ | | 100 | 108-109 |
| 168 | -(CH₂)₆CH₃ | | 98 | 101-103 |
| 169 | -(CH₂)₇CH₃ | | 100 | 114-116 |
| 170 | -(CH₂)₈CH₃ | | 100 | 102-104 |
| 171 | -(CH₂)₉CH₃ | | 100 | 88-90 |
| 172 | i-Propyl | | 100 | 163-163.5 |
| 173 | t-Butyl | | 96 | 151-152 |
| 174 | -(CH₂)₂Cl | | 91 | 131-134 |
| 175 | -(CH₂)₂Br | | 96 | 90-91 |
| 176 | -(CH₂)₂NHBoc | 4-methoxybenzyl | 100 | amorphous |
| 177 | -(CH₂)₂OH | | 59 | amorphous |
| 178 | 2-methoxyphenyl | | 100 | 161-162 |
| 179 | 3-methoxyphenyl | | 100 | 149-151 |
| 180 | 4-methoxyphenyl | | 100 | 169-170.5 |
| 181 | 2-chlorophenyl | | 100 | 170-171.5 |
| 182 | 3-chlorophenyl | | 100 | 141-142 |
| 183 | 4-chlorophenyl | | 100 | 188-189 |
| 184 | 2-nitrophenyl | | 100 | 161-162 |
| 185 | 3-nitrophenyl | | 100 | 153-155 (hydrochloride) |
| 186 | 4-nitrophenyl | | 100 | amorphous (hydrochloride) |
| 187 | 2-fluorophenyl | | 100 | 181-183 |
| 188 | 3-fluorophenyl | | 100 | 168-170.5 |
| 189 | 4-fluorophenyl | | 94 | 176-177 |
| 190 | 2-methylphenyl | | 99 | 147-148.5 |
| 191 | 3-methylphenyl | | 93 | 175-176 |
| 192 | 4-methylphenyl | | 99 | 183-184 |
| 193 | 2-furyl | | 83 | 124-125 |
| 194 | 2-pyridyl | | 81 | 157-158 |
| 195 | 3-pyridyl | | 45 | 153.5-154.5 |
| 196 | 4-pyridyl | | 100 | 148-150 |
| 197 | 2,3-dimethoxyphenyl | | 86 | 147-148 |
| 198 | 2,4-dimethoxyphenyl | | 39 | 194-196 |
| 199 | 2,5-dimethoxyphenyl | | 59 | 135-136 |
| 200 | 2,6-dimethoxyphenyl | | 100 | 144-145 |
| 201 | 3,4-dimethoxyphenyl | 4-methoxybenzyl | 99 | 159-160 |
| 202 | 3,5-dimethoxyphenyl | | 100 | 198-200 |
| 203 | 2,3-dichlorophenyl | | 100 | 157-158 |
| 204 | 2,4-dichlorophenyl | | 100 | 147-148 |
| 205 | 2,5-dichlorophenyl | | 100 | 155-156 |
| 206 | 2,6-dichlorophenyl | | 85 | 178-179 |
| 207 | 3,4-dichlorophenyl | | 98 | 195-196 |
| 208 | 3,5-dichlorophenyl | | 100 | 154-155 |
| 209 | 2,4-dinitrophenyl | | 89 | 116-119 |
| 210 | 3,4-methylenedioxyphenyl | | 96 | 170-171 |
| 211 | 1-naphtyl | | 78 | 161-162 |
| 212 | 2-naphtyl | | 79 | 220-221.5 |
| 213 | 2,3,4-trimethoxyphenyl | | 50 | 123-125 |
| 214 | 2,3,6-trimethoxyphenyl | | 90 | amorphous |
| 215 | 2,4,5-trimethoxyphenyl | | 35 | 189-190 |
| 216 | 2,4,6-trimethoxyphenyl | | 73 | amorphous |
| 217 | 3,4,5-trimethoxyphenyl | | 94 | amorphous |
| 218 | 2,3,5-trichlorophenyl | | 100 | 189-190 |
| 219 | 2,3,6-trichlorophenyl | | 89 | amorphous |
| 220 | 2,4,6-trichlorophenyl | | 100 | 159-160 |
| 221 | benzyl | | 68 | 140-140.5 |
| 222 | 2-methoxybenzyl | | 96 | 134-136 |
| 223 | 3-methoxybenzyl | | 57 | 179-180 |
| 224 | 4-methoxybenzyl | | 100 | 129-130 |
| 225 | 2-chlorobenzyl | | 100 | 190-193 |
| 226 | 3-chlorobenzyl | 4-methoxybenzyl | 97 | 178-181 |
| 227 | 4-chlorobenzyl | | 92 | 158-159.5 |
| 228 | 2-flurobenzyl | | 100 | 170-171 |
| 229 | 3-fluorobenzyl | | 100 | 156-158.5 |
| 230 | 4-fluorobenzyl | | 99 | 159-160 |
| 231 | 2-nitrobenzyl | | 100 | 198-200 |
| 232 | 3-nitrobenzyl | | 100 | 162-164 |
| 233 | 4-nitrobenzyl | | 94 | 149-150 |
| 234 | 2-pyridylmethyl | | 74 | 131-132 |
| 235 | 3-pyridylmethyl | | 48 | 109-112 |
| 236 | 2,5-dimethoxybenzyl | | 97 | 130-132 |
| 237 | 3,4-dimethoxybenzyl | | 100 | amorphous |
| 238 | 3,5-dimethoxybenzyl | | 91 | amorphous |
| 239 | 2,4-dichlorobenzyl | | 100 | 182-184 |
| 240 | 2,6-dichlorobenzyl | | 99 | amorphous |
| 241 | 3,4-dichlorobenzyl | | 93 | 150-151 |
| 242 | 2,4-dinitrobenzyl | | 96 | 131-132 |
| 243 | 3,4-methylenedioxybenzyl | | 86 | 155-156 |
| 244 | 2,3,6-trichlorobenzyl | | 100 | amorphous |
| 245 | 3,4,5-trimethoxybenzyl | | 96 | amorphous |
| 246 | 2-(3,4-methylenedioxyphenyl)ethyl | | 71 | 123-125 |
| 288 | 1,3-benzo-dioxol-4-yl | | 82 | 167-167.5 |
| 289 | 5-benzimidazolyl | | 81 | amorphous |
| 290 | 5-indolyl | | 93 | amorphous |
| 291 | 1,4-benzo-dioxan-6-yl | | 92 | 187-188 |
| 292 | 3,4-difluorophenyl | | 69 | 159-160 |
| 293 | 5-benzo[b]furanyl | | 77 | 188-188.5 |
| 294 | 6-benzo[b]furanyl | | 88 | 186-187 |
| 295 | 4-benzo[b]furanyl | | 90 | 173-174 |
| 296 | 2,3-dihydrobenzo[b]furan-5-yl | | 98 | 162-164 |
| 297 | 5-benzoxazolyl | | 88 | 178-179 |
| 298 | 6-benzoxazolyl | | 83 | 185-186 |
| 299 | 6-benzothiazolyl | | 75 | 153-154 |
| 300 | 4-hydroxy-3-methoxyphenyl | | 88 | 131-135 (hydrochloride) |
| 301 | 3-hydroxy-4-methoxyphenyl | | 96 | amorphous (hydrochloride) |
| 247 | -(CH₂)₆CH₃ | 4-chlorobenzyl | 70 | 142-143 |
| 248 | -(CH₂)₇CH₃ | | 79 | 137.5-138.5 |
| 249 | -(CH₂)₈CH₃ | | 70 | 136-137 |
| 250 | 3,4-methylenedioxyphenyl | | 85 | 162-163 |
| 251 | 4-methoxybenzyl | | 82 | 132-133 |
| 252 | 4-chlorobenzyl | | 77 | 163-164 |
| 253 | 3,4-methylenedioxybenzyl | | 89 | 203-204 |
| 254 | cyclohexyl | 2-phenylethyl | 78 | 120-121 |
| 255 | -(CH₂)₆CH₃ | | 99 | 83-84 |
| 256 | -(CH₂)₇CH₃ | | 94 | 72-73 |
| 257 | -(CH₂)₈CH₃ | | 92 | 77-78 |
| 258 | 2-furyl | | 84 | 109-110 |
| 259 | 3,4-methylenedioxyphenyl | | 85 | 158-158.5 |
| 260 | benzyl | | 47 | amorphous |
| 261 | 4-methoxybenzyl | | 81 | 135-136 |
| 262 | 4-chlorobenzyl | | 80 | 144-145.5 |
| 263 | 4-nitrobenzyl | | 91 | 169-170 |

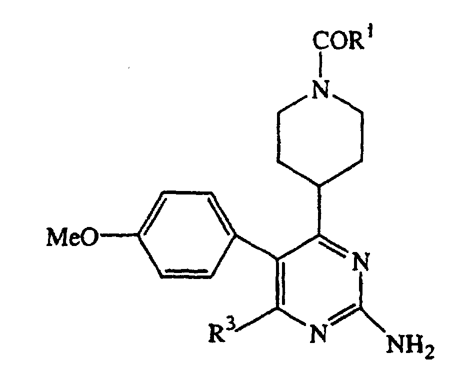

**Table 5**

| Example No. | R¹ | Yield (%) | M.P. (°C) |
|---|---|---|---|
| 264 | cyclohexyl | 74 | 225-226 |
| 265 | 2-furyl | 82 | 236-237 |
| 266 | 3,4-methylenedioxyphenyl | 97 | 220-222 |
| 267 | benzyl | 61 | 196-197 |

### Example 268-277

The urethane and sulfonamide derivatives in Tables 6 and 7 were prepared by a procedure similar to that described in Example 12, treating various chloroformates or sulfonyl chlorides instead of acid chlorides with piperidinyl derivatives, i.e., 2-amino-4-(4-piperidinyl)quinazoline and 2-amino-4-(4-methoxyphenylmethyl) -6- (4-piperidinyl)pyrimidine.

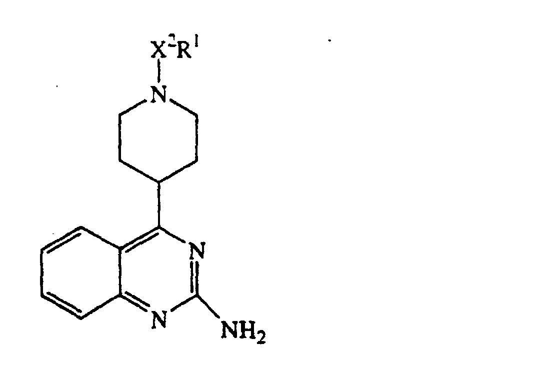

### Example 268-277

**Table 6**

| Example No. | X² | R¹ | Yield (%) | M.P. (°C) |
|---|---|---|---|---|
| 268 | -(CO)O- | -Ph | 93 | amorphous |
| 269 | | -CH₂Ph | 90 | 148-150 |
| 270 | -SO₂- | -(CH₂)₇CH₃ | 57 | 144-145 |
| 271 | | -Ph | 89 | 212-214 |
| 272 | | -CH₂Ph | 48 | 234-235 |

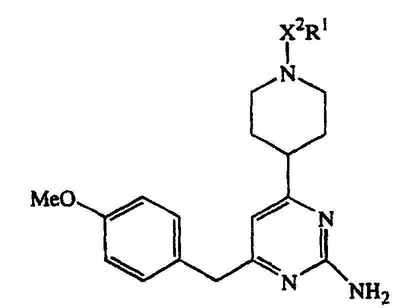

**Table 7**

| Example No. | X² | R¹ | Yield (%) | M.P. (°C) |
|---|---|---|---|---|
| 273 | -(CO)O- | -Ph | 72 | 164-166 |
| 274 | | -CH₂Ph | 61 | 160-161 |
| 275 | -SO₂- | -(CH₂)₇CH₃ | 55 | 135-136 |
| 276 | | -Ph | 84 | 156-157.5 |
| 277 | | -CH₂Ph | 60 | 177-178 |

### Example 278 Synthesis of 2-amino-4-(1-phenylcarbamoyl-4-piperidinyl)quinazoline

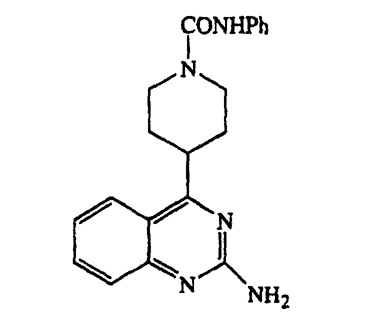

To a solution of 2-amino-4-(4-piperidinyl)-quinazoline (100 mg, 0.44 mmol) obtained in Example 11(1) and triethylamine (133 mg, 1.3 mmol) in dichloromethane (50 ml) was added phenyl isocyanate (50 mg, 0.42 mmol) under a nitrogen atmosphere. The mixture was stirred for about 3 hours at room temperature. The reaction mixture was washed with saturated sodium hydrogencarbonate aqueous solution. The organic layer was dried over sodium sulfate and evaporated in vacuo. The residue was purified by silicagel column chromatography (chloroform: methanol = 99:1) to give the titled compound (127 mg).
Melting point: 178-180°C

### Example 279 Synthesis of 2-amino-4-(1-octylcarbamoyl-4-piperidinyl)quinazoline

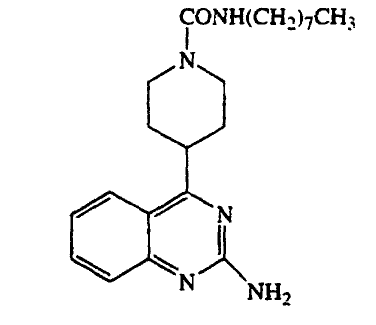

By a procedure similar to that described in Example 278, the titled compound (122 mg) was prepared from 2-amino-4-(4-piperidinyl)quinazoline (100 mg, 0.44 mmol) obtained in Example 11(1).
Melting point: 85-87°C

### Example 280 Synthesis of 2-amino-4-(1-benzyl-carbamoyl-4-piperidinyl)quinazoline

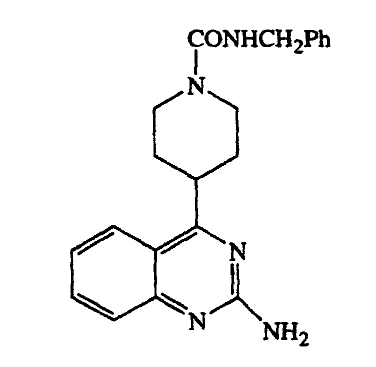

By a procedure similar to that described in Example 278, the titled compound (132 mg) was prepared from of 2-amino-4-(4-piperidinyl)quinazoline (100 mg, 0.44 mmol) obtained in Example 11(1).
Melting point: 217-218°C

### Example 281 Synthesis of 2-amino-4-(4-methoxyphenylmethyl)-6-(1-phenylcarbamoyl-4-piperidinyl)-pyrimidine

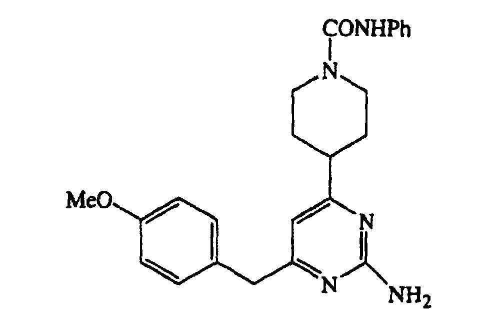

By a procedure similar to that described in Example 278, the titled compound (108 mg) was prepared from 2-amino-4-(4-methoxyphenylmethyl)-6-(4-piperidinyl)-pyrimidine (100 mg, 0.34 mmol).
Melting point: 178-180°C

### Example 282 Synthesis of 2-amino-4-(4-methoxyphenylmethyl)-6-(1-octylcarbamoyl-4-piperidinyl)pyrimidine

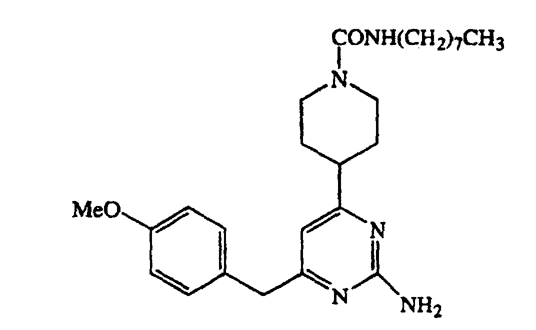

By a procedure similar to that described in Example 278, the titled compound (120 mg) was prepared from of 2-amino-4-(4-methoxyphenylmethyl)-6-(4-piperidinyl)pyrimidine(100 mg, 0.34 mmol).
Melting point: 104-106°C

### Example 283 Synthesis of 2-amino-4-(1-benzylcarbamoyl-4-piperidinyl) -6-(4-methoxyphenylmethyl)pyrimidine

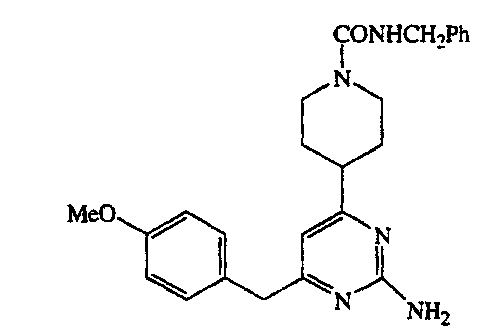

By a procedure similar to that described in Example 278, the titled compound (119 mg) was prepared from of 2-amino-4-(4-methoxyphenylmethyl) -6-(4-piperidinyl)pyrimidine (100 mg, 0.34 mmol).
Melting point: 128-131°C

### Example 284 Synthesis of 2-amino-4-[1-(7-aminoheptanoyl)-4-piperidinyl]quinazoline

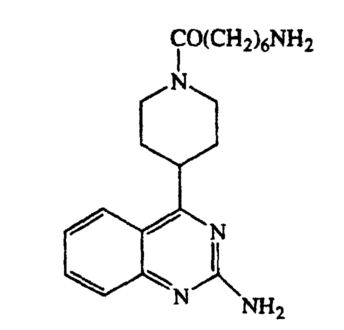

To a solution of 2-amino-4-[1-(7-t-butoxycarbonylaminoheptanoyl)-4-piperidinyl]quinazoline (160 mg) obtained in Example 27 in tetrahydrofuran (1 ml) was added 4 N hydrogen chloride-dioxane solution (1 ml). The reaction mixture was stirred for about 1 hour at room temperature and was evaporated. The residue was triturated with diethyl ether to give the title compound (131 mg) as hydrochloride.
Melting point: 230-233°C

### Example 285 Synthesis of 2-amino-4-[1-(8-aminooctanoyl)-4-piperidinyl]quinazoline

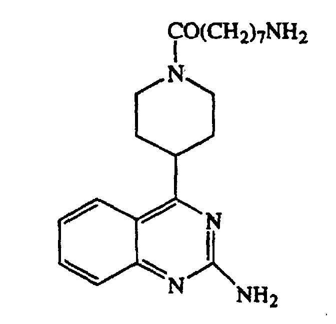

By a procedure similar to that described in Example 284, the titled compound (91 mg) as hydrochloride was prepared from 2-amino-4-[1-(8-t-butoxycarbonylaminooctanoyl)-4-piperidinyl]quinazoline (100 mg) obtained in Example 28.
Melting point: 222-224°C

### Example 286 Synthesis of 2-amino-4-[1-(11-aminoundecanoyl)-4-piperidinyl] quinazoline

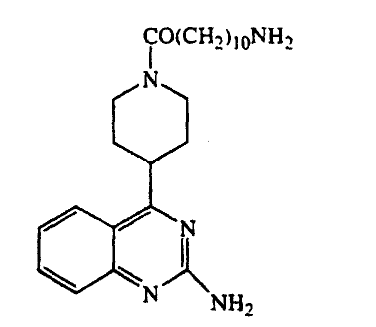

By a procedure similar to that described in Example 284, the titled compound (130 mg) as hydrochloride was prepared from 2-amino-4-[1-(11-t-butoxycarbonylaminoundecanoyl)-4-piperidinyl]quinazoline (170 mg) obtained in Example 29.
Melting point: 163-165°C

### Example 287 Synthesis of 2-amino-4-[1-(3-aminopropanoyl)-4-piperidinyl]-6-(4-methoxyphenylmethyl)pyrimidine

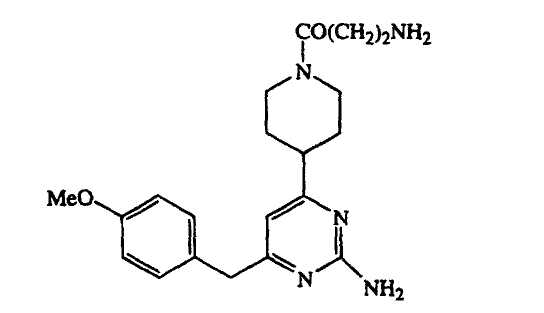

By a procedure similar to that described in Example 284, the titled compound (127 mg) as hydrochloride was prepared from 2-amino-4-[1-(3-t-butoxycarbonylaminopropanoyl)-4-piperidinyl]-6-(4-methoxyphenylmethyl)pyrimidine (170 mg) obtained in Example 176.
Melting point: amorphous

### Example 302 Synthesis of 2-amino-4-[1-(3,4-dihydroxybenzoyl)-4-piperidinyl]-6-(4-methoxyphenylmethyl)-pyrimidine

(1) 2-Amino-4-[1-(3,4-dibenzyloxybenzoyl)-4-piperidinyl]-6-(4-methoxyphenylmethyl) pyrimidine 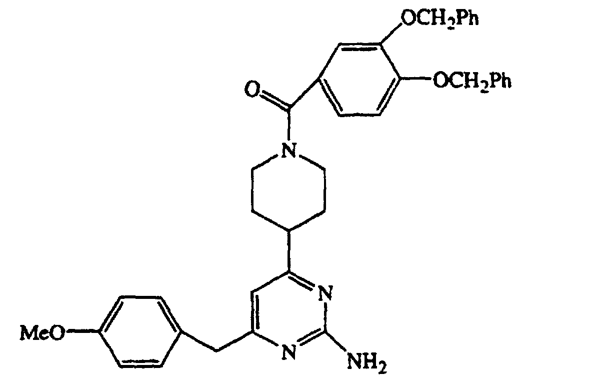
   To a solution of 2-amino-4-(4-methoxyphenylmethyl)-6-(4-piperidinyl)pyrimidine (160 mg, 0.54 mmol), 3,4-dibenzyloxybenzoic acid (179 mg, 0.54 mmol) and 1-hydroxybenzotriazole (72 mg, 0.54 mmol) in dichloromethane (150 ml) were added 1-ethyl-3-(N, N'-dimethylaminopropyl)carbodiimide hydrochloride (103 mg, 0.54 mmol) at room temperature under nitrogen atmosphere. After stirring for about 30 minutes, the mixture was washed successively with saturated sodium hydrogen carbonate solution and brine. The organic layer was dried over anhydrous sodium sulfate and then evaporated. The residue was purified by column chromatography (chloroform : methanol = 99 : 1) to give the titled compound (477 mg).
   ¹H-NMR (CDCl₃) : δ 1.77 (4H, m), 2.59 (1H, m), 2.84 (2H, m), 3.78 (3H, s), 3.82 (2H, s), 3.90 (1H, m), 4.70 (1H, m), 5.11 (2H, s), 5.17 (2H, s), 5.18 (2H,s), 6.24 (1H, s), 6.84-7.00 (5H, m), 7.14-7.44 (12H, m).
(2) 2-Amino-4-[1-(3,4-dihydroxybenzoyl)-4-piperidinyl]-6-(4-methoxyphenylmethyl) pyrimidine 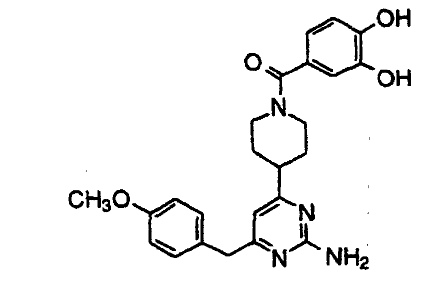
   2-Amino-4-[1-(3,4-dibenzyloxybenzoyl)-4-piperidinyl]-6-(4-methoxyphenylmethyl)pyrimidine (293 mg, 0.48 mmol) was dissolved in a mixture of methanol (3 ml) and ethyl acetate (3 ml), and hydrogenated under 1 atm in the presence of 10% palladium on carbon (80 mg) for about 3 hours. The catalyst was filtered off and washed with methanol. The filtrate was concentrated in vacuo and the residue was purified by column chromatography (chloroform : methanol = 97 : 3) to give the titled compound (155 mg).
   ¹H-NMR (CDCl₃): δ 1.74 (4H, m), 2.62 (1H, m), 2.87 (2H, m), 3.77 (3H, s), 3.82 (2H, s), 3.87 (1H, m), 4.65 (1H, m), 5.35 (2H, s), 6.25 (1H, s), 6.79 (5H, m), 7.14 (2H, d, J=8.6), 7.26 (2H, s).

### Example 303 Synthesis of 2-amino-4-(4-bromophenylmethyl)-6-[1-(3,4-methylenedioxybenzoyl)-4-piperidinyl] pyrimidine

(1) 1-(3,4-Methylenedioxybenzoyl)isonipecotic acid 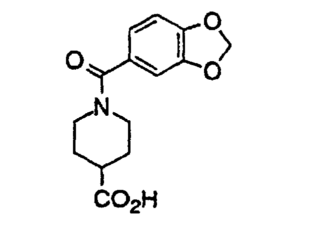
   To an ice cold solution of isonipecotic acid (119.77 g, 0.927mol) and potassium carbonate (256.32 g, 1.855mol) in water (900 ml) was added dropwise piperonyloyl chloride (155.6 g, 0.843mol) in tetrahydrofuran (900ml) with vigorous stirring over about 2 hours. The reaction temperature was kept below about 10°C during the addition and the mixture was then stirred for about 30 minutes. The reaction mixture was acidified with conc. hydrochloric acid and extracted with chloroform. The organic layer was washed with water and dried over anhydrous magnesium sulfate, then evaporated. The residue was crystallized from methanol to give the titled compound (210.81 g)
   ¹H-NMR (CDCl₃) : δ 1.49(2H, m), 1.81(2H, m), 2.51(1H, m), 2.99(2H, m), 3.98(2H, m), 6.07(2H, s), 6.88(1H, dd, J=1.3, J=7.9), 6.95(2H, m), 12.29(1H, s).
(2) Ethyl 3-[1-(3,4-methylenedioxybenzoyl)-4-piperidinyl)]-3-oxopropionate 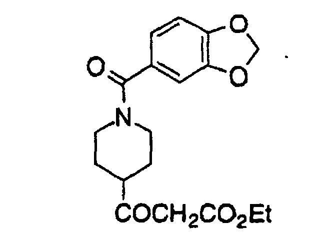
   To a THF solution(2 L) of 1-(3,4-methylenedioxybenzoyl)isonipecotic acid (110 g, 0.397 mol) was added carbonyldiimidazole (70.8 g, 0.437 mol) in several portions at room temperature under nitrogen atmosphere. After stirring for about 3.5 hours, magnesium ethyl malonate (125.12 g, 0.437 mol) was added and refluxed for about 3 hours. The solvent was removed under reduced pressure. The residue was partitioned between ethyl acetate (3 L) and saturated sodium hydrogen carbonate (1.5 L). The organic layer was washed with brine twice and dried over anhydrous magnesium sulfate, then evaporated. The residue was purified by column chromatography (hexane : ethyl acetate = 1 : 1) to give the titled compound as colorless oil (132.40 g).
   Magnesium ethyl malonate was prepared as follows: To a stirred THF solution (1 L) of monoethyl malonate (183.18 g, 1.39 mol) was added magnesium ethoxide (79.54 g, 0.70 mol) in several portions. After about 4 hours, the solvent was removed under reduced pressure and the residue was reevaporated with toluene to give magnesium ethyl malonate (190.82 g).
(3) 4-{1-[4-(4-Bromophenyl)-1,3-dioxobutyl]}-1-(3,4-methylenedioxybenzoyl) piperidine 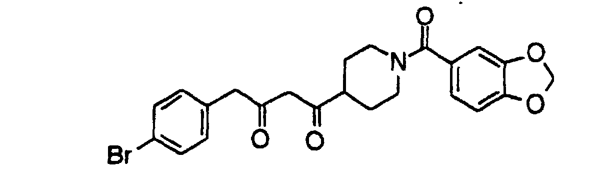
   To an ice cooled solution of ethyl 3-[1-(3,4-methylenedioxybenzoyl)-4-piperidinyl)]-3-oxo-propionate (7 g, 20 mmol) in tetrahydrofuran (35 ml) was added sodium hydride (60% oil dispersion, 1.68 g, 42 mmol) in several portions under nitrogen atmosphere. The reaction temperature was kept below about 5°C during addition and the mixture was stirred for about a further 30 minutes. To the mixture 4-bromophenylacetyl chloride (5.18 g, 0.22 mmol) in tetrahydrofuran (15 ml) was added dropwise over about one hour at about 5°C. After stirring about further 30 minutes, the mixture was acidified by 4 N-hydrochloric acid and was extracted with ethyl acetate. The organic layer was washed with water and dried over anhydrous sodium sulfate, then evaporated to give a diketoester as a crude oil. The diketoester was used following reaction without further purification. The mixture of diketoester, water (1.6 ml) and dimethylsufoxide (18 ml) was heated at about 110°C for about 7.5 hours. The mixture was poured into water and extracted with ethyl acetate. The organic layer was washed with water and dried over anhydrous sodium sulfate, then evaporated. The residue was purified by flash column chromatography (hexane : ethyl acetate = 1 : 1) to give the titled compound (5.54 g) as an oil.
   ¹H-NMR (CDCl₃): δ 1.63(2H, m), 1.81(2M, m), 2.42(1H, m), 2.91(2H, m,), 3.57(2H, s), 3.9-4.9(2H,m), 5.43(1H, s), 6.00(2H, s), 6.86(3H, m), 7.10(2H, d, J=8.6), 7.47(2H, d, J=8.6), 15.37(1H, s).
(4) 2-Amino-4-(4-bromophenylmethyl)-6-[1-(3,4-methylenedioxybenzoyl)-4-piperidinyl)pyrimidine

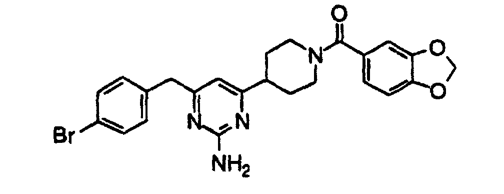

A mixture of 4-{1-[4-(4-bromophenyl)-1,3-dioxobutyl]}-1-(3,4-methylenedioxybenzoyl) piperidine (5.3 g, 11.2 mmol), guanidine carbonate (2.02 g, 11.2 mmol) and pyridine (25 ml) was heated at about 110°C for about 8 hours. The mixture was evaporated in vacuo and the residue was partitioned between chloroform and saturated sodium hydrogen carbonate aqueous solution. The organic layer was washed with brine and dried over anhydrous sodium sulfate, then evaporated. Crystallization from ethanol gave the titled compound (3.88 g). Melting point: 162-162.5°C
¹H-NMR (CDCl₃) : δ 1.65-1.84(4H, m), 2.66(1H, m), 2.92(2H, m), 3.83(2H, s), 4-4.8(2H,m), 4.96(2H, s), 6.00(2H, s), 6.26(1H, s), 6.82(1H, d, J=8.3), 6.93(2H, m), 7.12(2H, d, J=8.6), 7.44(2H, d, J=8.25).

The free amine (300 mg) was dissolved in chloroform and treated with 1 N hydrogen chloride-diethyl ether solution (1 ml). The solvent was removed in vacuo. The residue was dissolved in small amount of isopropanol and triturated with ether to give the hydrochloride (300 mg) of the titled compound.

### Example 304-314

The amide compounds shown in Table 8 were prepared by a similar method described in Example 303 using a reaction of ethyl 3-[1-(3,4-methylenedioxybenzoyl)-4-piperidinyl)]-3-oxo-propionate with various carboxylic acids.

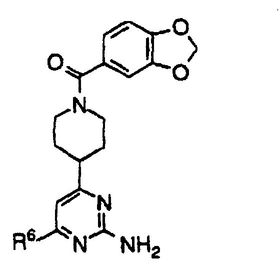

**Table 8**

| Example No. | R⁶ | Yield (%) | M.P. (°C) |
|---|---|---|---|
| 304 | benzyl | 39 | 115-118 (hydrochloride) |
| 305 | 3-methoxybenzyl | 68 | amorphous (hydrochloride) |
| 306 | 3,4-dimethoxybenzyl | 55 | 150-151 |
| 307 | 2,5-dimethoxybenzyl | 60 | 150-152 |
| 308 | 3,5-dimethoxybenzyl | 52 | 146-149 (hydrochloride) |
| 309 | 3,4,5-trimethoxybenzyl | 26 | 154-155 |
| 310 | 2-chlorobenzyl | 21 | amorphous (hydrochloride) |
| 311 | 3,4-dichlorobenzyl | 31 | 169-170 |
| 312 | 2,4-dichlorobenzyl | 32 | 205-206 |
| 313 | 2,6-dichlorobenzyl | 35 | 213-214 |
| 314 | 3,4-methylenedioxybenzyl | 58 | amorphous (hydrochloride) |

### Example 315 Synthesis of 2-amino-4-(4-hydroxyphenylmethyl)-6-(1-(3,4-methylenedioxybenzoyl)-4-piperidinyl] pyrimidine

(1) 2-Amino-4-(4-benzyloxyphenylmethyl)-6-[1-(3,4-methylenedioxybenzoyl)-4-piperidinyl] pyrimidine 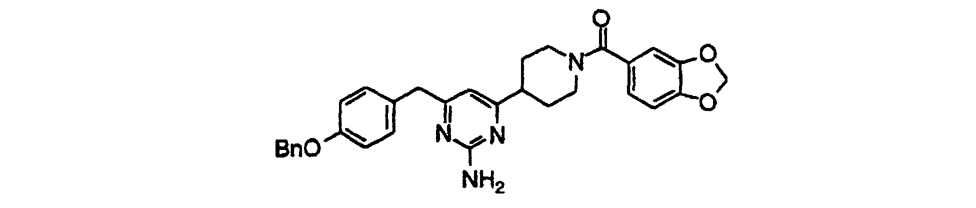
   According to similar procedures described in Example 303 (3) and (4), the titled compound (570 mg) was prepared by treating ethyl 3-[1-(3,4-methylenedioxybenzoyl)-4-piperidinyl)]-3-oxo-propionate(2.0 g) with 4-benzyloxyphenylacetyl chloride (1.66 g).
   ¹H-NMR (CDCl₃): δ 1.77 (4H, m), 2.65 (1H, m), 2.91 (2H, m), 3.83 (2H, s), 4-4.8 (2H, m), 4.93 (2H, s), 5.05 (2H, s), 5.99 (2H, s), 6.27 (1H, s), 6.81 (2H, d, J=8.1), 6.92 (3H, m), 7.15 (2H, d, J=8.6), 7.38 (5H, m).
(2) 2-Amino-4-(4-hydroxyphenylmethyl)-6-[1-(3,4-methylenedioxybenzoyl)-4-piperidinyl] pyrimidine 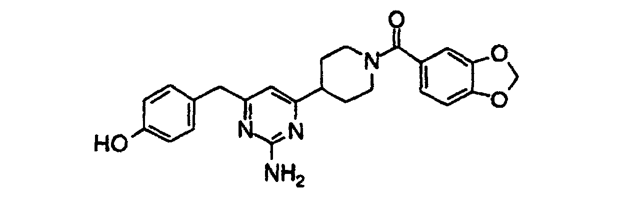
   2-Amino-4-(4-benzyloxyphenylmethyl)-6-[1-(3,4-methylenedioxybenzoyl)-4-piperidinyl) pyrimidine (570 mg, 1.09 mmol) was dissolved in acetic acid (5 ml) and hydrogenated under 1 atm in the presence of 10% palladium on carbon (360 mg) for about 3.5 hours. The catalyst was filtered off. The filtrate was poured into aqueous sodium hydrogen carbonate and extracted with chloroform. The organic layer was concentrated in vacuo and the residue was purified by column chromatography (chloroform : methanol = 97 : 3) to give the titled compound (256 mg) as a foam.
   ¹H-NMR (CDCl₃): δ 1.85 (4H, m), 2.67 (1H m}, 2.90 (2H, m), 3.80 (2H, s), 4.0-4.8 (2H, m), 5.10 (2H, s), 5.99 (2H, s), 6.30 (1H, s), 6.72 (2H, d, J=8.6), 6.81 (1H, d, J=8.3), 6.93 (2H, m), 7.02 (2H, d, J=8.6), 7.26 (1H, s).
   The free amine (256 mg) was dissolved in chloroform and treated with 1N hydrogen chloride-diethyl ether solution (0.6 ml). The solvent was removed in vacuo. The residue was dissolved in small amount of isopropanol and triturated with ether to give the hydrochloride (240 mg) of the titled compound.
   Melting point: 176-179 °C

### Example 316 Inhibitory activity of TNFα Production

BALB/c mice (female, 5-7 weeks old, Charles River Japan, Tokyo, Japan) were injected i.p. with 1 ml of 2.4% thioglycollate broth. After 4 days, the mice were sacrificed. Peritoneal exudated cells (PEC's) were collected from the peritoneal cavity by washing with minimum essential medium (MEM, Handai biseibutubyou kenkyukai, Osaka, Japan) containing 5 U/ml heparin, 1% fetal bovine serum (FBS, Filtron, Victoria, Australia) and antibiotics. The cells were washed two times with MEM, suspended with MEM containing 10% FBS (complete MEM, cMEM) and seeded into 96-well culture plates (Costar, Cambridge, MA, USA) at 2 x 10⁵ cells/well. The cells were incubated for 1 hour at 37°C in a humidified 5% CO₂ incubator and washed two times to remove non-adherent cells.

TNFα was induced by 10 µg/ml of LPS (E. coli O111B4, DIFCO, Detroit, MI, USA) the cells were cultured for 18 hours at 37°C. A compound of the present invention was dissolved in DMSO and added to the culture simultaneously with LPS. The final DMSO concentration was below 0.1%. The TNFα concentration in supernatant and recombinant mouse TNFa standard (rm-TNFα, Genzyme, MA, USA) were incubated overnight at 4°C in a 96-well plate coated with anti-mouse TNFα monoclonal antibody (PharMingen, San Diego, CA, USA) that previously had been blocked with 10% FBS. Each well of the plate first was incubated at room temperature with biotinylated anti-mouse TNFα polyclonal antibodies (PharMingen) for 45 minutes, then with peroxidase-conjugated streptavidin (Kirkegaard & Perry Laboratories Inc., Gaitherburg, MD, USA) for 30 minutes. After each incubation, the plate was washed 4 to 8 times with PBS containing 0.2% Tween 20. TMB (Kirkegaard & Perry Laboratories Inc.) was the peroxidase substrate used, the absorbance of each well was quantified at 450 nm by a microplatereader (Molecular Devices Corp., CA, USA). TNFα levels in the supernatant were quantified with rm-TNFα as the standard.

Inhibitory activity of TNFα production was also quantified using human peripheral blood mononuclear cells(PBMC) instead of PEC's.

### Example 317 Determination of the effect of a compound of the invention on HIV-1 LTR-driven CAT gene expression Materials and methods

### Plasmids:

p469, HIV-1 LTR driven CAT and pSV-Tat, Tat-expression vector were kindly provided from Dr. Joseph Sodroski, Dana-Farber Cancer Institute( C.A. Rosen, J.G. Sodroski and W.A. Haseltine, Cell Vol.41, 813-823, 1985, A Caputo, J.G. Sodroski and W.A. Haseltine, J. AIDS, Vol. 3, 327-379, 1990).

### Medium:

RPMI 1640 medium (Life Technologies, Grand Island, NY, USA) was supplemented with heat-inactivated fetal calf serum (FBS; Sigma, St. Louis, MO, USA).

### Cells:

Human T lymphoma cell line, Jurkat, was cultured in medium described above

### Transfection;

Introduction of plasmid DNA into cells was carried out according to the method described in Chiang, J. Li et. al., Proc. Natl. Acad. Sci. Vol. 92, 5461-5464 (1995). Briefly, the cells were suspended with the medium at 2 x 10⁷ cells/ml followed by addition 1 mg of p469 and 0.5 mg of DEAE-dextran (Sigma). In some experiments the cells were co-transfected with 0.5 mg of pSV-Tat. After 10 minutes incubation at room temperature with occasionally mixing,'the cells were pulsed with electroporation apparatus at 140 V. The cells were resuspended with the fresh medium and cultured for 20 hours at 37°C.

### Determination of the effect of a compound of the invention on HIV-1 LTR-driven CAT gene expression.

A compound tested was dissolved in dimethylsulfoxide at 100 mM as a stock solution. The aliquots of the stock solution were kept frozen at -20°C. Jurkat cells transfected with p469 were treated with 10 ng/ml of phorbol 12-myristate 13-acetate (PMA; Sigma) to activate HIV-1-LTR directed CAT gene transcription in the presence of the compound. In the case of cells transfected with p469 and pSV-Tat, they were simply treated with the compound. After the cells were cultured for an additional 24 hours at 37°C, the cells were washed 2 times with phosphate-buffered saline and lysed in 50 mM Tris-HCl plus 15% glycerol with repeated cycles of freeze and thaw. The cell lysates were clarified by centrifugation followed by determination of protein concentration by Bradford method (Bio-Rad, Hercules, CA, USA).
Measurement of a CAT activity in cell lysates was carried out according to a similar method described in Fridovich-Keil J. L. et. al., Cell Growth and Differentiation, Vol. 2, 67-76(1991). Equal amount (30 mg of protein) of cell lysates was incubated at 37°C with 35 mg of acetyl coenzyme A (GIBCO/BRL, Grand Island, MO, USA) and 0.1 mCi of ¹⁴C-chloramphenicol (Dupont, NEN Boston, MA, USA) in 42 ml solution for 2 hours. Acetyl-¹⁴C-chloramphenicol was extracted with 9 volumes of ethyl acetate and fractionated on a TLC gel with 1:19 (v/v) in methanol/chloroform. The CAT activity was detected by autoradiography and quantified by an Imaging Densitometer (model GS-700; Bio-Rad).

### Reference Example 1 Synthesis of ethyl 1-benzoylisonipecotate

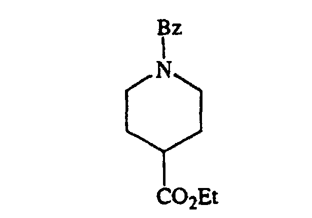

To an ice cold solution of ethyl isonipecotate (60 g, 0.382 mol) and triethylamine (38.65 g, 0.382 mol) in tetrahydrofuran (360 ml ) was added dropwise benzoyl chloride (53.7 g, 0.382 mol) over a period of about 2 hours under nitrogen atmosphere. The reaction mixture was stirred for about 2 hours at the same temperature, and was filtered. The filtrate was evaporated in vacuo. The residue was partitioned between dichloromethane and water. The organic layer was dried over magnesium sulfate, and evaporated in vacuo. Crystallization from isopropyl alcohol gave the titled compound (90.9 g).
Melting point: 71-72°C
¹H-NMR (CDCl₃): δ 1.27 (3H, t, J=7.3), 1.70 (4H, m), 2.57 (1H, m), 3.05 (2H, m), 3.75 (1H, m), 4.16 (2H, q, J=7.3), 4.54 (1H, m), 7.40 (5H, m)

### Reference Example 2 Synthesis of 1-benzoylisonipecotic acid

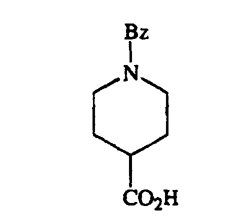

To an ice cold solution of isonipecotic acid (90 g, 0.697 mol) and potassium carbonate (81 g, 1.31 mol) in water (1.3 liter) was added dropwise benzoyl chloride (81 ml, 0.697 mol) over a period of about an hour at about 5°C. The mixture was stirred at room temperature overnight. The reaction mixture was acidified with 6 N hydrochloric acid and extracted with dichloromethane. The organic layer was washed with water and then dried over magnesium sulfate and the solvent was evaporated in vacuo. Crystallization from isopropyl alcohol-diethyl ether to give the titled compound (154.4 g).
Melting point: 143-144°C

### Reference Example 3 Synthesis of 4-cyanopiperidine

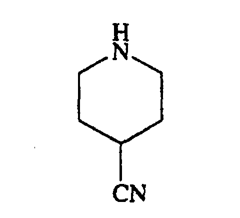

To vigorously stirred isonipecotamide (75 g, 0.585 mol) was added dropwise phosphorus oxychloride (182 ml) over a period of about 30 minutes. The reaction mixture was refluxed for about further 2 hours, the reaction mixture was evaporated in vacuo. The residue was poured onto ice (about 600 g) to decompose an excess of phosphorus oxychloride. The aqueous solution was neutralized with sodium hydrogencarbonate and then made basic with 2 N sodium hydroxide. The aqueous layer was extracted 4 times with chloroform (500 ml each). The organic layer was dried over sodium sulfate, and evaporated in vacuo. The residue was distilled in vacuo (b.p. 70°C, 3 mmHg) to give the titled compound (50.9 g) as an oil.

### Industrial Applicability

The piperidinylpyrimidine derivatives of the present invention exhibit an excellent TNFα inhibiting activity and are useful as TNFα inhibitors. The piperidinylpyrimidine derivatives of the present invention not only will normalize the abnormal TNFα production indicating immunopathy caused by viral infection but also will inhibit the proliferation of HIV. And they also inhibited HIV-LTR transcriptional activation in vitro. Therefore, the piperidinylpyrimidine derivatives of the present invention are expected to be effective for the treatment of AIDS.

The piperidinylpyrimidine derivatives of the present invention are also expected to be useful for the treatment of diseases wherein a TNFα level reportedly is increased in blood and/or tissue, e.g., osteoarthritis, rheumatoid arthritis, multiple sclerosis, Kawasaki disease, inflammatory bowel diseases such as ulcerative colitis or Crohn's disease, Beh et disease, systemic lupus erythematosus, graft versus host disease (GvHD), multiple organ failure, malaria, meningitis, hepatitis, non-insulin-dependent diabetes mellitus, asthma, adult respiratory distress syndrome (ARDS), tuberculosis, atherosclerosis, Alzheimer's disease, etc.

## Claims

1. Use of a compound which is a piperidinylpyrimidine derivative of formula (1) 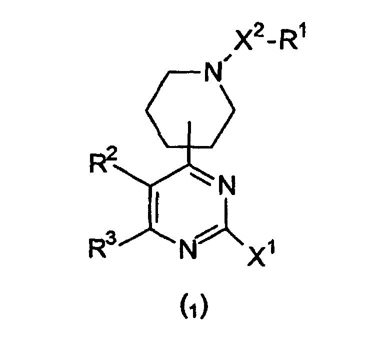 wherein X¹ is amino or hydroxyl;
X² is carbonyl (-(CO)-), carbonyloxy (-(CO)O-), carbonylamino (-(CO)NH-), or sulfonyl (-(SO₂)-);
R¹ is alkyl, cycloalkyl, aryl, substituted aryl, heteroaryl, or -(CH₂)ₘ- A, wherein m is 1, 2, 3, 4, 5, 6, 7, 8, 9 or 10, and A is a halogen atom, cycloalkyl, hydroxy, amino, cyano, nitro, carboxyl, alkoxy, alkylthio, alkoxycarbonyl, aryloxycarbonyl, alkyloxycarbonylamino, alkylamino, diakylamino, a saturated heterocyclic group, aryl, substituted aryl or heteroaryl,
R² is hydrogen, alkyl, or aryl, and
R³ is alkyl, aryl, substituted aryl, heteroaryl, or -(CH₂)ₘ-A', wherein m' is 1, 2, 3 or 4, and A' is a halogen atom, cycloalkyl, hydroxyl, amino, cyano, nitro, carboxyl, alkoxy, alkylthio, alkoxycarbonyl, aryloxycarbonyl, alkyloxycarbonylamino, alkylamino, dialkylamino, saturated heterocyclic, aryl, substituted aryl or heteroaryl,
or R² and R³ are combined with the pyrimidine ring to form quinazoline or a pyridopyrimidine, wherein said quinazoline may be substituted with a substituent or substituents selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl and said pyridopyrimidine may be substituted in the 5, 6, 7 and/or 8 position with a substituent selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl,
wherein said aryl group of R¹, A, R², R³ or A' has at least one hydrocarbon ring having up to 10 carbon atoms; said substituted aryl group of R¹, A, R³ or A' is an aryl group substituted with one or more substituents selected from halogen; alkyl having 1 to 10 carbon atoms; alkoxy having 1 to 6 carbon atoms; nitro; cyano; carboxyl; hydroxy; amino; trifluoromethyl; alkylamino having 1 to 4 carbon atoms; dialkylamino having 2 to 8 carbon atoms; alkanoyl having 1 to 6 carbon atoms; alkanoylamino having 1 to 6 carbon atoms; sulfamoyl; alkylaminosulfonyl
wherein the alkylamino thereof has 1 to 4 carbon atoms; dialkylaminosulfonyl wherein the dialkylamino thereof has 2 to 8 carbon atoms; alkoxycarbonyl wherein the alkoxy therefore has 1 to 6 carbon atoms; and substituents which are combined together to form methylenedioxy, ethylenedioxy or dihydroxyfuran; and
said heteroaryl group of R¹, A, R³ or A' comprises not more than 9 carbon atoms and 1 to 3 heteroatom(s) selected from nitrogen, oxygen and sulfur, which may be the same or different,
or a pharmaceutically acceptable salt thereof, in the manufacture of a medicament for inhibiting the production or secretion of tumour necrosis factor.

2. Use of a compound which is a piperidinylpyrimidine derivative of formula (1-e) 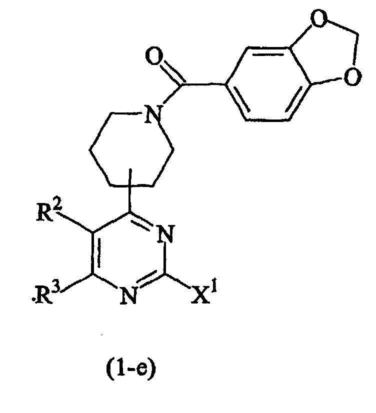 where X¹ is amino or hydroxy;
R² is hydrogen, alkyl, or aryl;
R³ is alkyl, aryl, substituted aryl, heteroaryl, or -(CH₂)ₘ-A', wherein m' is 1, 2, 3 or 4, and A' is a halogen atom, cycloalkyl, hydroxyl, amino, cyano, nitro, carboxyl, alkoxy, alkylthio, alkylthio, alkoxycarbonyl, aryloxycarbonyl, alkyloxycarbonylamino, alkylamino, dialkylamino, a saturated heterocyclic group, aryl, substituted aryl or heteroaryl,
or R² and R³ are combined with the pyrimidine ring to form quinazoline or a pyridopyrimidine, wherein said quinazoline may be substituted with a substituent or substituents selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl and said pyridopyrimidine may be substituted in the 5, 6, 7 and/or 8 position with a substituent selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl,
wherein said aryl group of R¹, A, R², R³ or A' has at least one hydrocarbon ring having up to 10 carbon atoms; said substituted aryl group of R¹, A, R³ or A' is an aryl group substituted with one or more substituents selected from halogen; alkyl having 1 to 10 carbon atoms; alkoxy having 1 to 6 carbon atoms; nitro; cyano; carboxyl; hydroxy; amino; trifluoromethyl; alkylamino having 1 to 4 carbon atoms; dialkylamino having 2 to 8 carbon atoms; alkanoyl having 1 to 6 carbon atoms; alkanoylamino having 1 to 6 carbon atoms; sulfamoyl; alkylaminosulfonyl
wherein the alkylamino thereof has 1 to 4 carbon atoms; dialkylaminosulfonyl wherein the dialkylamino thereof has 2 to 8 carbon atoms; alkoxycarbonyl wherein the alkoxy therefore has 1 to 6 carbon atoms; and substituents which are combined together to form methylenedioxy, ethylenedioxy or dihydroxyfuran; and
said heteroaryl group of R¹, A, R³ or A' comprises not more than 9 carbon atoms and 1 to 3 heteroatom(s) selected from nitrogen, oxygen and sulfur, which may be the same or different, or a pharmaceutically acceptable salt thereof, in the manufacture of a medicament for inhibiting human immunodeficiency virus-1 long terminal repeat transcriptional activation.

3. A compound which is a piperidinylpyrimidine derivative of formula (1-a), 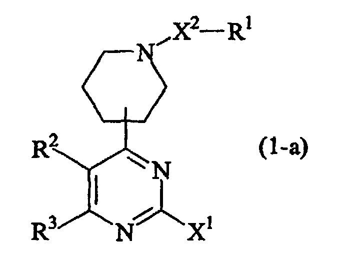 wherein
(1) X¹ is amino or hydroxyl;
X² is carbonyl (-(CO)-), carbonyloxy (-(CO)O-), carbonylamino (-(CO)NH-), or sulfonyl (-(SO₂)-);
R¹ is alkyl, cycloalkyl, aryl, substituted aryl, heteroaryl, or -(CH₂)ₘ- A, wherein m is 1, 2, 3, 4, 5, 6, 7, 8, 9, or 10, and A is a halogen atom, cycloalkyl, hydroxyl, amino, cyano, nitro, carboxyl, alkoxy, alkylthio, alkoxycarbonyl, aryloxycarbonyl, alkyloxycarbonylamino, alkylamino, dialkylamino, a saturated heterocyclic group, aryl, substituted aryl or heteroaryl,
R² is hydrogen, alkyl or aryl;
R³ is alkyl, aryl, substituted aryl, heteroaryl, or -(CH₂)_{m'}-A', wherein m' is 1, 2, 3 or 4 and A' is a halogen atom, cycloalkyl, hydroxyl, amino, cyano, nitro, carboxyl, alkoxy, alkylthio, alkoxycarbonyl, aryloxycarbonyl, alkyloxycarbonylamino, alkylamino, dialkylamino, a saturated heterocyclic group, aryl, substituted aryl or heteroaryl;
or
(2) X¹ is amino;
X² is carbonyl (-(CO)-)
R¹ is C₆-C₁₀ alkyl, cycloalkyl, aryl substituted by two or three halogen atoms, 2, 3-methylenedioxyphenyl, 3,4-methylenedioxyphenyl, C₇-C₁₅ aralkyl or -(CH₂)ₘ-A, wherein m is 6, 7, 8, 9 or 10, and A is a halogen atom, cycloalkyl, hydroxyl, amino, cyano, carboxyl, alkoxy, alkoxycarbonyl, aryloxycarbonyl, alkyloxycarbonylamino, alkylamino, dialkylamino, a saturated heterocyclic group, aryl or substituted aryl; and
R² and R³ are combined with the pyrimidine ring to form quinazoline, quinazoline substituted with a substituent or substituents selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl, pyridopyrimidine, or pyridopyrimidine substituted in the 5, 6, 7 and/or 8 position with a substituent selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl, wherein said aryl group of R¹, A, R², R³ or A' has at least one hydrocarbon ring having up to 10 carbon atoms; said substituted aryl group of R¹, A, R³ or A' is an aryl group substituted with one or more substituents selected from halogen; alkyl having 1 to 10 carbon atoms; alkoxy having 1 to 6 carbon atoms; nitro; cyano; carboxyl; hydroxy; amino; trifluoromethyl; alkylamino having 1 to 4 carbon atoms; dialkylamino having 2 to 8 carbon atoms; alkanoyl having 1 to 6 carbon atoms; alkanoylamino having 1 to 6 carbon atoms; sulfamoyl; alkylaminosulfonyl wherein the alkylamino thereof has 1 to 4 carbon atoms; dialkylaminosulfonyl
wherein the dialkylamino thereof has 2 to 8 carbon atoms; alkoxycarbonyl wherein the alkoxy therefore has 1 to 6 carbon atoms; and substituents which are combined together to form methylenedioxy, ethylenedioxy or dihydroxyfuran; and
said heteroaryl group of R¹, A, R³ or A' comprises not more than 9 carbon atoms and 1 to 3 heteroatom(s) selected from nitrogen, oxygen and sulfur, which may be the same or different,
or a pharmaceutically acceptable salt thereof.

4. A compound according to claim 3 wherein, in formula (1-a), X¹ is amino.

5. A compound according to claim 3 wherein, in formula (1-a) X¹ is amino or hydroxyl;
X² is carbonyl (-(CO)-), carbonyloxy (-(CO)O-), carbonylamino (-(CO)NH-), or sulfonyl (-(SO₂)-);
R¹ is alkyl, cycloalkyl, aryl, substituted aryl, heteroaryl, or -(CH₂)ₘ-A, wherein m is 1, 2, 3, 4, 5, 6, 7, 8, 9 or 10, and A is a halogen atom, cycloalkyl, hydroxyl, amino, cyano, nitro, carboxyl, alkoxy, alkylthio, alkoxycarbonyl, aryloxycarbonyl, alkoxycarbonylamino, alkylamino, dialkylamino, a saturated heterocyclic group, aryl, substituted aryl or heteroaryl;
R² is hydrogen, alkyl, or aryl;
R³ is alkyl, aryl, substituted aryl, heteroaryl, or -(CH₂)_{m'}-A', wherein m' is 1, 2, 3 or 4, and A' is a halogen atom, cycloalkyl, hydroxyl, amino, cyano, nitro, carboxyl, alkoxy, alkylthio, alkoxycarbonyl, aryloxycarbonyl, alkoxycarbonylamino, alkylamino, dialkylamino, a saturated heterocyclic group, aryl, substituted aryl or heteroaryl.

6. A compound according to claim 3 wherein, in formula (1-a), X¹ is amino;
X² is carbonyl (-(CO)-),carbonyloxy (-(CO)O-), or carbonylamino (-(CO)NH-);
R¹ is cycloalkyl, aryl, substituted aryl, heteroaryl or -(CH₂)ₘ-A, wherein m is 1, 2, 3, 4, 5, 6, 7, 8, 9 or 10, and A is a halogen atom, cycloalkyl, hydroxyl, amino, cyano, nitro, carboxyl, alkoxy, alkylthio, alkoxycarbonyl, aryloxycarbonyl, alkyloxycarbonylamino, alkylamino, dialkylamino, a saturated heterocyclic group, aryl, substituted aryl or heteroaryl;
R² is hydrogen, alkyl, or aryl;
R³ is aryl, substituted aryl, heteroaryl, or -(CH₂)_{m'}-A',
wherein m' is 1, 2, 3 or 4 and A' is a halogen atom, cycloalkyl, hydroxyl, amino, cyano, nitro, carboxyl, alkoxy, alkylthio, alkoxycarbonyl, aryloxycarbonyl, alkyloxycarbonylamino, alkylamino, dialkylamino, a saturated heterocyclic group, aryl, substituted aryl or heteroaryl.

7. A compound according to claim 6 wherein X² is carbonyl (-(CO)-) or carbonylamino (-(CO)NH-).

8. A compound which is a piperinylpyrimidine derivative of formula (1-e) 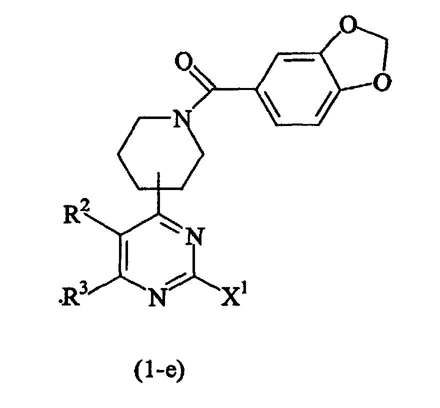 wherein X¹ is amino or hydroxy;
R² is hydrogen, alkyl, or aryl;
R³ is alkyl, aryl, substituted aryl, heteroaryl, or -(CH₂)_{m'}-A' wherein m' is 1, 2, 3 or 4, and A' is a halogen atom, cycloalkyl, hydroxyl, amino, cyano, nitro, carboxyl, alkoxy, alkylthio, alkoxycarbonyl, aryloxycarbonyl, alkoxycarbonylamino, alkylamino, dialkylamino, a saturated heterocyclic group, aryl, substituted aryl or heteroaryl;
or R² and R³ are combined with the pyrimidine ring to form a quinazoline or a pyridopyrimidine, wherein said quinazoline may be substituted with a substituent or substituents selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl and said pyridopyrimidine may be substituted in the 5, 6, 7 and/or 8 position with a substituent selected from a halogen atom; alkyl; alkoxy; nitro; cyano; carboxyl; hydroxy; amino; trifluoromethyl; alkylamino; dialkylamino; alkanoyl; alkanoylamino; sulfamoyl; alkylaminosulfonyl; dialkylaminosulfonyl; and alkoxycarbonyl,
wherein said aryl group of R², R³ or A' has at least one hydrocarbon ring having up to 10 carbon atoms; said substituted aryl group of R³ or A' is an aryl group substituted with one or more substituents selected from halogen; alkyl having 1 to 10 carbon atoms; alkoxy having 1 to 6 carbon atoms; nitro; cyano; carboxyl; hydroxy; amino; trifluoromethyl; alkylamino having 1 to 4 carbon atoms; dialkylamino having 2 to 8 carbon atoms; alkanoyl having 1 to 6 carbon atoms; alkanoylamino having 1 to 6 carbon atoms; sulfamoyl; alkylaminosulfonyl; wherein the alkylamino thereof has 1 to 4 carbon atoms; dialkylaminosulfonyl wherein the dialkylamino thereof has 2 to 8 carbon atoms; alkoxycarbonyl wherein the alkoxy therefore has 1 to 6 carbon atoms; and substituents which are combined together to form methylenedioxy, ethylenedioxy or dihydroxyfuran; and said heteroaryl group of R³ or A' comprises not more than 9 carbon atoms and 1 to 3 heteroatom(s) selected from nitrogen, oxygen and sulfur, which may be the same or different,
or a pharmaceutically acceptable salt thereof.

9. A compound according to claim 3 which is a piperidinylpyrimidine derivative of formula (1-b) 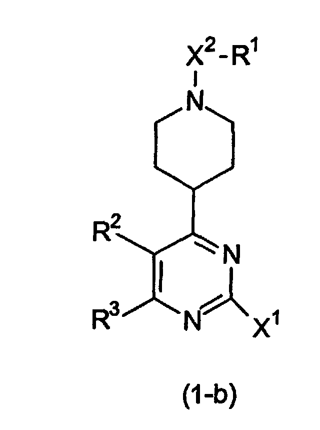 wherein X¹ is amino;
X² is carbonyl (-(CO)-), carbonyloxy (-(CO)O-), or carbonylamino (-(CO)NH-);
R¹ is cycloalkyl, aryl, substituted aryl, heteroaryl, or -(CH₂)ₘ-A, wherein m is 1, 2, 3, 4, 5, 6, 7 ,8, 9 or 10, and A is a halogen atom, cycloalkyl, hydroxyl, amino, cyano, nitro, carboxyl, alkoxy, alkylthio, alkoxycarbonyl, aryloxycarbonyl, alkyloxycarbonylamino, alkylamino, dialkylamino, a saturated heterocyclic group, aryl, substituted aryl or heteroaryl;
R² is hydrogen, or alkyl; and
R³ is aryl, substituted aryl, heteroaryl, or -(CH₂)ₘ,-A'
wherein m' is 1 or 2, and A' is aryl, substituted aryl or heteroaryl; or a pharmaceutically acceptable salt thereof.

10. A compound according to claim 3 which is a piperidinylpyrimidine derivative of formula (1-d), 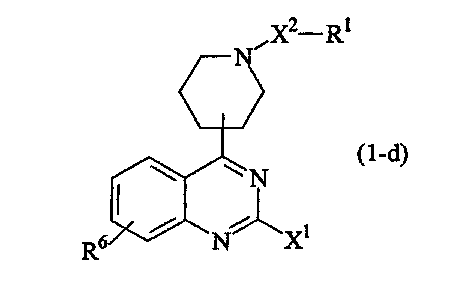 wherein X¹ is amino;
X² is carbonyl (-(CO)-);
R¹ is C₆-C₁₀ alkyl, cycloalkyl, aryl substituted by two or three halogen atoms, 2, 3-methylenedioxyphenyl, 3, 4-methylenedioxyphenyl, C₇-C₁₅ aralkyl or -(CH₂)ₘ-A, wherein m is 6, 7, 8, 9 or 10, and A is a halogen atom, cycloalkyl, hydroxyl, amino, cyano, carboxyl, alkoxy, alkoxycarbonyl, aryloxycarbonyl, alkloxycarbonylamino, alkylamino, dialkylamino, a saturated heterocyclic group, aryl or substituted aryl;
R⁶ is a halogen atom, alkyl, alkoxy, nitro, cyano, carboxyl, hydroxyl, amino, trifluoromethyl, alkylamino, dialkylamino, alkanoyl, alkanoylamino, sulfamoyl, alkylaminosulfonyl, dialkylaminosulfonyl or alkoxycarbonyl;
or a pharmaceutically acceptable salt thereof.

## Patentansprüche

1. Verwendung einer Verbindung, die ein Piperidinylpyrimidinderivat der Formel (1) ist 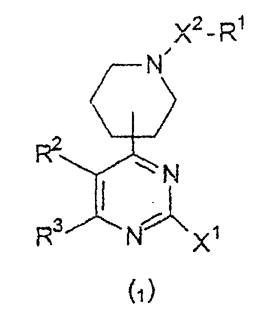 wobei X¹ Amino oder Hydroxy ist,
X² Carbonyl (-(CO)-), Carbonyloxy (-(CO)O-), Carbonylamino (-(CO)NH-) oder Sulfonyl (-(SO₂)-) ist;
R¹ Alkyl, Cycloalkyl, Aryl, substituiertes Aryl, Heteroaryl oder -(CH₂)ₘ-A ist, wobei m 1, 2, 3, 4, 5, 6, 7, 8, 9 oder 10 ist, und A ein Halogenatom, Cycloalkyl, Hydroxy, Amino, Cyano, Nitro, Carboxy, Alkoxy, Alkylthio, Alkoxycarbonyl, Aryloxycarbonyl, Alkyloxycarbonylamino, Alkylamino, Dialkylamino, eine gesättigte heterocyclische Gruppe, Aryl, substituiertes Aryl oder Heteroaryl ist,
R² Wasserstoff, Alkyl oder Aryl ist, und
R³ Alkyl, Aryl, substituiertes Aryl, Heteroaryl oder -(CH₂)_{m'}-A'ist, wobei m' 1, 2, 3 oder 4 ist und A' ein Halogenatom, Cycloalkyl, Hydroxy, Amino, Cyano, Nitro, Carboxy, Alkoxy, Alkylthio, Alkoxycarbonyl, Aryloxycarbonyl, Alkyloxycarbonylamino, Alkylamino, Dialkylamino, eine gesättigte heterocyclische Gruppe, Aryl, substituiertes Aryl oder Heteroaryl ist,
oder R² und R³ mit dem Pyrimidinring verbunden sind, um Chinazolin oder ein Pyridopyrimidin zu bilden, wobei das Chinazolin mit einem oder mehreren Substituenten substituiert sein kann, ausgewählt aus einem Halogenatom; Alkyl; Alkoxy; Nitro; Cyano; Carboxy; Hydroxy; Amino; Trifluormethyl; Alkylamino; Dialkylamino; Alkanoyl; Alkanoylamino; Sulfamoyl; Alkylaminosulfonyl; Dialkylaminosulfonyl; und Alkoxycarbonyl; und das Pyridopyrimidin an Position 5, 6, 7 und/oder 8 durch einen Substituenten substituiert werden kann, ausgewählt aus einem Halogenatom; Alkyl; Alkoxy; Nitro; Cyano; Carboxy; Hydroxy; Amino; Trifluormethyl; Alkylamino; Dialkylamino; Alkanoyl; Alkanoylamino; Sulfamoyl; Alkylaminosulfonyl; Dialkylaminosulfonyl und Alkoxycarbonyl,
wobei die Arylgruppe von R¹, A, R², R³ oder A' mindestens einen Kohlenwasserstoffring mit bis zu 10 Kohlenstoffatomen besitzt; die substituierte Arylgruppe von R¹, A, R³ oder A' eine Arylgruppe ist, substituiert durch einen oder mehrere Substituenten, ausgewählt aus Halogen; Alkyl mit 1 bis 10 Kohlenstoffatomen; Alkoxy mit 1 bis 6 Kohlenstoffatomen; Nitro; Cyano; Carboxy; Hydroxy; Amino; Trifluormethyl; Alkylamino mit 1 bis 4 Kohlenstoffatomen; Dialkylamino mit 2 bis 8 Kohlenstoffatomen; Alkanoyl mit 1 bis 6 Kohlenstoffatomen; Alkanoylamino mit 1 bis 6 Kohlenstoffatomen; Sulfamoyl; Alkylaminosulfonyl, wobei das Alkylamino davon 1 bis 4 Kohlenstoffatome hat; Dialkylaminosulfonyl, wobei das Dialkylamino davon 2 bis 8 Kohlenstoffatome hat; Alkoxycarbonyl, wobei das Alkoxy davon 1 bis 6 Kohlenstoffatome hat; und Substituenten, die miteinander verbunden sind, um Methylendioxy, Ethylendioxy oder Dihydroxyfuran zu bilden, und
die Heteroarylgruppe von R¹, A, R³ oder A' nicht mehr als 9 Kohlenstoffatome und 1 bis 3 Heteroatom(e) ausgewählt aus Stickstoff, Sauerstoff und Schwefel, umfasst, die gleich oder verschieden sein können,
oder eines pharmazeutisch verträglichen Salzes davon, bei der Herstellung eines Medikaments zur Hemmung der Produktion oder Sekretion des Tumomekrosefaktors.

2. Verwendung einer Verbindung, die ein Piperidinylpyrimidinderivat der Formel (1-e) ist 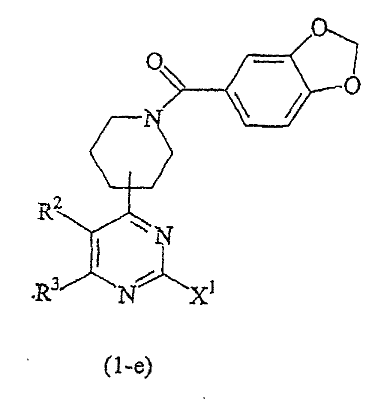 wobei X¹ Amino oder Hydroxy ist,
R² Wasserstoff, Alkyl oder Aryl ist,
R³ Alkyl, Aryl, substituiertes Aryl, Heteroaryl oder -(CH₂)_{m'}-A' ist, wobei m' 1, 2, 3 oder 4 ist und A' ein Halogenatom, Cycloalkyl, Hydroxy, Amino, Cyano, Nitro, Carboxy, Alkoxy, Alkylthio, Alkoxycarbonyl, Aryloxycarbonyl, Alkyloxycarbonylamino, Alkylamino, Dialkylamino, eine gesättigte heterocyclische Gruppe, Aryl, substituiertes Aryl oder Heteroaryl ist,
oder R² und R³ mit dem Pyrimidinring verbunden sind, um Chinazolin oder ein Pyridopyrimidin zu bilden, wobei das Chinazolin mit einem oder mehreren Substituenten substituiert sein kann, ausgewählt aus einem Halogenatom; Alkyl; Alkoxy; Nitro; Cyano; Carboxy; Hydroxy; Amino; Trifluormethyl; Alkylamino; Dialkylamino; Alkanoyl; Alkanoylamino; Sulfamoyl; Alkylaminosulfonyl; Dialkylaminosulfonyl; und Alkoxycarbonyl; und das Pyridopyrimidin an Position 5, 6, 7 und/oder 8 durch einen Substituenten substituiert sein kann, ausgewählt aus einem Halogenatom; Alkyl; Alkoxy; Nitro; Cyano; Carboxy; Hydroxy; Amino; Trifluormethyl; Alkylamino; Dialkylamino; Alkanoyl; Alkanoylamino; Sulfamoyl; Alkylaminosulfonyl; Dialkylaminosulfonyl und Alkoxycarbonyl,
wobei die Arylgruppe von R¹, A, R², R³ oder A' mindestens einen Kohlenwasserstoffring mit bis zu 10 Kohlenstoffatomen besitzt; die substituierte Arylgruppe von R¹, A, R³ oder A' eine Arylgruppe ist, substituiert durch einen oder mehrere Substituenten, ausgewählt aus Halogen; Alkyl mit 1 bis 10 Kohlenstoffatomen; Alkoxy mit 1 bis 6 Kohlenstoffatomen; Nitro; Cyano; Carboxy; Hydroxy, Amino; Trifluormethyl; Alkylamino mit 1 bis 4 Kohlenstoffatomen; Dialkylamino mit 2 bis 8 Kohlenstoffatomen; Alkanoyl mit 1 bis 6 Kohlenstoffatomen; Alkanoylamino mit 1 bis 6 Kohlenstoffatomen; Sulfamoyl; Alkylaminosulfonyl, wobei das Alkylamin davon 1 bis 4 Kohlenstoffatome hat; Dialkylaminosulfonyl, wobei das Dialkylamin davon 2 bis 8 Kohlenstoffatome hat; Alkoxycarbonyl, wobei das Alkoxy davon 1 bis 6 Kohlenstoffatome hat und Substituenten, die miteinander verbunden sind, um Methylendioxy, Ethylendioxy oder Dihydroxyfuran zu bilden; und
die Heteroarylgruppe von R¹, A, R³ oder A' nicht mehr als 9 Kohlenstoffatome und 1 bis 3 Heteroatom(e), ausgewählt aus Stickstoff, Sauerstoff und Schwefel umfasst, die gleich oder verschieden sein können,
oder eines pharmazeutisch verträglichen Salzes davon, bei der Herstellung eines Medikaments zur Hemmung der wiederholten Transkriptionsaktivierung langer endständiger Ketten des HI-Virus 1

3. Verbindung, die ein Piperidinylpyrimidinderivat der Formel (1-a) ist, 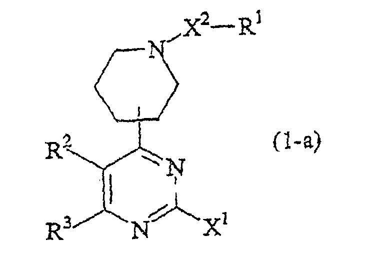 wobei
(1) X¹ Amino oder Hydroxy ist,
X² Carbonyl (-(CO)-), Carbonyloxy (-(CO)O-), Carbonylamino (-(CO)NH-) oder Sulfonyl (-(SO₂)-) ist;
R¹ Alkyl, Cycloalkyl, Aryl, substituiertes Aryl, Heteroaryl oder -(CH₂)ₘ-A ist, wobei m 1, 2, 3, 4, 5, 6, 7, 8, 9 oder 10 ist, und A ein Halogenatom, Cycloalkyl, Hydroxy, Amino, Cyano, Nitro, Carboxy, Alkoxy, Alkylthio, Alkoxycarbonyl, Aryloxycarbonyl, Alkyloxycarbonylamino, Alkylamino, Dialkylamino, eine gesättigte heterocyclische Gruppe, Aryl, substituiertes Aryl oder Heteroaryl ist,
R² Wasserstoff, Alkyl oder Aryl ist,
R³ Alkyl, Aryl, substituiertes Aryl, Heteroaryl oder -(CH₂)_{m'}-A' ist, wobei m' 1, 2, 3 oder 4 ist und A' ein Halogenatom, Cycloalkyl, Hydroxy, Amino, Cyano, Nitro, Carboxy, Alkoxy, Alkylthio, Alkoxycarbonyl, Aryloxycarbonyl, Alkyloxycarbonylamino, Alkylamino, Dialkylamino, eine gesättigte heterocyclische Gruppe, Aryl, substituiertes Aryl oder Heteroaryl ist;
oder
(2) X¹ Amino ist;
X² Carbonyl (-(CO)-) ist;
R¹ C₆-C₁₀ Alkyl, Cycloalkyl, Aryl substituiert durch zwei oder drei Halogenatome, 2, 3-Methylendioxyphenyl, 3,4-Methylendioxyphenyl, C₇-C₁₅-Aralkyl oder -(CH₂)ₘ-A, wobei m 6, 7, 8, 9 oder 10 ist, und A ein Halogenatom, Cycloalkyl, Hydroxy, Amino, Cyano, Carboxy, Alkoxy, Alkoxycarbonyl, Aryloxycarbonyl, Alkyloxycarbonylamino, Alkylamino, Dialkylamino, eine gesättigte heterocyclische Gruppe, Aryl oder substituiertes Aryl ist; und
R² und R³ mit dem Pyrimidinring verbunden sind, um Chinazolin, Chinazolin substituiert mit einem oder mehreren Substituenten, ausgewählt aus einem Halogenatom; Alkyl; Alkoxy; Nitro; Cyano; Carboxy; Hydroxy; Amino; Trifluormethyl; Alkylamino; Dialkylamino; Alkanoyl; Alkanoylamino; Sulfamoyl; Alkylaminosulfonyl; Dialkylaminosulfonyl; und Alkoxycarbonyl, Pyridopyrimidin oder Pyridopyrimidin, substituiert an Position 5, 6, 7 und/oder 8 durch einen Substituenten, ausgewählt aus einem Halogenatom; Alkyl; Alkoxy; Nitro; Cyano; Carboxy; Hydroxy, Amino; Trifluormethyl; Alkylamino; Dialkylamino; Alkanoyl; Alkanoylamino; Sulfamoyl; Alkylaminosulfonyl; Dialkylaminosulfonyl; und Alkoxycarbonyl zu bilden,
wobei die Arylgruppe von R¹, A, R², R³ oder A' mindestens einen Kohlenwasserstoffring mit bis zu 10 Kohlenstoffatomen besitzt; die substituierte Arylgruppe von R¹, A, R³ oder A' eine Arylgruppe ist, substituiert durch einen oder mehrere Substituenten, ausgewählt aus Halogen; Alkyl mit 1 bis 10 Kohlenstoffatomen; Alkoxy mit 1 bis 6 Kohlenstoffatomen; Nitro; Cyano; Carboxy; Hydroxy; Amino; Trifluormethyl; Alkylamino mit 1 bis 4 Kohlenstoffatomen; Dialkylamino mit 2 bis 8 Kohlenstoffatomen; Alkanoyl mit 1 bis 6 Kohlenstoffatomen; Alkanoylamino mit 1 bis 6 Kohlenstoffatomen; Sulfamoyl; Alkylaminosulfonyl wobei das Alkylamino davon 1 bis 4 Kohlenstoffatome hat; Dialkylaminosulfonyl, wobei das Dialkylamino davon 2 bis 8 Kohlenstoffatome hat; und Alkoxycarbonyl, wobei das Alkoxy davon 1 bis 6 Kohlenstoffatome hat und Substituenten, die miteinander verbunden sind, um Methylendioxy, Ethylendioxy oder Dihydroxyfuran zu bilden; und
die Heteroarylgruppe von R¹, A, R³ oder A' nicht mehr als 9 Kohlenstoffatome und 1 bis 3 Heteroatom(e), ausgewählt aus Stickstoff, Sauerstoff und Schwefel, umfasst, die gleich oder verschieden sein können,
oder ein pharmazeutisch verträgliches Salz davon.

4. Verbindung gemäß Anspruch 3, wobei, in Formel (1-a) X¹ Amino ist.

5. Verbindung gemäß Anspruch 3, wobei, in der Formel (1-a) X¹ Amino oder Hydroxy ist;
X² Carbonyl (-(CO)-), Carbonyloxy (-(CO)O-), Carbonylamino (-(CO)NH-) oder Sulfonyl (-(SO₂)-) ist;
R¹ Alkyl, Cycloalkyl, Aryl, substituiertes Aryl, Heteroaryl oder -(CH₂)ₘ-A ist, wobei m 1, 2, 3, 4, 5, 6, 7, 8, 9 oder 10 ist, und A ein Halogenatom, Cycloalkyl, Hydroxy, Amino, Cyano, Nitro, Carboxy, Alkoxy, Alkylthio, Alkoxycarbonyl, Aryloxycarbonyl, Alkoxycarbonylamino, Alkylamino, Dialkylamino, eine gesättigte heterocyclische Gruppe, Aryl, substituiertes Aryl oder Heteroaryl ist,
R² Wasserstoff, Alkyl oder Aryl ist,
R³ Alkyl, Aryl, substituiertes Aryl, Heteroaryl oder -(CH₂)_{m'}-A' ist, wobei m' 1, 2, 3 oder 4 ist und A' ein Halogenatom, Cycloalkyl, Hydroxy, Amino, Cyano, Nitro, Carboxy, Alkoxy, Alkylthio, Alkoxycarbonyl, Aryloxycarbonyl, Alkoxycarbonylamino, Alkylamino, Dialkylamino, eine gesättigte heterocyclische Gruppe, Aryl, substituiertes Aryl oder Heteroaryl ist.

6. Verbindung gemäß Anspruch 3, wobei, in der Formel (1-a) X¹ Amino ist;
X² Carbonyl (-(CO)-), Carbonyloxy (-(CO) O-) oder Carbonylamino (-(CO)NH-) ist;
R¹ Cycloalkyl, Aryl, substituiertes Aryl, Heteroaryl oder -(CH₂)ₘ-A ist, wobei m 1, 2, 3, 4, 5, 6, 7, 8, 9 oder 10 ist, und A ein Halogenatom, Cycloalkyl, Hydroxy, Amino, Cyano, Nitro, Carboxy, Alkoxy, Alkylthio, Alkoxycarbonyl, Aryloxycarbonyl, Alkyloxycarbonylamino, Alkylamino, Dialkylamino, eine gesättigte heterocyclische Gruppe, Aryl, substituiertes Aryl oder Heteroaryl ist,
R² Wasserstoff, Alkyl oder Aryl ist,
R³ Aryl, substituiertes Aryl, Heteroaryl oder -(CH₂)_{m'}-A' ist, wobei m' 1, 2, 3 oder 4 ist und A' ein Halogenatom, Cycloalkyl, Hydroxy, Amino, Cyano, Nitro, Carboxy; Alkoxy, Alkylthio, Alkoxycarbonyl, Aryloxycarbonyl, Alkyloxycarbonylamino, Alkylamino, Dialkylamino, eine gesättigte heterocyclische Gruppe, Aryl, substituiertes Aryl oder Heteroaryl ist.

7. Verbindung gemäß Anspruch 6, wobei X² Carbonyl (-(CO)-) oder Carbonylamino (-(CO)NH-) ist.

8. Verbindung, die ein Piperidinylpyrimidinderivat der Formel (1-e) ist 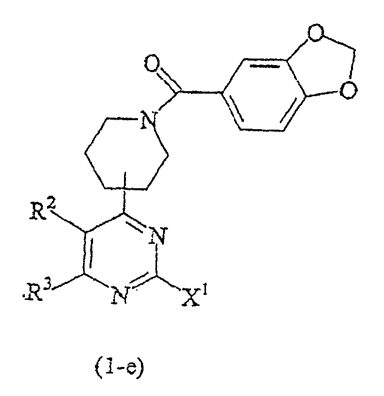 wobei X¹ Amino oder Hydroxy ist,
R² Wasserstoff, Alkyl oder Aryl ist,
R³ Alkyl, Aryl, substituiertes Aryl, Heteroaryl oder -(CH₂)_{m'}-A' ist, wobei m' 1, 2, 3 oder 4 ist und A' ein Halogenatom, Cycloalkyl, Hydroxy, Amino, Cyano, Nitro, Carboxy, Alkoxy, Alkylthio, Alkoxycarbonyl, Aryloxycarbonyl, Alkoxycarbonylamino, Alkylamino, Dialkylamino, eine gesättigte heterocyclische Gruppe, Aryl, substituiertes Aryl oder Heteroaryl ist,
oder R² und R³ mit dem Pyrimidinring verbunden sind, um ein Chinazolin oder ein Pyridopyrimidin zu bilden, wobei das Chinazolin mit einem oder mehreren Substituenten substituiert sein kann, ausgewählt aus einem Halogenatom; Alkyl; Alkoxy; Nitro; Cyano; Carboxy; Hydroxy; Amino; Trifluormethyl; Alkylamino; Dialkylamino; Alkanoyl; Alkanoylamino; Sulfamoyl; Alkylaminosulfonyl; Dialkylaminosulfonyl; und Alkoxycarbonyl, und das Pyridopyrimidin an Position 5, 6, 7 und/oder 8 durch einen Substituenten substituiert sein kann, der aus einem Halogenatom; Alkyl; Alkoxy; Nitro; Cyano; Carboxy; Hydroxy; Amino; Trifluormethyl; Alkylamino; Dialkylamino; Alkanoyl; Alkanoylamino; Sulfamoyl; Alkylaminosulfonyl; Dialkylaminosulfonyl; und Alkoxycarbonyl ausgewählt ist,
wobei die Arylgruppe von R², R³ oder A' mindestens einen Kohlenwasserstoffring mit bis zu 10 Kohlenstoffatomen besitzt; die substituierte Arylgruppe von R³ oder A' eine Arylgruppe ist, substituiert durch einen oder mehrere Substituenten, ausgewählt aus Halogen; Alkyl mit 1 bis 10 Kohlenstoffatomen; Alkoxy mit 1 bis 6 Kohlenstoffatomen; Nitro; Cyano; Carboxy; Hydroxy; Amino; Trifluormethyl; Alkylamino mit 1 bis 4 Kohlenstoffatomen; Dialkylamino mit 2 bis 8 Kohlenstoffatomen; Alkanoyl mit 1 bis 6 Kohlenstoffatomen; Alkanoylamino mit 1 bis 6 Kohlenstoffatomen; Sulfamoyl; Alkylaminosulfonyl; wobei das Alkylamino davon 1 bis 4 Kohlenstoffatome hat; Dialkylaminosulfonyl, wobei das Dialkylamino davon 2 bis 8 Kohlenstoffatome hat; und Alkoxycarbonyl, wobei das Alkoxy davon 1 bis 6 Kohlenstoffatome hat; und Substituenten, die miteinander verbunden sind, um Methylendioxy, Ethylendioxy oder Dihydroxyfuran zu bilden, und
die Heteroarylgruppe von R³ oder A' nicht mehr als 9 Kohlenstoffatome und 1 bis 3 Heteroatom(e), ausgewählt Stickstoff, Sauerstoff und Schwefel, umfasst, die gleich oder verschieden sein können, oder ein pharmazeutisch verträgliches Salz davon.

9. Verbindung gemäß Anspruch 3, die ein Piperidinylpyrimidinderivat der Formel (1-b) ist 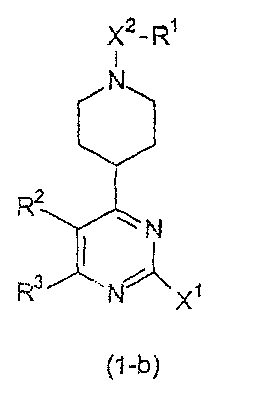 wobei X¹ Amino ist;
X² Carbonyl (-(CO)-), Carbonyloxy (-(CO)O-) oder Carbonylamino (-(CO)NH-) ist;
R¹ Cycloalkyl, Aryl, substituiertes Aryl, Heteroaryl oder -(CH₂)ₘ-A ist, wobei m 1, 2, 3, 4, 5, 6, 7, 8, 9 oder 10 ist, und A ein Halogenatom, Cycloalkyl, Hydroxy, Amino, Cyano, Nitro, Carboxy, Alkoxy, Alkylthio, Alkoxycarbonyl, Aryloxycarbonyl, Alkyloxycarbonylamino, Alkylamino, Dialkylamino, eine gesättigte heterocyclische Gruppe, Aryl, substituiertes Aryl oder Heteroaryl ist,
R² Wasserstoff oder Alkyl ist; und
R³ Alkyl, Aryl, substituiertes Aryl, Heteroaryl oder -(CH₂)_{m'}-A' ist, wobei m' 1, 2 und A' Aryl, substituiertes Aryl oder Heteroaryl ist; oder ein pharmazeutisch verträgliches Salz davon.

10. Verbindung gemäß Anspruch 3, die ein Piperidinylpyrimidinderivat der Formel (1-d) ist 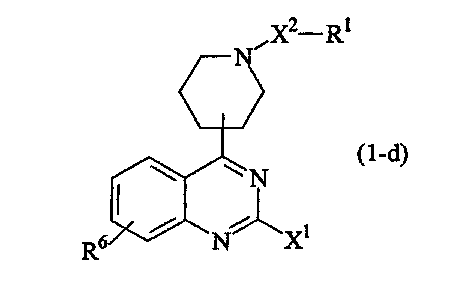 wobei X¹ Amino ist;
X² Carbonyl (-(CO)-) ist;
R¹ C₆-C₁₀⁻Alkyl, Cycloalkyl, Aryl substituiert durch zwei oder drei Halogenatome, 2,3-Methylendioxyphenyl, 3,4-Methylendioxyphenyl, C₇-C₁₅-Aralkyl oder -(CH₂)ₘ-A, wobei m 6, 7, 8, 9 oder 10 ist, und A ein Halogenatom, Cycloalkyl, Hydroxy, Amino, Cyano, Carboxy, Alkoxy, Alkoxycarbonyl, Aryloxycarbonyl, Alkyloxycarbonylamino, Alkylamino, Dialkylamino, eine gesättigte heterocyclische Gruppe, Aryl oder substituiertes Aryl ist;
R⁶ ein Halogenatom, Alkyl, Alkoxy, Nitro, Cyano, Carboxy, Hydroxy, Amino, Trifluormethyl, Alkylamino, Dialkylamino, Alkanoyl, Alkanoylamino, Sulfamoyl, Alkylaminosulfonyl, Dialkylaminosulfonyl oder Alkoxycarbonyl ist, oder ein pharmazeutisch verträgliches Salz davon.

## Revendications

1. Utilisation d'un composé qui est un dérivé de pipéridinylpyrimidine de formule (1) 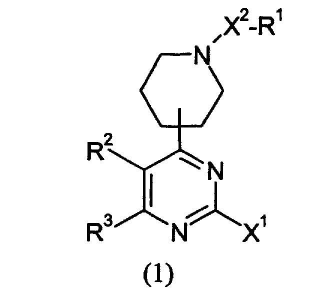 dans laquelle X¹ est un groupe amino ou hydroxyle ;
X² est un groupe carbonyle, (-(CO)-), carbonyloxy (-(CO)O-), carbonylamino (-(CO)NH-), ou sulfonyle (-(SO₂)-) ;
R¹ est un groupe alkyle, cycloalkyle, aryle, aryle substitué, hétéroaryle ou -(CH₂)ₘ-A , où m vaut 1, 2, 3, 4, 5, 6, 7, 8, 9 ou 10 et A est un atome d'halogène, un groupe cycloalkyle, hydroxy, amino, cyano, nitro, carboxyle, alcoxy, alkylthio, alcoxycarbonyle, aryloxycarbonyle, alkyloxycarbonylamino, alkylamino, dialkylamino, un groupe hétérocyclique saturé, aryle, aryle substitué ou hétéroaryle,
R² est un atome d'hydrogène ou un groupe alkyle ou aryle, et
R³ est un groupe alkyle, aryle, aryle substitué, hétéroaryle ou -(CH₂)ₘ-A', où m' vaut 1, 2, 3 ou 4, et A' est un atome d'halogène, un groupe cycloalkyle, hydroxyle, amino, cyano, nitro, carboxyle, alcoxy, alkylthio, alcoxycarbonyle, aryloxycarbonyle, alkyloxycarbonylamino, alkylamino, dialkylamino, hétérocyclique saturé, aryle, aryle substitué ou hétéroaryle,
ou R² et R³ sont combinés au noyau pyrimidine pour former une quinazoline ou une pyridopyrimidine, où ladite quinazoline peut être substituée par un plusieurs substituants choisis parmi les atomes d'halogène et les groupes alkyle, alcoxy, nitro, cyano, carboxyle, hydroxy, amino, trifluorométhyle, alkylamino, dialkylamino, alcanoyle, alcanoylamino, sulfamoyle, alkylaminosulfonyle, dialkylaminosulfonyle et alcoxycarbonyle, et ladite pyridopyrimidine peut être substituée sur la position 5, 6, 7 et/ou 8 par un substituant choisi parmi les atomes d'halogène et les groupes alkyle, alcoxy, nitro, cyano, carboxyle, hydroxy, amino, trifluorométhyle, alkylamino, dialkylamino, alcanoyle, alcanoylamino, sulfamoyle, alkylaminosulfonyle, dialkylaminosulfonyle et alcoxycarbonyle,
où ledit groupe aryle de R¹, A, R², R³ ou A' possède au moins un noyau hydrocarboné ayant jusqu'à 10 atomes de carbone ; ledit groupe aryle substitué de R¹, A, R³ ou A' est un groupe aryle substitué par un ou plusieurs substituants choisis parmi les halogènes, les groupes alkyle ayant de 1 à 10 atomes de carbone, alcoxy ayant de 1 à 6 atomes de carbone, nitro, cyano, carboxyle, hydroxy, amino, trifluorométhyle, alkylamino ayant de 1 à 4 atomes de carbone, dialkylamino ayant de 2 à 8 atomes de carbone, alcanoyle ayant de 1 à 6 atomes de carbone, alcanoylamino ayant de 1 à 6 atomes de carbone, sulfamoyle, alkylaminosulfonyle où le fragment alkylamino a de 1 à 4 atomes de carbone, dialkylaminosulfonyle où le fragment dialkylamino a de 2 à 8 atomes de carbone, alcoxycarbonyle où le fragment alcoxy a de 1 à 6 atomes de carbone, et les substituants qui sont combinés les uns aux autres pour former un groupe méthylènedioxy, éthylènedioxy ou dihydroxyfuranne ; et
ledit groupe hétéroaryle de R¹, A, R³ ou A' n'a pas plus de 9 atomes de carbone et a de 1 à 3 hétéroatomes choisis parmi l'azote, l'oxygène et le soufre, qui peuvent être différents,
ou d'un de ses sels acceptables d'un point de vue pharmaceutique, pour fabriquer un médicament destiné à inhiber la production ou la sécrétion du facteur de nécrose tumorale.

2. Utilisation d'un composé qui est un dérivé de pipéridinylpyrimidine de formule (1-e) 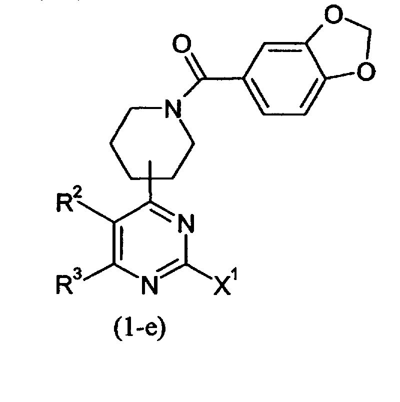 dans laquelle X¹ est un groupe amino ou hydroxy ;
R² est un atome d'hydrogène ou un groupe alkyle ou aryle ;
R³ est un groupe alkyle, aryle, aryle substitué, hétéroaryle ou (CH₂)ₘ-A', où m' vaut 1,2, 3 ou 4 et A' est un atome d'halogène ou un groupe cycloalkyle, hydroxyle, amino, cyano, nitro, carboxyle, alcoxy, alkylthio, alkylthio, alcoxycarbonyle, aryloxycarbonyle, alkyloxycarbonylamino, alkylamino, dialkylamino, un groupe hétérocyclique saturé, aryle, aryle substitué ou hétéroaryle,
ou R² et R³ sont combinés au noyau pyrimidine pour former une quinazoline ou une pyridopyrimidine, où ladite quinazoline peut être substituée par un plusieurs substituants choisis parmi les atomes d'halogène et les groupes alkyle, alcoxy, nitro, cyano, carboxyle, hydroxy, amino, trifluorométhyle, alkylamino, dialkylamino, alcanoyle, alcanoylamino, sulfamoyle, alkylaminosulfonyle, dialkylaminosulfonyle et alcoxycarbonyle, et ladite pyridopyrimidine peut être substituée sur la position 5, 6, 7 et/ou 8 par un substituant choisi parmi les atomes d'halogène et les groupes alkyle, alcoxy, nitro, cyano, carboxyle, hydroxy, amino, trifluorométhyle, alkylamino, dialkylamino, alcanoyle, alcanoylamino, sulfamoyle, alkylaminosulfonyle, dialkylaminosulfonyle et alcoxycarbonyle,
où ledit groupe aryle de R¹, A, R², R³ ou A' possède au moins un noyau hydrocarboné ayant jusqu'à 10 atomes de carbone ; ledit groupe aryle substitué de R¹, A, R³ ou A' est un groupe aryle substitué par un ou plusieurs substituants choisis parmi les halogènes, les groupes alkyle ayant de 1 à 10 atomes de carbone, alcoxy ayant de 1 à 6 atomes de carbone, nitro, cyano, carboxyle, hydroxy, amino, trifluorométhyle, alkylamino ayant de 1 à 4 atomes de carbone, dialkylamino ayant de 2 à 8 atomes de carbone, alcanoyle ayant de 1 à 6 atomes de carbone, alcanoylamino ayant de 1 à 6 atomes de carbone, sulfamoyle, alkylaminosulfonyle où le fragment alkylamino a de 1 à 4 atomes de carbone, dialkylaminosulfonyle où le fragment dialkylamino a de 2 à 8 atomes de carbone, alcoxycarbonyle où le fragment alcoxy a de 1 à 6 atomes de carbone, et les substituants qui sont combinés les uns aux autres pour former un groupe méthylènedioxy, éthylènedioxy ou dihydroxyfuranne ; et
ledit groupe hétéroaryle de R¹, A, R³ ou A' n'a pas plus de 9 atomes de carbone et a de 1 à 3 hétéroatomes choisis parmi l'azote, l'oxygène et le soufre, qui peuvent être différents, ou d'un de ses sels acceptables d'un point de vue pharmaceutique, pour fabriquer un médicament destiné à inhiber une activation transcriptionnelle à répétition terminale longue provoquée par le virus-1 de l'immunodéficience humaine.

3. Composé qui est un dérivé de pipéridinylpyrimidine de formule (1-a) 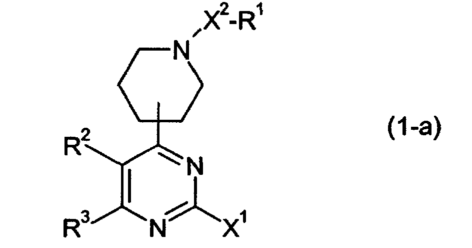 dans laquelle
(1) X¹ est un groupe amino ou hydroxyle ;
X² est un groupe carbonyle, (-(CO)-), carbonyloxy (-(CO)O-), carbonylamino (-(CO)NH-), ou sulfonyle (-(SO₂)-) ;
R¹ est un groupe alkyle, cycloalkyle, aryle, aryle substitué, hétéroaryle ou -(CH₂)ₘ-A, où m vaut 1, 2, 3, 4, 5, 6, 7, 8, 9 ou 10 et A est un atome d'halogène, un groupe cycloalkyle, hydroxy, amino, cyano, nitro, carboxyle, alcoxy, alkylthio, alcoxycarbonyle, aryloxycarbonyle, alkyloxycarbonylamino, alkylamino, dialkylamino, un groupe hétérocyclique saturé, aryle, aryle substitué ou hétéroaryle,
R² est un atome d'hydrogène ou un groupe alkyle ou aryle, et
R³ est un groupe alkyle, aryle, aryle substitué, hétéroaryle ou -(CH₂)ₘ-A', où m' vaut 1, 2, 3 ou 4, et A' est un atome d'halogène, un groupe cycloalkyle, hydroxyle, amino, cyano, nitro, carboxyle, alcoxy, alkylthio, alcoxycarbonyle, aryloxycarbonyle, alkyloxycarbonylamino, alkylamino, dialkylamino, hétérocyclique saturé, aryle, aryle substitué ou hétéroaryle ;
ou
(2) X¹ est un groupe amino ;
X² est le groupe carbonyle (-(CO)-),
R¹ est un groupe alkyle en C₆-C₁₀, cycloalkyle, aryle substitué par deux ou trois atomes d'halogène, 2, 3-méthylènedioxyphényle, 3,4-méthylènedioxyphényle, aralkyle en C₇-C₁₅ ou -(CH₂)ₘ-A, où m vaut 6, 7, 8, 9, ou 10, et A est un atome d'halogène ou un groupe cycloalkyle, hydroxyle, amino, cyano, carboxyle, alcoxy, alcoxycarbonyle, aryloxycarbonyle, alkyloxycarbonylamino, alkylamino, dialkylamino, un groupe hétérocyclique saturé, aryle ou aryle substitué ; et
R² et R³ sont combinés au noyau pyrimidine pour former une quinazoline, une quinazoline substituée par un ou plusieurs substituants choisis parmi les atomes d'halogène, les groupes alkyle, alcoxy, nitro, cyano, carboxyle, hydroxy, amino, trifluorométhyle, alkylamino, dialkylamino, alcanoyle, alcanoylamino, sulfamoyle, alkylaminosulfonyle, dialkylaminosulfonyle et alcoxycarbonyle ; une pyridopyrimidine, ou une pyridopyrimidine substituée sur la position 5, 6, 7 et/ou 8 par un substituant choisi parmi les atomes d'halogène, les groupes alkyle, alcoxy, nitro, cyano, carboxyle, hydroxy, amino, trifluorométhyle, alkylamino, dialkylamino, alcanoyle, alcanoylamino, sulfamoyle, alkylaminosulfonyle, dialkylaminosulfonyle et alcoxycarbonyle, où ledit groupe aryle de R¹, A, R³ ou A' a au moins un noyau hydrocarboné ayant jusqu'à 10 atomes de carbone ; ledit groupe aryle substitué de R¹, A, R³ ou A' est un groupe aryle substitué par un ou plusieurs substituants choisis parmi les atomes d'halogène, les groupes alkyle ayant de 1 à 10 atomes de carbone, alcoxy ayant de 1 à 6 atomes de carbone, nitro, cyano, carboxyle, hydroxy, amino, trifluorométhyle, alkylamino ayant de 1 à 4 atomes de carbone, dialkylamino ayant de é à 8 atomes de carbone, alcanoyle ayant de 1 à 6 atomes de carbone, alcanoylamino ayant de 1 à 6 atomes de carbone, sulfamoyle, alkylaminosulfonyle où le fragment alkylamino a de 1 à 4 atomes de carbone, dialkylaminosulfonyle où le fragment dialkylamino a de 2 à 8 atomes de carbone, alcoxycarbonyle où le fragment alcoxy a de 1 à 6 atomes de carbone ; et les substituants qui sont combinés les uns aux autres pour former un groupe méthylènedioxy, éthylènedioxy ou dihydroxyfuranne ; et
ledit groupe hétéroaryle de R¹, A, R³ ou A' n'a pas plus de 9 atomes de carbone et 1 à 3 hétéroatomes choisis parmi l'azote, l'oxygène et le soufre, qui peuvent être identiques ou différents,
ou l'un de ses sels acceptables d'un point de vue pharmaceutique.

4. Composé selon la revendication 3, dans lequel, dans la formule (1-a), X¹ est le groupe amino.

5. Composé selon la revendication 3, dans lequel, dans la formule (1-a), X¹ est un groupe amino ou hydroxyle ;
X² est un groupe carbonyle, (-(CO)-), carbonyloxy (-(CO)O-), carbonylamino (-(CO)NH-), ou sulfonyle (-(SO₂)-) ;
R¹ est un groupe alkyle, cycloalkyle, aryle, aryle substitué, hétéroaryle ou -(CH₂)ₘ-A, où m vaut 1, 2, 3, 4, 5, 6, 7, 8, 9 ou 10 et A est un atome d'halogène, un groupe cycloalkyle, hydroxy, amino, cyano, nitro, carboxyle, alcoxy, alkylthio, alcoxycarbonyle, aryloxycarbonyle, alkyloxycarbonylamino, alkylamino, dialkylamino, un groupe hétérocyclique saturé, aryle, aryle substitué ou hétéroaryle,
R² est un atome d'hydrogène ou un groupe alkyle ou aryle, et
R³ est un groupe alkyle, aryle, aryle substitué, hétéroaryle ou -(CH₂)ₘ-A', où m' vaut 1, 2, 3 ou 4, et A' est un atome d'halogène, un groupe cycloalkyle, hydroxyle, amino, cyano, nitro, carboxyle, alcoxy, alkylthio, alcoxycarbonyle, aryloxycarbonyle, alkyloxycarbonylamino, alkylamino, dialkylamino, hétérocyclique saturé, aryle, aryle substitué ou hétéroaryle.

6. Composé selon la revendication 3, dans lequel, dans la formule (1-a), X¹ est le groupe amino ;
X² est le groupe carbonyle (-(CO)-), carbonyloxy (-(CO)O-), ou carbonylamino (-(CO)NH-);
R¹ est un groupe cycloalkyle, aryle, aryle substitué, hétéroaryle ou -(CH₂)ₘ-A où m vaut 1, 2, 3, 4, 5, 6, 7, 8, 9 ou 10, et A est un atome d'halogène ou un groupe cycloalkyle, hydroxyle, amino, cyano, nitro, carboxyle, alcoxy, alkylthio, alcoxycarbonyle, aryloxycarbonyle, alkyloxycarbonylamino, alkylamino, dialkylamino, un groupe hétérocyclique saturé, aryle, aryle substitué ou hétéroaryle ;
R² est un atome d'hydrogène ou un groupe alkyle ou aryle ;
R³ est un groupe aryle, aryle substitué, hétéroaryle ou -(CH₂)_{m'}-A' où m' vaut 1, 2, 3 ou 4, et A' est un atome d'halogène, un groupe cycloalkyle, hydroxyle, amino, cyano, nitro, carboxyle, alcoxy, alkylthio, alcoxycarbonyle, aryloxycarbonyle, alkyloxycarbonylamino, alkylamino, dialkylamino, un groupe hétérocyclique saturé, aryle, aryle substitué ou hétéroaryle.

7. Composé selon la revendication 6, dans lequel X² est le groupe carbonyle (-(CO)-) ou carbonylamino (-(CO)NH-).

8. Composé qui est un dérivé de pipéridinylpyrimidine de formule (1-e) 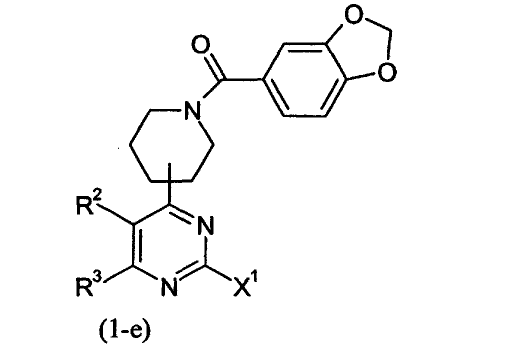 dans laquelle X¹ est le groupe amino ou hydroxy;
R² est un atome d'hydrogène ou un groupe alkyle ou aryle ;
R³ est un groupe alkyle, aryle, aryle substitué, hétéroaryle ou (CH₂)_{m'}-A' où m' vaut 1, 2, 3 ou 4, et A' est un atome d'halogène ou un groupe cycloalkyle, hydroxyle, amino, cyano, nitro, carboxyle, alcoxy, alkylthio, alcoxycarbonyle, aryloxycarbonyle, alcoxycarbonylamino, alkylamino, dialkylamino, un groupe hétérocyclique saturé, aryle, aryle substitué ou hétéroaryle;
ou R² et R³ sont combinés au noyau pyrimidine pour former une quinazoline ou une pyridopyrimidine, où ladite quinazoline peut être substituée par un plusieurs substituants choisis parmi les atomes d'halogène et les groupes alkyle, alcoxy, nitro, cyano, carboxyle, hydroxy, amino, trifluorométhyle, alkylamino, dialkylamino, alcanoyle, alcanoylamino, sulfamoyle, alkylaminosulfonyle, dialkylaminosulfonyle et alcoxycarbonyle, et ladite pyridopyrimidine peut être substituée sur la position 5, 6, 7 et/ou 8 par un substituant choisi parmi les atomes d'halogène et les groupes alkyle, alcoxy, nitro, cyano, carboxyle, hydroxy, amino, trifluorométhyle, alkylamino, dialkylamino, alcanoyle, alcanoylamino, sulfamoyle, alkylarninosulfonyle, dialkylaminosulfonyle et alcoxycarbonyle,
où ledit groupe aryle de R², R³ ou A' possède au moins un noyau hydrocarboné ayant jusqu'à 10 atomes de carbone ; ledit groupe aryle substitué de R³ ou A' est un groupe aryle substitué par un ou plusieurs substituants choisis parmi les halogènes, les groupes alkyle ayant de 1 à 10 atomes de carbone, alcoxy ayant de 1 à 6 atomes de carbone, nitro, cyano, carboxyle, hydroxy, amino, trifluorométhyle, alkylamino ayant de 1 à 4 atomes de carbone, dialkylamino ayant de 2 à 8 atomes de carbone, alcanoyle ayant de 1 à 6 atomes de carbone, alcanoylamino ayant de 1 à 6 atomes de carbone, sulfamoyle, alkylaminosulfonyle où le fragment alkylamino a de 1 à 4 atomes de carbone, dialkylaminosulfonyle où le fragment dialkylamino a de 2 à 8 atomes de carbone, alcoxycarbonyle où le fragment alcoxy a de 1 à 6 atomes de carbone, et les substituants qui sont combinés les uns aux autres pour former un groupe méthylènedioxy, éthylènedioxy ou dihydroxyfuranne ; et
ledit groupe hétéroaryle de R³ ou A' n'a pas plus de 9 atomes de carbone et a de 1 à 3 hétéroatomes choisis parmi l'azote, l'oxygène et le soufre, qui peuvent être différents, ou l'un de ses sels acceptables d'un point de vue pharmaceutique.

9. Composé selon la revendication 3, qui est un dérivé de pipéridinylpyrimidine de formule (1-b) 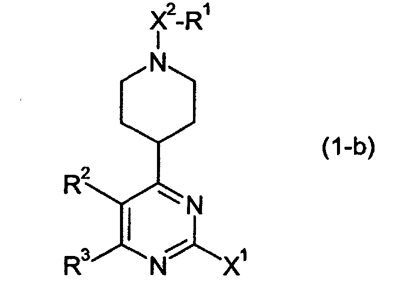 dans laquelle X¹ est le groupe amino;
X² est le groupe carbonyle (-(CO)-), carbonyloxy (-(CO)O-), ou carbonylamino (-(CO)NH-);
R¹ est un groupe cycloalkyle, aryle, aryle substitué, hétéroaryle ou -(CH₂)ₘ-A où m vaut 1, 2, 3, 4, 5, 6, 7, 8, 9 ou 10, et A est un atome d'halogène ou un groupe cycloalkyle, hydroxyle, amino, cyano, nitro, carboxyle, alcoxy, alkylthio, alcoxycarbonyle, aryloxycarbonyle, alkyloxycarbonylamino, alkylamino, dialkylamino, un groupe hétérocyclique saturé, aryle, aryle substitué ou hétéroaryle ;
R² est un atome d'hydrogène ou un groupe alkyle ou aryle ; et
R³ est un groupe aryle, aryle substitué ou -(CH₂)_{m'}-A' où m' vaut 1 ou 2, et A' est un groupe aryle, aryle substitué ou hétéroaryle ; ou l'un de ses sels acceptables d'un point de vue pharmaceutique.

10. Composé selon la revendication 3, qui est un dérivé de pipéridinylpyrimidine de formule (1-d) 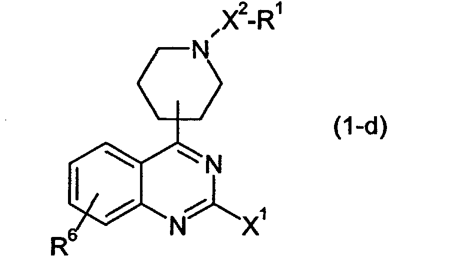 dans laquelle X¹ est un groupe amino ;
X² est le groupe carbonyle (-(CO)-),
R¹ est un groupe alkyle en C₆-C₁₀, cycloalkyle, aryle substitué par deux ou trois atomes d'halogène, 2, 3-méthylènedioxyphényle, 3,4-méthylènedioxyphényle, aralkyle en C₇-C₁₅ ou -(CH₂)ₘ-A, où m vaut 6, 7, 8, 9, ou 10, et A est un atome d'halogène ou un groupe cycloalkyle, hydroxyle, amino, cyano, carboxyle, alcoxy, alcoxycarbonyle, aryloxycarbonyle, alkyloxycarbonylamino, alkylamino, dialkylamino, un groupe hétérocyclique saturé, aryle ou aryle substitué ;
R⁶ est un atome d'halogène ou un groupe alkyle, alcoxy, nitro, cyano, carboxyle, hydroxyle, amino, trifluorométhyle, alkylamino, dialkylamino, alcanoyle, alcanoylamino, sulfamoyle, alkylaminosulfonyle, dialkylaminosulfonyle ou alcoxycarbonyle;
ou l'un de ses sels acceptables d'un point de vue pharmaceutique.
